(19) Europäisches Patentamt
European Patent Office
Office européen des brevets

(11) Publication number: **0 494 622 A1**

# (12) EUROPEAN PATENT APPLICATION

(21) Application number: **92100122.8**

(22) Date of filing: **07.01.92**

(51) Int. Cl.[5]: **C07D 493/08**, A61K 31/34, C12P 17/18, C07H 19/01, C12P 19/02, A61K 31/71, //(C07D493/08,319:00,307:00)

The applicant has filed a statement in accordance with Rule 28 (4) EPC (issue of a sample only to an expert). Accession numbers of the deposits: CAB IMI 322962.

The microorganism has been deposited with the Commonwealth Mycologial Institute under number CMI CC number 332962.

(30) Priority: **09.01.91 GB 9100434**
**09.01.91 GB 9100435**
**09.01.91 GB 9100436**
**09.01.91 GB 9100438**
**09.01.91 GB 9100439**
**09.01.91 GB 9100440**
**09.01.91 GB 9100419**
**09.01.91 GB 9100442**
**01.08.91 GB 9116613**
**07.08.91 GB 9116982**

(43) Date of publication of application:
**15.07.92 Bulletin 92/29**

(84) Designated Contracting States:
**PT**

(71) Applicant: **GLAXO GROUP LIMITED**
**Glaxo House, Berkeley Avenue**
**Greenford, Middlesex UB6 0NN(GB)**

(72) Inventor: **Sidebottom, Philip James**
**Glaxo Group Research Limited, Berkeley Avenue**
**Greenford, Middlesex UB6 0HE(GB)**
Inventor: **Hartley, Charles David**
**Glaxo Group Research Limited, Berkeley Avenue**
**Greenford, Middlesex UB6 0HE(GB)**
Inventor: **Procopiou, Panayiotis Alexandrou**
**Glaxo Group Research Limited, Berkeley Avenue**
**Greenford, Middlesex UB6 0HE(GB)**
Inventor: **Lester, Michael George**
**Glaxo Group Research Limited, Berkeley Avenue**
**Greenford, Middlesex UB6 0HE(GB)**
Inventor: **Watson, Nigel Stephen**
**Glaxo Group Research Limited, Berkeley Avenue**
**Greenford, Middlesex UB6 0HE(GB)**
Inventor: **Kirk, Barrie Edward**
**Glaxo Group Research Limited, Berkeley Avenue**
**Greenford, Middlesex UB6 0HE(GB)**
Inventor: **Cox, Brian**
**Glaxo Group Research Limited, Berkeley Avenue**
**Greenford, Middlesex UB6 0HE(GB)**
Inventor: **Roberts, Suzanne Elaine**
**Glaxo Group Research Limited, Berkeley Avenue**
**Greenford, Middlesex UB6 0HE(GB)**
Inventor: **Ross, Barry Clive**
**Glaxo Group Research Limited, Park Road**
**Ware, Hertfordshire, SG12 0DG(GB)**

(74) Representative: **Caffin, Lee et al**
**Glaxo Holdings plc Glaxo House Berkeley Avenue**
**Greenford, Middlesex UB6 0NN(GB)**

(54) **Bridged cyclic ketal derivatives.**

(57) Compounds are described of the formula

EP 0 494 622 A1

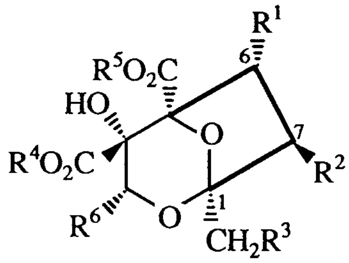

(I)

wherein $R^1$ represents a hydrogen atom or a hydroxyl, acyloxy, carbonate, carbamate or ether group;

$R^2$ represents a hydrogen atom, a hydroxyl group, a group $-OCOR^7$ or a group $-OCO_2R^7$ (where $R^7$ is a group selected from $C_{1-8}$alkyl, aryl, aryl$C_{1-4}$alkyl and $C_{3-8}$cycloalkyl);

$R^3$ represents a group selected from

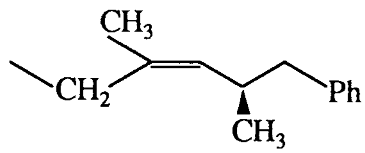

,

$-CH{=}CR^8CR^9R^{10}CHR^{11}(CH_2)_mPh$,

$-CH_2CR^{12}{=}CR^{11}CR^9R^{10}CHR^{11}(CH_2)_nPh$,

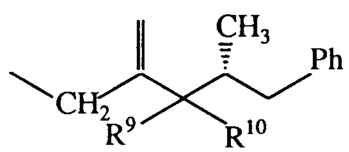

,

$-CH_2C(CH_3)\overset{E}{=}CHCH(CH_2R^{13})CH_2Ph$, $-CH_2C(CH_2OH)\overset{Z}{=}CHCH(CH_3)CH_2Ph$,

$-CH_2C(=CH_2)CH(OH)CH(CH_2OH)CH_2Ph$,

$-CH_2C(=CH_2)CH(NHCOCH_3)CH(CH_3)CH_2Ph$,

$-CH_2C(CH_2NHCOCH_3)\overset{E}{=}CHCH(CH_3)CH_2Ph$ and

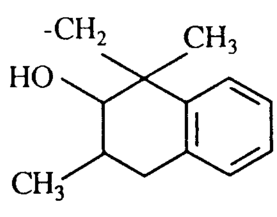

(where the dotted line represents the absence or presence of a single bond, $R^8$ represents a hydrogen atom or a hydroxyl, acyloxy, $C_{1-6}$alkoxy or $C_{1-4}$alkyl group, $R^9$ represents a hydrogen atom and $R^{10}$ represents a hydrogen atom or a hydroxyl, $C_{1-6}$alkoxy or acyloxy group or $CR^9R^{10}$ forms a group C=0, $R^{11}$ represents a hydrogen atom or a $C_{1-4}$alkyl group, $R^{12}$ represents a hydrogen atom or a methyl group, $R^{13}$ represents a hydrogen atom or a hydroxyl group, m represents 1 or 2 and n represents zero or 1);

$R^4$ and $R^5$ may each independently represent a hydrogen atom or a methyl group;

$R^6$ represents a hydrogen atom or a hydroxymethyl group; and salts thereof; with the proviso $R^1$ and $R^2$ cannot both represent hydrogen atoms.

These compounds inhibit the enzyme squalene synthase and/or are intermediates for the preparation of

compounds which inhibit the enzyme squalene synthase. Compounds of the invention may be formulated for use in a variety of conditions where a lowering of the level of blood plasma cholesterol in animals would be beneficial and for use in combating fungal infections in animals.

This invention relates to novel compounds having hypocholesterolemic, hypolipidemic and/or antifungal activity, to processes for their preparation, to pharmaceutical compositions containing them and to their use in medicine, particularly in the treatment and/or prevention of atherosclerosis and associated cardiovascular diseases. The invention also relates to novel compounds which are useful as intermediates for the preparation of compounds having hypocholesterolemic, hypolipidemic and/or antifungal activity.

High levels of blood cholesterol and blood lipids are conditions which are implicated in the onset of vessel wall disease. Methods for effective reduction of plasma cholesterol levels are therefore of high interest. Cholesterol concentrations can be reduced, for example, by lowering the dietary intake of the sterol, by enhancing its metabolism and elimination or by decreasing its rate of biosynthesis. The most effective approaches to lowering physiological cholesterol levels are likely to include inhibition of cholesterol biosynthesis as a component since cholesterol synthesis is subject to feedback regulation, so that decreases in cholesterol levels tend to be compensated for by increased biosynthesis.

One rate-controlling step in the biosynthesis of cholesterol is the formation of mevalonic acid from 3-hydroxy-3-methylglutaryl coenzyme A (HMG CoA) and clinical successes have been achieved with the mevinic acid family of HMG CoA reductase inhibitors in the treatment of hypercholesterolemia. Mevalonic acid, however, is a common precursor of all isoprenyl derivatives, including in animals coenzyme Q, heme A and the dolichols.

The first biosynthetic step which leads exclusively to sterols, the condensation of two farnesyl pyrophosphates to give squalene, is a second site of regulation. The synthesis of squalene from farnesyl pyrophosphate involves an isolable intermediate, presqualene pyrophosphate, and the entire synthetic sequence is catalysed by squalene synthase (farnesyldiphosphate: farnesyldiphosphate farnesyltransferase, EC 2.5.1.21), a membrane-bound enzyme. Agents which act to inhibit the enzyme squalene synthase are therefore potential drugs for the regulation of cholesterogenesis. The use of such agents is attractive as non-steroidal pathways should be minimally affected.

The biosynthesis of ergosterol, the major sterol component of fungal cell membranes, is analogous to that of cholesterol in mammals, including humans, and is thus mediated by the enzyme squalene synthase. Agents which act to inhibit the enzyme squalene synthase in fungal cells are therefore potential drugs for antifungal chemotherapy.

We have now found a group of novel compounds which act as inhibitors of the enzyme squalene synthase and/or are intermediates for the preparation of compounds which act as inhibitors of the enzyme squalene synthase.

Thus, in a first aspect of the present invention, we provide compounds of the general formula (I)

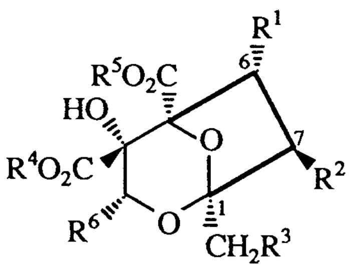

(I)

wherein $R^1$ represents a hydrogen atom or a hydroxyl, acyloxy, carbonate, carbamate or ether group;

$R^2$ represents a hydrogen atom, a hydroxyl group, a group $-OCOR^7$ or a group $-OCO_2R^7$ (where $R^7$ is a group selected from $C_{1-8}$-alkyl, aryl, aryl$C_{1-4}$alkyland $C_{3-8}$cycloalkyl);

$R^3$ represents a group selected from

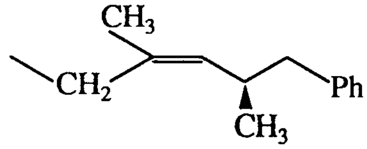

,

$-CH{=}CR^8CR^9R^{10}CHR^{11}(CH_2)_mPh$,

$-CH_2CR^{12}\text{---}CR^{11}CR^9R^{10}CHR^{11}(CH_2)_nPh$,

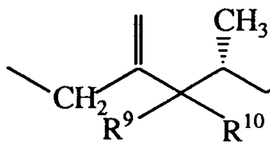,

$-CH_2C(CH_3)\overset{E}{=}CHCH(CH_2R^{13})CH_2Ph$, $-CH_2C(CH_2OH)\overset{Z}{=}CHCH(CH_3)CH_2Ph$,

$-CH_2C(=CH_2)CH(OH)CH(CH_2OH)CH_2Ph$,

$-CH_2C(=CH_2)CH(NHCOCH_3)CH(CH_3)CH_2Ph$,

$-CH_2C(CH_2NHCOCH_3)\overset{E}{=}CHCH(CH_3)CH_2Ph$ and

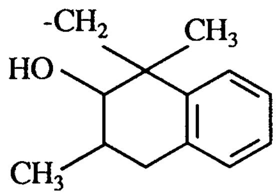

(where the dotted line represents the absence or presence of a single bond, $R^8$ represents a hydrogen atom or a hydroxyl, acyloxy, $C_{1-6}$alkoxy or $C_{1-4}$alkyl group, $R^9$ represents a hydrogen atom and $R^{10}$ represents a hydrogen atom or a hydroxyl, $C_{1-6}$alkoxy or acyloxy group or $CR^9R^{10}$ forms a group C=0, $R^{11}$ represents a hydrogen atom or a $C_{1-4}$alkyl group, $R^{12}$ represents a hydrogen atom or a methyl group, $R^{13}$ represents a hydrogen atom or a hydroxyl group, m represents 1 or 2 and n represents zero or 1);

$R^4$ and $R^5$ may each independently represent a hydrogen atom or a methyl group;

$R^6$ represents a hydrogen atom or a hydroxymethyl group; and salts thereof; with the proviso $R^1$ and $R^2$ cannot both represent hydrogen atoms.

It is to be understood that where the configuration of any double bond or chiral centre present in $R^1$, $R^2$ and/or $R^3$ in formula (I) is not defined the present invention is intended to cover all geometrical and optical isomers, including diastereoisomers, of such compounds of formula (I).

It will also be appreciated that compounds of the invention containing a keto group may exist in the corresponding enolic form.

It is to be understood that when the dotted line in the relevant $R^3$ groupings represents the absence of a single bond

$$-CH\text{---}CR^8CR^9R^{10}CHR^{11}(CH_2)_mPh \text{ and}$$
$$-CH_2CR^{12}\text{---}CR^{11}CR^9R^{10}CHR^{11}(CH_2)_nPh$$

will represent $-CH_2CHR^8CR^9R^{10}CHR^{11}(CH_2)_mPh$ and $-CH_2CHR^{12}CHR^{11}CR^9R^{10}CHR^{11}(CH_2)_nPh$ respectively, and when the dotted line represents the presence of a single bond

$$-CH\text{---}CR^8CR^9R^{10}CHR^{11}(CH_2)_mPh$$
$$\text{and } -CH_2CR^{12}\text{---}CR^{11}CR^9R^{10}CHR^{11}(CH_2)_nPh$$

will represent $-CH=CR^8CR^9R^{10}CHR^{11}(CH_2)_mPh$ and $-CH_2CR^{12}=CR^{11}CR^9R^{10}CHR^{11}(CH_2)_nPh$ respectively.

Examples of $R^1$ as an acyloxy group include

$-OCOCH\overset{E}{=}CHCH(CH_3)(CH_2)_3CH_3$,

$-OCOCH\overset{E}{=}CHC(CH_3)\overset{E}{=}CHCH(CH_3)CH_2CH_3$, $-OCO-X-CH_2CH(CH_3)CH_2CH_3$

[where X is $-CH\overset{E}{=}CHCH(CH_3)-$, $-CH_2CH(OH)CH(CH_3)-$, $-CH\overset{E}{=}CHC(OH)(CH_3)-$,

$-CH_2CH(OH)CH_2-$ or $-CH_2CH_2CH(CH_3)-$],

alkanoyloxy, alkenoyloxy, aroyloxy, arylalkanoyloxy, arylalkenoyloxy, cycloalkanoyloxy, cycloalkylalkanoyloxy and cycloalkylalkenoyloxy.

Examples of $R^1$ as a carbonate group include alkylcarbonates, alkenylcarbonates, arylcarbonates, arylalkylcarbonates, arylalkenylcarbonates, cycloalkylcarbonates, cycloalkylalkylcarbonates and cycloalkylalkenylcarbonates.

When $R^1$ represents a carbamate group this may be, for example, a group $-OCONR^{14}R^{15}$ where $R^{14}$ represents a hydrogen atom or an alkyl group and $R^{15}$ represents a hydrogen atom or a group selected from alkyl, alkenyl, aryl, arylalkyl, arylalkenyl, cycloalkyl, cycloalkylalkyl and cycloalkylalkenyl.

Examples of $R^1$ as an ether group include alkoxy, alkenyloxy, arylalkoxy, arylalkenyloxy, cycloalkyloxy, cycloalkylalkoxy and cycloalkylalkenyloxy.

The term 'alkyl' as part of a group within $R^1$ may be an alkyl chain containing 1-10 carbon atoms optionally substituted by a hydroxyl group, an oxo function (=0) or one or more $C_{1-4}$alkyl groups. Examples of suitable alkyl groups include methyl, ethyl, n-propyl, i-propyl, n-butyl, s-butyl, t-butyl, n-pentyl, n-hexyl, n-heptyl and n-nonyl.

The term 'alkenyl' as part of a group within $R^1$ may be an alkenyl group containing 2-10 carbon atoms and is optionally substituted by one or more $C_{1-4}$alkyl groups. When the alkenyl portion is part of an arylalkenoyloxy group it may represent, for example, an ethenyl group.

The term 'aryl' as a group or part of a group means phenyl or phenyl substituted by one or more moieties including for example halogen atoms, hydroxyl, $C_{1-3}$alkyl and $C_{1-3}$alkoxy groups.

The term 'cycloalkyl' as a group or part of a group means a $C_{3-8}$cycloalkyl group. Examples of suitable cycloalkyl groups include cyclopentyl, cyclohexyl and adamantyl.

The term 'alkyl' within $R^2$ means a straight or branched alkyl chain. Examples of suitable alkyl groups include methyl, ethyl n-propyl, i-propyl, n-butyl, s-butyl, t-butyl and n-heptyl.

Compounds of formula (I) in which $R^4$ and $R^5$ represent hydrogen atoms or physiologically acceptable cations are generally preferred.

$R^1$ preferably represents an acyloxy group.

Examples of preferred acyloxy groups within $R^1$ include

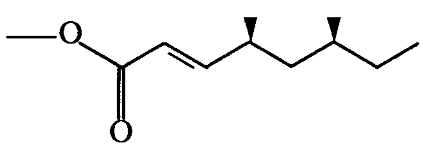 , 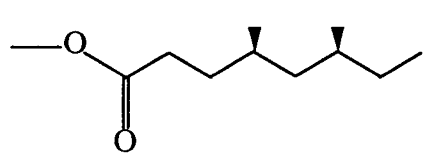

and alkanoyloxy. Particular alkanoyloxy groups within $R^1$ include $-OCO(CH_2)_2CH_3$, $-OCO(CH_2)_5CH_3$, $-OCO(CH_2)_7CH_3$ and $-OCO(CH_2)_2CH(CH_3)_2$.

$R^2$ preferably represents a hydroxyl, acetoxy or $-OCO_2CH_3$ group.

When $R^8$ and/or $R^{10}$ represents an acyloxy group this may be, for example, a $C_{1-6}$alkanoyloxy group and is preferably acetoxy.

$R^3$ preferably represents a group selected from

$-CH\overset{---}{=}CR^8CR^9R^{10}CHR^{11}CH_2Ph$ and

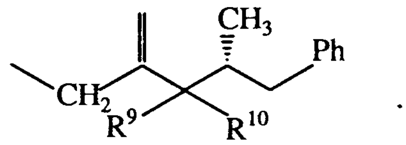

The group

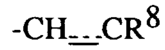

within $R^3$ may preferably represent a group selected from $-CH_2CH(CH_3)-$ and $-CH_2C(=0)-$.

The group $-CR^9R^{10}$ within $R^3$ may preferably represent a group selected from -CH(OH)-, $-CH_2-$ and-CH-($OCOCH_3$)-.

A particular group of compounds of this invention are compounds of formula (I) in which $R^1$ to $R^5$ are as defined above and $R^6$ represents a hydrogen atom.

Another particular group of compounds of this invention are compounds of formula (I) in which $R^1$ to $R^5$ are as defined above and $R^6$ represents a hydroxymethyl group.

It is to be understood that this invention covers any combination of the aforementioned particular and preferred groupings.

Particularly preferred compounds of the invention are :

[1S-[1α(4R*,5S*),3α,4β,5α,6α(4S*,6R*),7β]] 1-(4-hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate);

[1S-[1α(4R*,5S*),4β,5α,6α(2E,4R*,6R*),7β]] 1-(4-acetyloxy-5-methyl-3-methylene-6-phenylhexyl)-4,6,7-trihydroxy-2,8-dioxabicyclo [3.2.1]octane-4,5-dicarboxylic acid, 6-(4,6-dimethyl-2-octenoate);

[1S-[1α(4R*,5S*),3α,4β,5α,6α(2E,4R*,6R*),7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate);

[1S-[1α(4R*,5S*),3α,4β,5α,6α(4S*,6R*),7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate);

[1S-[1α(4R*,5S*),3α,4β,5α,6α(2E,4R*,6R*),7β]] 1-(4-hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate);

[1S-[1α(4R*,5S*),3α,4β,5α,6α,7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-heptanoate;

[1S-[1α(4R*,5S*),3α,4β,5α,6α,7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-nonanoate;

[1S-[1α(4R*,5S*),3α,4β,5α,6α(4R*S*),7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4-methyl)pentanoate;

[1S-[1α(4R*,5S*),3α,4β,5α,6α(4S*,6R*),7β]] 1-(4-hydroxy-5-methyl-3-oxo-6-phenylhexyl)-3-hydroxymethyl-4,6,7-trihydroxy-2,8-dioxabicyclo[3.2.1]octane-4,5-dicarboxylic acid, 6-(4,6-dimethyloctanoate);

and physiologically acceptable salts thereof.

Compounds of formula (I) in which $R^1$ represents a hydroxyl group are particularly useful as intermediates for the preparation of related structures having squalene synthase inhibitory activity.

Compounds of the present invention may form salts with inorganic and organic bases. Physiologically acceptable salts include inorganic base salts such as alkali metal salts (e.g. sodium and potassium salts including the disodium and dipotassium salts), alkaline earth metal salts (e.g. calcium salts), ammonium salts and amino acid salts (e.g. lysine and arginine salts including the di-L-lysine salts). Suitable organic base salts include amine salts such as trialkylamine (e.g. triethylamine), dialkylamine (e.g. dicyclohexylamine), optionally substituted benzylamine (e.g. p-bromobenzylamine) and tris(hydroxymethyl)-methylamine salts.

Compounds of the invention have been found to inhibit the enzyme squalene synthase and cholesterol biosynthesis and are therefore of use in medicine, particularly in a variety of conditions where a lowering of the level of blood plasma cholesterol in animals (especially humans) would be beneficial. Examples of such conditions include diseases associated with hypercholesterolemia and hyperlipoproteinemia, especially atherosclerosis and cardiovascular diseases (such as cardiac ischaemic diseases, cerebral ischaemic

diseases and peripheral arterial disease).

Compounds of the invention which inhibit squalene synthase may also be of use in combating fungal infections in animals, including humans. For example, they may be useful in the treatment of systemic infections caused by, for example Candida (e.g. Candida albicans, Candida glabrata, Candida parapsilosis and Candida pseudotrop), Cryptococcus neoformans, Aspergillus Sp (e.g. Aspergillus flavus and Aspergillus fumigatus), Coccidioides (e.g. Coccidioides immitis), Paracoccidioides (e.g. Paracoccidioides brasiliensis), Histoplasma (e.g. Histoplasma capsulatum) or Blastomyces (e.g. Blastomyces dermatitidis). They may also be useful in treating topical infections caused by species of Trichophyton, Microsporum or Epidermophyton (e.g. Trichophyton mentographytes, Microsporum canis or Epidermophyton floccosum). They may also be of use in treating fungal diseases caused by Torulopsis glabrata and Pityrosporum ovale.

The in vitro evaluation of the anti-fungal activity of compounds of the invention can be performed by determining the minimum inhibitory concentration (MIC) which is the concentration of the test compound in a suitable medium at which growth of a particular microorganism fails to occur.

In view of their potential in antifungal therapy, compounds of the invention which inhibit squalene synthase may recommend themselves for the treatment of a variety of fungal infections in human beings and animals. Such infections include mycotic infections such as candidiasis and chronic mucocandidiasis (e.g. thrush and vaginal candidiasis) and skin infections caused by fungi, cutaneous and mucocutaneous candidiasis, dermatophytoses including ringworm and tinea infections, athletes foot, paronychia, pityriasis versicolor, erythrasma, intertrigo, fungal nappy rash, candida vulvitis, candida balanitis and otitis externa. They may also be useful as prophylactic agents to prevent systemic and topical fungal infections. Use as prophylactic agents may, for example, be appropriate as part of a selective gut decontamination regimen in the prevention of infection in immunocompromised patients. Prevention of fungal overgrowth during antibiotic treatment may also be desirable in some disease syndromes or iatrogenic states.

The ability of compounds of the invention to inhibit the enzyme squalene synthase in mammals and fungi may be demonstrated in vitro using [2-$^{14}$C]farnesylpyrophosphate as a substrate under assay conditions similar to those described by S. A. Biller et al. in J. Medicinal Chemistry 31(10), 1869-1871 (1988); [$^{14}$C]squalene is separated from unreacted substrate on thin layer chromatography plates and determined by liquid scintillation counting. The ability of compounds of the invention to inhibit cholesterol biosynthesis may be demonstrated by measuring inhibition from [$^{14}$C]-acetate in liver slices from male Wistar rats using a method similar to that described by Y. Tsujita et al. in Biochem. Biophys. Acta, Volume 877, 50-60 (1986) and modified to include measurement of cholesterol by high performance liquid chromatography (h.p.l.c.).

While it is possible that, for use in therapy, compounds of the invention which inhibit squalene synthase may be administered as the raw chemical, it is preferable to present the active ingredient as a pharmaceutical formulation. The invention thus further provides a pharmaceutical formulation comprising compounds of the invention which inhibits squalene synthase together with one or more pharmaceutically acceptable carriers thereof and, optionally, other therapeutic and/or prophylactic ingredients. The carrier(s) must be 'acceptable' in the sense of being compatible with the other ingredients of the formulation and not deleterious to the recipient thereof.

The compositions of the invention include those in a form especially formulated for oral, buccal, parenteral, implant, rectal, topical, ophthalmic or genito-urinary administration or in a form suitable for administration by inhalation or insufflation.

Tablets and capsules for oral administration may contain conventional excipients such as binding agents, for example, syrup, acacia, gelatin, sorbitol, tragacanth, mucilage of starch or polyvinylpyrrolidone; fillers, for example, lactose, sugar, microcrystalline cellulose, maize-starch, calcium phosphate or sorbitol; lubricants, for example, magnesium stearate, stearic acid, talc, polyethylene glycol or silica; disintegrants, for example, potato starch or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxyethylcellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example, almond oil, fractionated coconut oil, oily esters, propylene glycol or ethyl alcohol; and preservatives, for example, methyl or propyl p-hydroxybenzoates or sorbic acid. The compositions may also be formulated as suppositories, e.g. containing conventional suppository bases such as cocoa butter or other glycerides.

For buccal administration the composition may take the form of tablets or lozenges formulated in

conventional manner.

The composition according to the invention may be formulated for parenteral administration by injection or continuous infusion. Formulations for injection may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compositions according to the invention are conveniently delivered in the form of an aerosol spray presentation from pressurised packs with the use of a suitable propellant, e.g. dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation the compositions according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The compositions may take the form of a suppository, e.g. containing a conventional suppository base, or a pessary, e.g. containing a conventional pessary base.

The compositions may also be formulated for topical administration in the form of ointments, creams, gels, lotions, shampoos, powders (including spray powders), pessaries, tampons, sprays, dips, aerosols, drops (e.g. eye, ear or nose drops) or pour-ons. Ointments and creams may, for example, be formulated with an aqueous or oily base with the addition of suitable thickening and/or gelling agents. Ointments for administration to the eye may be manufactured in a sterile manner using sterilised components. Pour-ons may, for example, be formulated for veterinary use in oils containing organic solvents, optionally with formulatory agents, e.g. stabilising and solubilising agents. Pessaries and tampons for vaginal insertion may be formulated using conventional techniques and, where appropriate, may contain an effervescent vehicle. Such compositions may also contain other active ingredients such as corticosteroids, antibiotics or antiparasitics as appropriate.

Liquid preparations for intranasal delivery may take the form of solutions or suspensions and may contain conventional excipients such as tonicity adjusting agents, for example, sodium chloride, dextrose or mannitol; preservatives, for example benzalkonium chloride, thiomersal, phenylethyl alcohol; and other formulating agents such as suspending, buffering, stabilising and/or dispersing agents.

Transdermal administration may be affected by the design of a suitable system which promotes adsorption of the active compound through the skin and would typically consist of a base formulation enclosed within an adhesive stick-on patch comprising backing films, membranes and release liners.

The composition according to the invention may also be formulated as a depot preparation. Such long acting formulations may be administered by implantation (for example subcutaneously or intramuscularly) or by intramuscular injection. Thus, for example, a compound of the invention may be formulated with suitable polymeric or hydrophobic materials (for example as an emulsion in an acceptable oil) or ion exchange resins, or as sparingly soluble derivatives, for example, as a sparingly soluble salt.

When the compositions comprise dosage units, each unit will preferably contain 0.001mg to 1000mg, advantageously 0.01mg to 400mg, of active ingredient where a compound of the invention is to be administered orally. The daily dosage as employed for adult human treatment will preferably range from 0.001mg to 5000mg of active ingredient, most preferably from 0.01mg to 2000mg which may be administered in 1 to 4 daily doses, for example, depending on the route of administration and on the condition of the patient and the disease to be treated.

The compound may be administered by intravenous infusion using, for example, up to 50mg/kg/day of the active ingredient. The duration of treatment will be dictated by the rate of response rather than by arbitrary numbers of days.

Compounds of the invention which inhibit squalene synthase may also be used in combination with other therapeutic agents, and the invention thus provides, in a further aspect, a combination comprising a compound of the invention which inhibits squalene synthase together with another therapeutically active agent, such as an inhibitor of 3-hydroxy-3-methylglutaryl coenzyme A (HMG CoA) reductase or another agent which reduces serum cholesterol and/or inhibits cholesterol biosynthesis, for example a bile acid sequestrant or an antihyperlipoproteinemic or antihyperlipemic agent such as probucol, gemfibrozil, clofibrate, dextrothyroxine or its sodium salt, colestipol or its hydrochloride salt, cholestyramine, nicotinic acid, neomycin, p-aminosalicylic acid, aspirin, DEAE-Sephadex, a poly(diallylmethylamine) derivative, an

ionene or poly(diallyldimethylammonium) chloride.

The combinations referred to above may conveniently be presented for use in the form of a pharmaceutical formulation and thus pharmaceutical formulations comprising a combination as defined above together with a pharmaceutically acceptable carrier thereof comprise a further aspect of the invention. The individual components of such combinations may be administered either sequentially or simultaneously in separate or combined pharmaceutical formulations.

When a compound of the invention is used in combination with a second therapeutic agent against the same condition the dose of each compound may differ from that when the compound is used alone. Appropriate doses will be readily appreciated by those skilled in the art.

According to another aspect of the present invention, we provide a compound of formula (I) or a physiologically acceptable salt thereof or a pharmaceutical composition comprising a compound of formula (I) or a physiologically acceptable salt thereof as defined above for use in therapy, particularly for the treatment of conditions where a lowering of the level of blood plasma cholesterol in animals (especially humans) would be beneficial, or for the treatment of fungal infections in animals (especially humans).

In a particular aspect of the present invention, we provide a compound of formula (I) or a physiologically acceptable salt thereof or a pharmaceutical composition comprising a compound of formula (I) or a physiologically acceptable salt thereof as defined above for use in the treatment of diseases associated with hypercholesterolemia and/or hyperlipoproteinemia, especially atherosclerosis and cardiovascular diseases (such as cardiac ischaemic diseases, cerebral ischaemic diseases and peripheral arterial disease).

According to a further aspect of the present invention, we provide the use of a compound of formula (I) or a physiologically acceptable salt thereof in the manufacture of a medicament for the treatment of diseases associated with hypercholesterolemia and/or hyperlipoproteinemia, especially atherosclerosis and cardiovascular diseases (such as cardiac ischaemic diseases, cerebral ischaemic diseases and peripheral arterial disease).

According to another aspect of the present invention, we provide the use of a compound of formula (I) or a physiologically acceptable salt thereof in the manufacture of a medicament for the treatment of fungal infections in a human or non-human animal patient.

According to a further aspect of the present invention, we provide a method of treatment of the human or non-human animal body to combat diseases associated with hypercholesterolemia and/or hyperlipoproteinemia, especially atherosclerosis and cardiovascular diseases (such as cardiac ischaemic diseases, cerebral ischaemic diseases and peripheral arterial disease) or to combat fungal diseases, which method comprises administering to said body an effective amount of a compound of formula (I) or a physiologically acceptable salt thereof.

It will be appreciated by those skilled in the art that references herein to treatment extend to prophylaxis as well as the treatment of established conditions or infections.

The compounds of the invention may be prepared by the processes described below.

Thus, a general process (A) for the preparation of compounds of formula (I) in which $R^6$ represents a hydrogen atom comprises reacting a compound of formula (II)

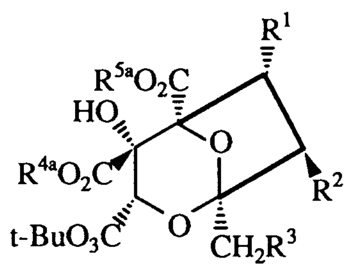

(II)

(wherein $R^1$, $R^2$ and $R^3$ are as defined previously and $R^{4a}$ and $R^{5a}$ are protecting groups) to remove the group $t\text{-}BuO_3C$ by reduction under free radical conditions, followed by removal of the protecting groups present.

The reductive decarboxylation may conveniently be effected by photochemical means using, for example, a 125W medium pressure mercury lamp in the presence of a hydrogen radical donor such as an appropriate alkylmercaptan (e.g. t-butylmercaptan) in an inert solvent such as an aromatic hydrocarbon (e.g. toluene).

A general process (B) for the preparation of compounds of formula (I) in which $R^6$ represents a hydroxymethyl group comprises reacting a compound of formula (III)

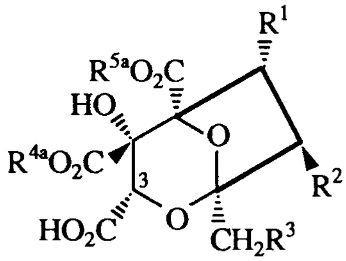

(III)

(wherein $R^1$, $R^2$, $R^3$, $R^{4a}$ and $R^{5a}$ are as defined previously) to reduce the 3-carboxyl group to a hydroxymethyl group, followed by removal of the protecting groups present.

The reaction may conveniently be effected by activation of the 3-carboxyl group followed by reduction with a suitable reducing agent such as a borohydride (e.g. sodium borohydride) in a solvent such as an ether (e.g. tetrahydrofuran) optionally in the presence of water at a suitable temperature, for example in the range of $0^0$ to $50^0$C (e.g. about room temperature).

Activation of the 3-carboxyl group may be effected, for example, by conversion to an active ester by reaction with a reagent such as N-hydroxysuccinimide in a suitable solvent such as an ether (e.g. tetrahydrofuran) at a temperature in the range $0^0$-$20^0$C and in the presence of a carbodiimide [e.g. 1-cyclohexyl-3-(2-morpholinoethyl)carbodiimide metho-p-toluenesulphonate] or by reaction with 2-chloro-3-ethylbenzoxazolium tetrafluoroborate in a suitable solvent such as a halogenated hydrocarbon (e.g. dichloromethane) in the presence of a non-nucleophilic organic base such as triethylamine at a temperature in the range $0^0$-$20^0$C.

Compounds of formula (I) in which $R^1$ represents a hydroxyl group may conveniently be prepared from compounds of formula (II) or (III) in which $R^1$ represents an acyloxy group as defined in formula (I) above by deacylation (under condition described hereinafter) following the free radical elimination reaction in process (A) or the reduction reaction in process (B) and prior to or subsequent to the removal of the protecting groups.

Compounds of formula (II) may conveniently be prepared from compounds of formula (III) by treating (III) with a carboxyl activating agent such as oxalyl chloride in dimethylformamide followed by treatment with t-butylhydroperoxide in the presence of a tertiary amine base such as triethylamine optionally in the presence of 4-dimethylaminopyridine. The reaction is conveniently carried out at a temperature in the range of $0^0$ to $20^0$C.

Compounds of formula (III) may conveniently be prepared from compounds of formula (IV)

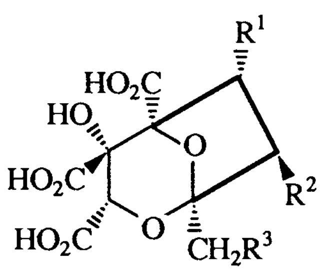

(IV)

(wherein $R^1$, $R^2$ and $R^3$ are as defined in formula (I) above) by standard carboxylic acid protection methods.

A process (C) for preparing compounds of formula (I) in which $R^3$ represents a group $-CH=CR^8CR^9R^{10}CHR^{11}(CH_2)_mPh$ or a group $-CH_2CR^{12}=CR^{11}CR^9R^{10}CHR^{11}(CH_2)_nPh$ where $R^8$ is hydrogen or $C_{1-4}$alkyl comprises reacting a compound of formula (V)

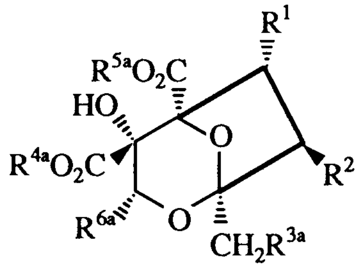

(V)

(wherein $R^{3a}$ represents CHO or $CH_2COR^{12}$ as appropriate, $R^1$ and $R^2$ are as defined previously, $R^{4a}$ and $R^{5a}$ are as defined for $R^4$ and $R^5$ above or are protecting groups and $R^{6a}$ is as defined for $R^6$ above or represents a protected hydroxymethyl group with a compound of formula (VIa) or(VIb)

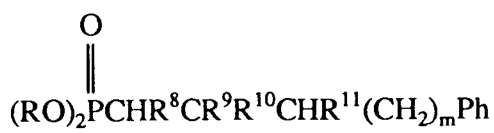

(VIa)

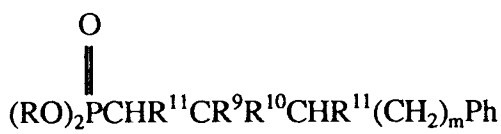

(VIb)

as appropriate (wherein $R^{11}$ is as defined previously, $R^8$ is hydrogen or $C_{1-4}$alkyl, $CR^9R^{10}$ forms a group C=0 and R represents a $C_{1-6}$alkyl group, e.g. methyl or an aryl group, e.g. phenyl) under Wadsworth-Emmons conditions, followed, where appropriate by converting $CR^9R^{10}$ as a group C=0 to a group $CHR^{10}$ where $R^{10}$ is hydroxy, $C_{1-6}$alkoxy or acyloxy according to the general procedures described hereinafter, and thereafter removing any protecting groups present.

The reaction between compounds (V) and (VIa) or (VIb) may conveniently be carried out in an ether solvent (e.g. tetrahydrofuran) in the presence of a strong base such as an alkali metal hydride (e.g. sodium hydride) at a temperature in the range of $0^0$ to $20^0$C.

Another general process (D) comprises converting a compound of formula (I) or a protected derivative thereof to a different compound of formula (I) or a protected derivative thereof, followed, if necessary, by the removal of any protecting groups present. Specific examples of interconversion reactions are described hereinafter.

Compounds of formula (I) in which $R^3$ represents a group $-CH_2CHR^8CR^9R^{10}CHR^{11}(CH_2)_mPh$ or $CH_2CHR^{12}CHR^{11}CR^9R^{10}CHR^{11}(CH_2)_nPh$ may be prepared from the corresponding compounds of formula (I) in which $R^3$ represents a group $-CH=CR^8CR^9R^{10}CHR^{11}(CH_2)_mPh$ or $CH_2CR^{12}=CR^{11}CR^9R^{10}CHR^{11}(CH_2)_nPh$ or protected derivatives thereof under appropriate reducing conditions, for example by catalytic hydrogenation [e.g. hydrogenation in the presence of palladium-on-carbon in a suitable solvent such as an ester (e.g. ethyl acetate) or an alcohol (e.g. ethanol)], followed, where appropriate, by removing any protecting groups present.

Compounds of formulae (VIa) and (VIb) are either known compounds or may be prepared by methods analogous to those used to prepare the known compounds of formulae (VIa) and (VIb).

Compounds of formula (V) in which $R^{3a}$ represents CHO may be prepared by ozonolysis of a compound of formula (VII)

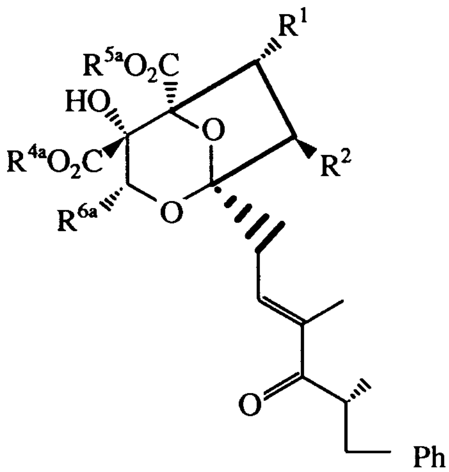

(VII)

(wherein $R^1$, $R^2$, $R^{4a}$, $R^{5a}$ and $R^{6a}$ are as defined previously). Thus, for example, a compound of formula (VII) may conveniently be treated with ozone in a halogenated hydrocarbon solvent (e.g. dichloromethane) at a low temperature (e.g. -70°C to 0°C), followed by treatment with either a triarylphosphine such as triphenylphosphine or a dialkyl sulphide such as dimethyl sulphide to provide the desired compound of formula (V).

Compounds of formula (VII) may be prepared by isomerization of a compound of formula (VIII)

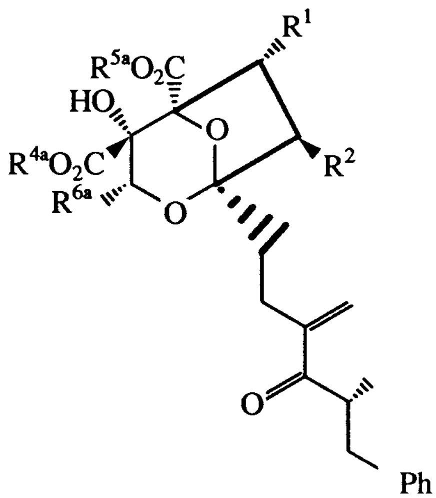

(VIII)

(wherein $R^1$, $R^2$, $R^{4a}$, $R^{5a}$ and $R^{6a}$ are as previously defined). The isomerization may conveniently be effected by heating a compound of formula (VIII) with a suitable transition metal catalyst such as rhodium trichloride in an aqueous alcoholic solvent (e.g. aqueous methanol).

Compounds of formula (I) in which $R^3$ represents a group

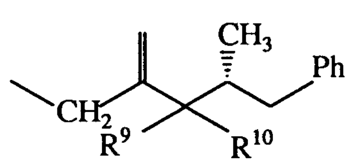

(wherein $CR^9R^{10}$ forms a group C=0) are either compounds of formula (VIII) or may be prepared from compounds of formula (VIII) by removing any protecting groups present. Compounds of formula (I) in which $R^9$ is hydrogen and $R^{10}$ is a hydroxyl group may be prepared by reducing the keto group in formula (VIII) using, for example, sodium borohydride in an alcoholic solvent (e.g. methanol) at a temperature in the range 0° to 20°C or using zinc dust in an aqueous ether (e.g. aqueous tetrahydrofuran), followed where necessary by removing any protecting groups present. Compounds of formula (I) in which $R^9$ is hydrogen and $R^{10}$ is

an acyloxy group may be prepared from the corresponding compounds in which $R^9$ is hydrogen and $R^{10}$ is a hydroxyl group or protected derivatives thereof by conventional acylation means described hereinafter, followed where necessary by removal of any protecting groups present. Compounds of formula (I) in which $R^9$ is hydrogen and $R^{10}$ is a $C_{1-6}$alkoxy group may be prepared from the corresponding compounds in which $R^9$ is hydrogen and $R^{10}$ is a hydroxyl group or protected derivatives thereof by conventional etherification means described hereinafter, followed where necessary by removal of any protecting groups present.

Compounds of formula (I) in which $R^3$ represents a group

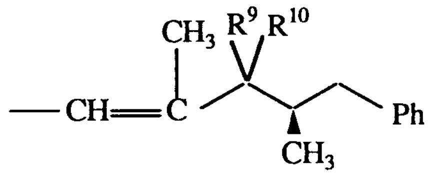

(wherein $CR^9R^{10}$ forms a group C=0) are either compounds of formula (VII) or may be prepared from compounds of formula (VII) by removing any protecting groups present. Compounds of formula (I) in which $R^9$ is hydrogen and $R^{10}$ is a hydroxyl group may be prepared by reducing the keto group in formula (VII) using, for example, sodium borohydride in an alcoholic solvent (e.g. methanol) at a temperature in the range $0^0$ to $20^0$C or using zinc dust in an aqueous ether (e.g. aqueous tetrahydrofuran), followed where necessary by removing any protecting groups present. Compounds of formula (I) in which $R^9$ is hydrogen and $R^{10}$ is an acyloxy group may be prepared from the corresponding compounds in which $R^9$ is hydrogen and $R^{10}$ is a hydroxyl group or a protected derivative thereof by conventional acylation means as described hereinafter, followed where necessary by removal of any protecting groups present. Compounds of formula (I) in which $R^9$ is hydrogen and $R^{10}$ is a $C_{1-6}$alkoxy group may be prepared from the corresponding compounds in which $R^9$ is hydrogen and $R^{10}$ is a hydroxyl group or protected derivatives thereof by conventional etherification means described hereinafter, followed where necessary by removal of any protecting groups present.

Compounds of formula (I) in which $R^3$ represents a group (wherein $R^8$ is

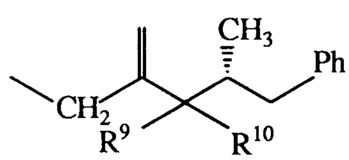

$C_{2-4}$alkyl, $R^9$ is a hydrogen atom and $R^{10}$ is a hydroxyl or acyloxy group or $CR^9R^{10}$ forms a group C=0) may be prepared from compounds of formula (VIII). Thus, compounds in which $CR^9R^{10}$ represents C=0 may be prepared by treating the compound of formula (VIII) with a suitable lithium cuprate $LiCu(R^{16})_2$ - (where $R^{16}$ is a $C_{1-3}$alkyl group, e.g. methyl) at reduced temperature (e.g. $-20^0$ to $+10^0$C) in an ether solvent (e.g. tetrahydrofuran), followed where necessary by removing any protecting groups present. The resulting keto compounds of formula (I) or protected derivatives thereof may then be converted to the corresponding compounds of formula (I) in which $R^8$ is $C_{2-4}$alkyl, $R^9$ is hydrogen and $R^{10}$ is a hydroxyl, $C_{1-6}$alkoxy or acyloxy group by reduction as described hereinabove, followed where appropriate by etherification or acylation as described hereinafter, followed, where necessary, by removal of any protecting groups present.

Compounds of formula (I) in which $R^3$ represents a group

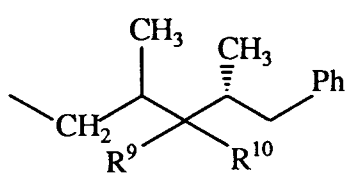

(wherein $R^9$ represents a hydrogen atom and $R^{10}$ represents a hydroxyl, $C_{1-6}$alkoxy or acyloxy group or $CR^9R^{10}$ forms as group C=0) may be prepared from compounds of formula (IX)

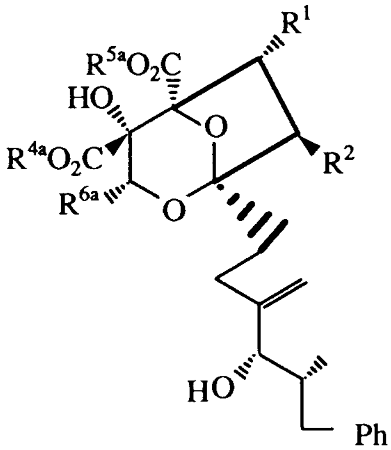

(IX)

(wherein $R^1$, $R^2$, $R^{4a}$, $R^{5a}$ and $R^{6a}$ are as previously defined). Thus, compounds in which $CR^9R^{10}$ represents C=0 may be prepared by treating a compound of formula (IX) with a suitable transition metal reagent such as a rhodium complex, e.g. $RhCl(PPh_3)_3$ at an elevated temperature (e.g. at reflux), in a suitable solvent such as an aqueous alcohol (e.g. aqueous methanol), or using a suitable metal catalyst such as palladium-on-carbon in an organic solvent, for example an ester or an alcohol at ambient temperature, followed where necessary by removing any protecting groups present. The resulting keto compounds of formula (I) or protected derivatives thereof may then be converted to the corresponding compounds of formula (I) in which $R^9$ is hydrogen and $R^{10}$ is a hydroxyl, $C_{1-6}$alkoxy or acyloxy group by reduction as described hereinabove, followed where appropriate by etherification or acylation as described hereinafter, followed by removal of any protecting groups present.

The aforementioned conversion of $CR^9R^{10}$ as CHOH to $CHR^{10}$ where $R^{10}$ is a $C_{1-6}$alkoxy group may be effected by reaction under conventional conditions for ether formation. Thus, for example, the conversion may conveniently be effected by reaction with a halide $R^{17}$Hal (where Hal is a halogen atom such as bromine or iodine and $R^{17}$ is a $C_{1-6}$alkyl group) preferably in the presence of a suitable base such as an alkali metal hydroxide (e.g. potassium hydroxide), an alkali metal hydride (e.g. sodium hydride) or an alkali metal alkoxide (e.g. potassium tert-butoxide) in a solvent such as an ether (e.g. tetrahydrofuran) or a dialkylamide (e.g. dimethylformamide).

The aforementioned conversion of $CR^9R^{10}$ as CHOH to $CHR^{10}$ where $R^{10}$ is an acyloxy group may conveniently be effected by reaction with a suitable acylating agent under conventional conditions. Thus, for example the conversion may conveniently be effected by reaction with an acyl halide, for example an acyl chloride, in the presence of 4-dimethylaminopyridine with or without a suitable base such as a tertiary amine (e.g.triethyamine) or using an alkali metal carbonate or alkaline earth metal carbonate (e.g. calcium carbonate) in a solvent such as a halogenated hydrocarbon (e.g. dichloromethane).

Compounds of formula (I) in which $R^3$ represents a group

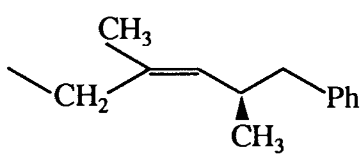

may be prepared from compounds of formula (IX) by catalytic hydrogenolysis using a suitable palladium catalyst such as palladium-on-barium sulphate in a suitable solvent such as an alcohol (e.g. ethanol), followed where necessary by removing any protecting groups present.

Compounds of formula (I) in which $R^3$ represents

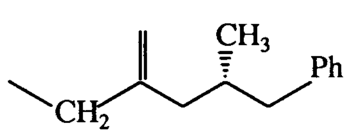

may be prepared from compounds of formula (X)

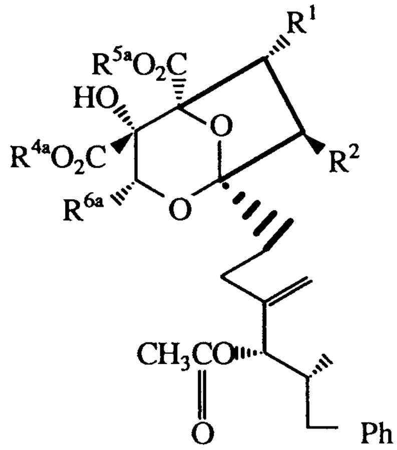

(X)

(wherein $R^1$, $R^2$, $R^{4a}$, $R^{5a}$ and $R^{6a}$ are as defined previously) by heating the compound of formula (X) with ammonium formate in the presence of a suitable palladium catalyst such as $(Ph_3P)_2PdCl_2$ and in a suitable solvent such as an ether (e.g. dioxan), followed where necessary by removing any protecting groups present.

Compounds of formula (I) in which $R^3$ represents

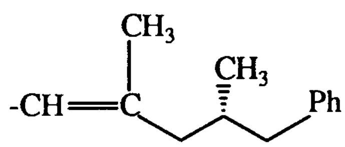

may be prepared from a compound of formula (I) in which $R^3$ represents

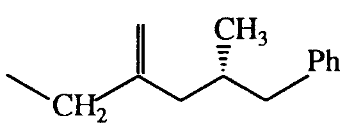

by isomerization using, for example, a hydrogen halide (e.g. hydrogen chloride) in a solvent such as an ether (e.g. dioxan).

Compounds of formula (I) in which $R^3$ represents

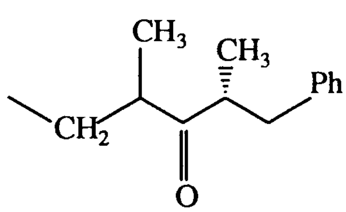

may also be prepared from compounds of formula (I) in which $R^3$ represents

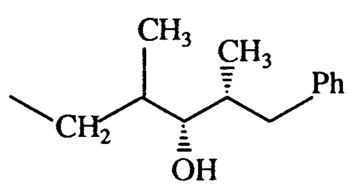

or protected derivatives thereof using a suitable oxidising agent such as pyridinium chlorochromate in a halogenated hydrocarbon solvent (e.g. dichloromethane), followed where necessary by removal of any protecting groups present.

Compounds of formula (I) in which $R^3$ represents

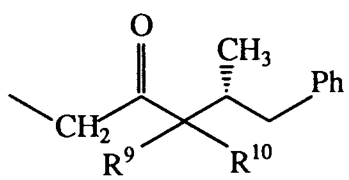

may be prepared from corresponding compounds in which $R^3$ represents

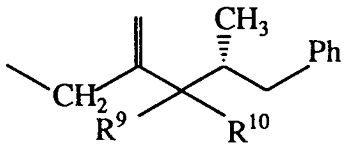

by ozonolysis using for example ozone in a solvent such as a halogenated hydrocarbon (e.g. dichloromethane) at a low temperature (e.g. -70°C to 0°C), followed by treatment with either a triarylphosphine such as triphenylphosphine or a dialkyl sulphide such as dimethyl sulphide, and thereafter where necessary followed by removing any protecting groups present.

Compounds of formula (I) in which $R^3$ represents

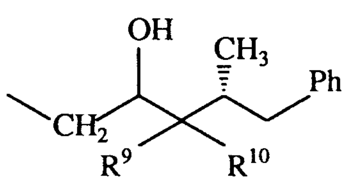

(where $R^9$ and $R^{10}$ are as defined previously except that $CR^9R^{10}$ may not represent C=O) may be prepared from compounds of formula (I) in which $R^3$ represents

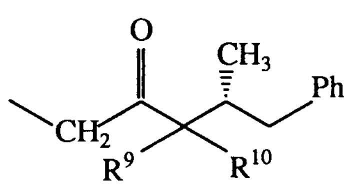

or protected derivatives thereof by reduction, for example using a suitable reducing agent such as sodium borohydride in an appropriate solvent (e.g. an alcohol such as methanol) at reduced temperature (e.g. -10°C to +10°C), followed where necessary by removal of any protecting groups present.

Compounds of formula (I) in which $R^3$ represents a group

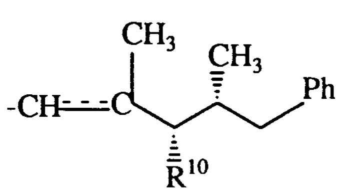

(in which $R^{10}$ is hydrogen, hydroxy or acetoxy) may also be prepared from compounds of formulae (IX) and (X) as appropriate by catalytic hydrogenation using hydrogen in the presence of a suitable palladium

catalyst (e.g. palladium-on-carbon) in a solvent such as ethyl acetate, or a mixture of an alcohol (e.g. ethanol) and a halogenated hydrocarbon (e.g. dichloromethane) in the presence of triethylamine, or by isomerisation using a suitable palladium catalyst as defined above under the conditions described just above, optionally in the presence of hydrogen and followed where appropriate by removing any protecting groups present.

Compounds of formula (V) in which $R^{3a}$ represents $CH_2CHO$ may conveniently be prepared from the aforementioned compounds of formula (I) in which $R^3$ represents

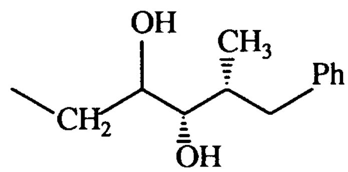

by oxidation using a suitable oxidising agent such as a periodate (e.g. sodium periodate) in an aqueous ether (e.g aqueous tetrahydrofuran) conveniently at a temperature in the range $0^{o}$-$20^{o}C$.

Compounds of formula (V) in which $R^{3a}$ represents $CH_2COCH_3$ may conveniently be prepared from compounds of formula (XI)

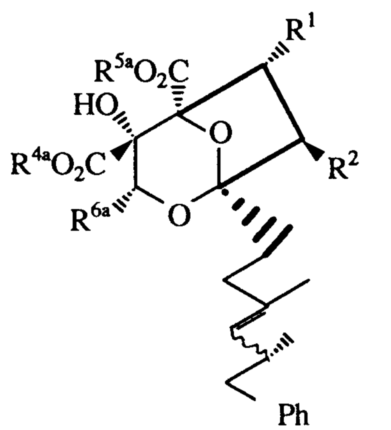

(XI)

(wherein $R^1$, $R^2$, $R^{4a}$, $R^{5a}$ and $R^{6a}$ are as defined previously) by ozonolysis using for example ozone in a solvent such as a halogenated hydrocarbon (e.g. dichloromethane) at a low temperature (e.g. $-70^{o}C$ to $0^{o}C$), followed by treatment with either a triarylphosphine such as triphenylphosphine or a dialkyl sulphide such as dimethyl sulphide.

Compounds of formula (XI) in which $R^{4a}$ and $R^{5a}$ represent protecting groups may be prepared from the corresponding compounds of formula (XI) in which $R^{4a}$ and $R^{5a}$ are hydrogen atoms using standard methods of carboxylic acid group protection. Such compounds of formula (XI) in which $R^{4a}$ and $R^{5a}$ represent hydrogen atoms may conveniently be prepared from compounds of formula (IX) in which $R^{4a}$ and $R^{5a}$ are hydrogen atoms by catalytic hydrogenolysis using palladium-on-barium sulphate in a suitable solvent such as an alcohol (e.g. ethanol).

Compounds of formula (VIII) may conveniently be prepared from compounds of formula (IX) by oxidation, using for example a suitable oxidising agent such as pyridinium chlorochromate in a halogenated hydrocarbon solvent (e.g. dichloromethane).

It will be appreciated that certain of the above reactions (e.g. hydrogenation, ozonolysis and isomerisation) may not be suitable for compounds in which $R^1$ contains one or two double bonds. In these cases the reaction will be effected on a compound containing an $R^1$ group stable under the conditions of the reaction. The reaction may then be followed by appropriate means described herein to introduce the desired $R^1$ grouping.

Compounds of formulae (IX) and (X) in which one or both of $R^{4a}$ and $R^{5a}$ represents a protecting group may be prepared from the corresponding carboxylic acid of formula (IX) or (X) using standard protection methods.

According to a further process (E) compounds of formula (I) in which $R^2$ represents a group $-OCOR^7$ or $-OCO_2R^7$ may be prepared by reacting a compound of formula (XII)

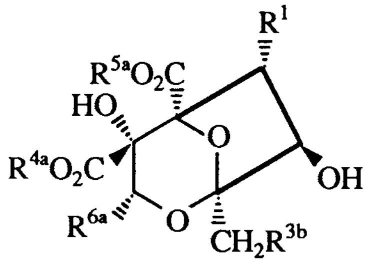

(XII)

(wherein $R^1$, $R^{4a}$, $R^{5a}$ and $R^{6a}$ are as defined previously and $R^{3b}$ is as defined for $R^3$ above or is a protected derivative thereof) under conventional conditions for ester and carbonate formation, followed by removal of the protecting groups present. Thus, for example, the reaction may conveniently be effected by treating a compound of formula (XII) with a compound $R^7COHal$ or $R^7OCOHal$ as appropriate, wherein Hal represents a halogen atom such as chlorine or bromine.

The reaction with a compound $R^7COHal$ or $R^7OCOHal$ may conveniently be effected in the presence of 4-dimethylaminopyridine with or without a suitable base such as a tertiary amine (e.g. triethylamine) or using an alkali metal carbonate or alkaline earth metal carbonate (e.g. calcium carbonate) in a solvent such as a halogenated hydrocarbon (e.g. dichloromethane).

It will be appreciated that when $R^1$ in the compounds of formula (XII) represents a hydroxyl group this hydroxyl group will also be susceptible to ester and carbonate formation. Thus, in the preparation of compounds of formula (I) in which $R^1$ is a hydroxyl group it may be appropriate to have protected the $R^1$ hydroxyl group in compounds of formula (XII) or utilise a compound of formula (XII) in which $R^1$ is an acyloxy group, and following the reaction to form the 7-position ester or carbonate group remove the protecting group or deacylate as appropriate to provide the desired compound of formula (I) in which $R^1$ is hydroxyl.

Compounds of formula (I) in which $R^2$ represents an acetoxy group may conveniently be prepared from compounds of formula (XII) by reaction with acetic anhydride followed, if necessary, by removal of any protecting groups present. The acetylation reaction may conveniently be effected in the presence of a suitable base such as a tertiary amine (e.g. triethylamine) in a solvent such as a halogenated hydrocarbon (e.g. dichloromethane).

According to another process (F), compounds of formula (I) in which $R^1$ represents an acyloxy, carbonate, carbamate or ether group may be prepared from compounds of formula (XIII)

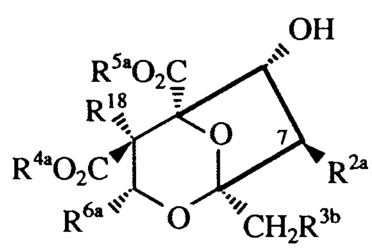

(XIII)

(wherein $R^{2a}$ is as defined for $R^2$ above or is a protected hydroxyl group, $R^{3b}$, $R^{4a}$, $R^{5a}$ and $R^{6a}$ are as defined previously and $R^{18}$ is a hydroxyl group or a protected hydroxyl group) by reaction to convert the 6 position hydroxyl group to an acyloxy, carbonate, carbamate or ether formation, followed by removal of any protecting groups present.

Thus, for example, the acylation reaction may conveniently be effected by treating a compound of formula (XIII) with an acyl halide (e.g. an acyl chloride) in the presence of 4-dimethylaminopyridine with or without a suitable base such as a tertiary amine (e.g. triethylamine) or using an alkali metal carbonate or alkaline earth metal carbonate (e.g. calcium carbonate) in a solvent such as a halogenated hydrocarbon (e.g. dichloromethane).

The reaction to form a carbonate may conveniently be effected by treating a compound of formula (XIII) with a suitable haloformate (e.g. a chloroformate) in the presence of dimethylaminopyridine with or without a suitable base such as a tertiary amine (e.g. triethylamine) or using an alkali metal carbonate or alkaline earth metal carbonate (e.g. calcium carbonate) in a solvent such as a halogenated hydrocarbon (e.g. dichloromethane).

Compounds of formula (I) in which $R^1$ represents a group $-OCONR^{14}R^{15}$ may be prepared from

compounds of formula (XIII) by reaction with a suitable carbamoylating agent under conventional conditions, followed by removal of the protecting groups present.

Thus, for example, compounds of formula (I) in which $R^1$ represents a group $-OCONR^{14}R^{15}$ (where at least one of $R^{14}$ and $R^{15}$ is a hydrogen atom) may conveniently be prepared by treating a compound of formula (XIII) with a suitable isocyanate in a solvent such as a halogenated hydrocarbon (e.g. dichloromethane) at a temperature in the range of $0^0$ to $20^0C$. When $R^1$ represents $-OCONH_2$ a compound of formula (XIII) may be reacted with an isocyanate capable of generating the desired compound under appropriate work-up conditions. Suitable isocyanates include, for example, chlorosulphonyl isocyanate and chloroacetyl isocyanate. Compounds in which $R^1$ represents $-OCONHR^{15}$ where $R^{15}$ is other than a hydrogen atom may be prepared using an isocyanate $R^{15}NCO$.

Compounds of formula (I) in which $R^1$ represents a group $-OCONR^{14}R^{15}$ may also be prepared by treating a compound of formula (XIII) with a reagent capable of converting the 6-OH group to a group -OCOX (where X is a leaving group such as a halogen atom, e.g. chlorine, or imidazole), followed by treatment with an amine $R^{14}R^{15}NH$ (including ammonia). Suitable reagents capable of effecting the desired conversion of $R^1$ to -OCOX include phosgene and carbonyldiimidazole. The reaction may conveniently be carried out in a solvent such as an ether (e.g. tetrahydrofuran) or a halogenated hydrocarbon (e.g. dichloromethane) at a reduced temperature (e.g. $-40^0$ to $0^0C$). When a reagent such as phosgene is used, the reaction is conveniently carried out in the presence of a non-nucleophilic organic base such as pyridine.

Compounds of formula (I) in which $R^1$ represents a group $-OCONR^{14}R^{15}$ where both $R^{14}$ and $R^{15}$ are other than hydrogen atoms may also be prepared from compounds of formula (XIII) by treating (XIII) with a reagent $HalCONR^{14}R^{15}$ where Hal is a halogen atom (e.g. chlorine) in a solvent such as a halogenated hydrocarbon (e.g. dichloromethane) in the presence of an organic base (e.g. pyridine).

The reaction to form an ether may conveniently be effected by treating a compound of formula (XIII) with a halide (e.g. an alkyl halide) in the presence of a suitable base such as an alkali metal hydroxide (e.g. potassium hydroxide) in a solvent such as a sulphoxide (e.g. dimethylsulphoxide).

If it is desired to prepare compounds of formula (I) in which $R^2$ represents a hydroxyl group from compounds of formula (XIII) then it may be necessary to have protected the 7-position hydroxyl group in compounds of formula (XIII). Suitable protecting groups and methods of deprotection are described hereinafter.

Compounds of formula (XII) in which $R^1$ represents an acyloxy, carbonate, carbamate or ether group may conveniently be prepared from compounds of formula (XIII) in which $R^{2a}$ represents a protected hydroxyl group using the procedure described in process (D) above, followed by removal of the protecting group(s) present.

Compounds of formula (XIII) may conveniently be prepared from compounds of formula (XIV)

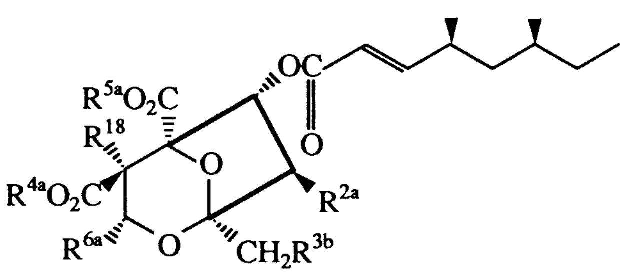

(XIV)

(wherein $R^{2a}$, $R^{3b}$, $R^{4a}$, $R^{5a}$, $R^{6a}$ and $R^{18}$ are as defined previously in formula (XIII) above) by treating (XIV) with a hydroxylamine (e.g. N-methylhydroxylamine hydrochloride) optionally in the presence of a suitable base (e.g. a trialkylamine such as triethylamine) in a solvent such as dimethylformamide.

Compounds of formula (XII) in which $R^1$ represents a hydroxyl group may also be prepared from compounds of formula (XIV) in which $R^{2a}$ and $R^{18}$ represent hydroxyl groups and $R^{3b}$, $R^{4a}$, $R^{5a}$ and $R^{6a}$ are as defined previously according to the deacylation procedure described above for the preparation of compounds of formula (XIII) from compounds of formula (XIV).

Compounds of formula (XIV) in which $R^{2a}$ and $R^{18}$ represent protected hydroxyl groups and/or $R^{3b}$ represents a protected $R^3$ group and/or $R^{6a}$ represents a protected hydroxymethyl group may conveniently be prepared from the corresponding compounds of formula (XIV) in which $R^{2a}$ and $R^{18}$ represent hydroxyl groups and/or $R^{3b}$ represents a group $R^3$ and/or $R^{6a}$ represents a hydroxymethyl group. Suitable hydroxyl protecting groups are described hereinafter.

Compounds of formula (XIV) in which $R^{2a}$ represents a group $-OCOR^7$ or $-OCO_2R^7$ may be prepared

from the corresponding compounds of formula (XIV) in which $R^{2a}$ represents a hydroxyl group according to the general procedure described hereinabove for the preparation of compounds of formula (I) from compounds of formula (XII).

Compounds of formula (XIV) in which $R^{2a}$ and $R^{18}$ represent hydroxyl groups, $R^{3b}$ represents a group $R^3$ and $R^{6a}$ represents $R^6$ may conveniently be prepared from compounds of formula (XV)

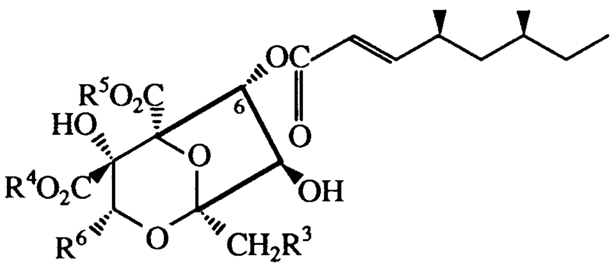

(XV)

(wherein $R^3$-$R^6$ are as defined in formula (I) above) by standard carboxylic acid protection methods.

Compounds of formula (XIII) in which $R^{2a}$ and $R^{18}$ represent hydroxyl groups, $R^{3b}$ is as defined for $R^3$ and $R^{6a}$ are presents $R^6$ may also be prepared from compounds of formula (XVI)

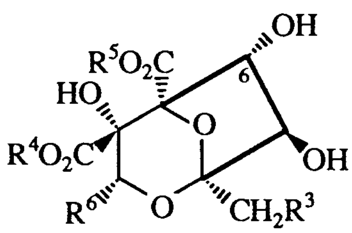

(XVI)

(wherein $R^3$-$R^6$ are as defined in formula (I) above) by standard carboxylic acid protection methods.

Suitable carboxylic acid protecting groups for $R^{4a}$ and $R^{5a}$ and hydroxyl protecting group where required include any conventional protecting group, for example as described in 'Protective Groups in Organic Chemistry', Ed. J. F. W. McOmie (Plenum Press, 1973) or 'Protective Groups in Organic Synthesis' by Theodora W. Greene (John Wiley and Sons, 1981). Examples of suitable carboxylic acid protecting groups include alkyl groups such as t-butyl, 2-methoxyethoxymethyl or aralkyl groups such as benzyl, diphenylmethyl or p-nitrobenzyl. Examples of suitable hydroxyl protecting groups include groups such as 2-methoxyethoxymethyl.

The protecting groups may be removed using conventional techniques. Thus, an alkyl group such as t-butyl may, for example, be removed under anhydrous acid conditions (for example using hydrogen chloride in a solvent such as an ether, e.g. dioxan). An aralkyl group may conveniently be removed by catalytic hydrogenation using for example a suitable metal catalyst such as palladium-on-carbon. Alternatively, a p-nitrobenzyl group may conveniently be removed using zinc metal and hydrochloric acid in a solvent such as an ether (e.g. tetrahydrofuran or aqueous tetrahydrofuran). A diphenylmethyl group or a 2-methoxyethoxymethyl group may conveniently be removed using aqueous formic acid or trifluoroacetic acid.

Compounds of formulae (XV) and (XVI) may conveniently be prepared from compounds of formula (III) by the methods described in processes (A) and (B) above.

Esterification of compounds of formula (IV) to the corresponding methyl esters may conveniently be effected by treatment with a methylating agent such as a methyl halide (e.g. methyl iodide) or dimethyl sulphate in a suitable organic solvent such as an amide (e.g. dimethylacetamide or preferably dimethylformamide) in the presence of a base such as a bicarbonate (e.g. sodium bicarbonate). The reaction may conveniently be carried out at a temperature ranging from $0^0$ to $100^0C$, preferably $20^0$ to $30^0C$. Alternatively, the esterification may be effected by treatment with an ethereal solution of diazomethane in a suitable solvent such as methanol. The esterification may also be effected by treatment with methanol in the presence of a suitable acid such as a mineral acid (e.g. hydrochloric acid) at about room temperature.

Conversion of one methyl ester of formula (IV) to a different methyl ester may be carried out by appropriate esterification/deesterification steps. The deesterification may be effected under standard conditions, for example by base hydrolysis.

Compounds of formula (IV) in which $R^1$ represents a hydrogen atom, a hydroxyl group or a group

selected from

$$-OCOCH\overset{E}{=}CHCH(CH_3)(CH_2)_3CH_3,$$

$$-OCOCH\overset{E}{=}CHC(CH_3)\overset{E}{=}CHCH(CH_3)CH_2CH_3, \text{ or } -OCO\text{-}X\text{-}CH_2CH(CH_3)CH_2CH_3$$

[where X is $-CH\overset{E}{=}CHCH(CH_3)-$, $-CH_2CH(OH)CH(CH_3)-$, $-CH\overset{E}{=}CHC(OH)(CH_3)-$, $-CH_2CH(OH)CH_2-$ or $-CH_2CH_2CH(CH_3]-$],

$R^2$ represents a hydrogen atom or a hydroxyl group and $R^3$ represents

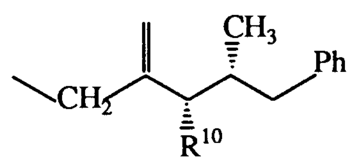

(where $R^{10}$ is a hydroxyl or acetoxy group),

$-CH_2C(CH_3)\overset{E}{=}CHCH(CH_2R^{13})CH_2Ph$, $-CH_2C(CH_2OH)\overset{Z}{=}CHCH(CH_3)CH_2Ph$,

$-CH_2C(=CH_2)CH(OH)CH(CH_2OH)CH_2Ph$,

$-CH_2C(=CH_2)CH(NHCOCH_3)CH(CH_3)CH_2Ph$,

$-CH_2C(CH_2NHCOCH_3)\overset{E}{=}CHCH(CH_3)CH_2Ph$ or

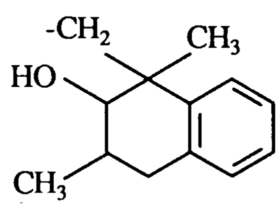

may be prepared according to the fermentation process described hereinafter or may be prepared from products of the fermentation process by acylation or deacylation at the 6-position as appropriate according to previously described acylation and deacylation methods. Other compounds of formula (IV) may be prepared from the fermented compounds of formula (IV) or acylated or deacylated derivatives thereof by modification of the $R^3$ grouping according to the general procedures described hereinabove.

Microorganisms capable of producing a compound of the formula (IV) may readily be identified by using a small scale test and analysing a test sample obtained from fermentation of the microorganism using standard methodology.

In particular the microorganism to be conventionally used is a strain of microorganism deposited on 31st May 1989 in the culture collection of Glaxo Group Research Limited, Microbiology Division, Greenford Road, Greenford, Middlesex, England, UB6 0HE (collection number 202 in the World Directory of Collections of Cultures of Microorganisms, 1982; curator: Miss A M Harris) under accession no. C2932 or a mutant thereof. It is to be understood that the above mentioned culture collection centre has given its unreserved and irrevocable consent to the microorganism deposited being made available to any person making a valid request therefor to the culture collection in accordance with Rule 17 of the UK Patents Rules 1982.

The strain deposited at Greenford under accession no. C2932 has also been deposited in the permanent culture collection of the CAB International Mycological Institute, Ferry Road, Kew, Surrey, England. The strain was received by the Institute on 25th May 1989 and was subsequently given the accession no. IMI 332962 and a deposit date of 27th June 1989 (date of confirmation of viability). The

deposited strain is identified herein by reference to the Institute accession no. IMI 332962. The characteristics thus far identified for IMI 332962 are given in Example 32 hereinafter.

It will be appreciated that the desired intermediates may also be prepared from a mutant of IMI 332962.

Mutants of the IMI 332962 may arise spontaneously or may be produced by a variety of methods including those outlined in Techniques for the Development of Micro-organisms by H. I. Adler in 'Radiation and Radioisotopes for Industrial Microorganisms', Proceedings of the Symposium, Vienna 1973, p241, International Atomic Energy Authority. Such methods include ionising radiation, chemical methods e.g. treatment with N-methyl-N'-nitro-N-nitrosoguanidine (NTG), heat, genetic techniques, such as recombination and transformation, and selective techniques for spontaneous mutants.

The production of compounds of the formula (IV) by fermentation of a suitable organism may be effected by conventional means i.e. by culturing the organism in the presence of assimilable sources of carbon, nitrogen and mineral salts.

Assimilable sources of carbon, nitrogen and minerals may be provided by either simple or complex nutrients. Sources of carbon will generally include glucose, maltose, starch, glycerol, molasses, dextrin, lactose, sucrose, fructose, galactose, myo-inositol, D-mannitol, soya bean oil, carboxylic acids, amino acids, glycerides, alcohols, alkanes and vegetable oils. Sources of carbon will generally comprise from 0.5 to 10% by weight of the fermentation medium. Fructose, glucose and sucrose represent preferred sources of carbon.

Sources of nitrogen will generally include soya bean meal, corn steep liquors, distillers solubles, yeast extracts, cottonseed meal, peptones, ground nut meal, malt extract, molasses, casein, amino acid mixtures, ammonia (gas or solution), ammonium salts or nitrates. Urea and other amides may also be used. Sources of nitrogen will generally comprise from 0.1 to 10% by weight of the fermentation medium.

Nutrient mineral salts which may be incorporated into the culture medium include the generally used salts capable of yielding sodium potassium, ammonium, iron, magnesium, zinc, nickel, cobalt, manganese, vanadium, chromium, calcium, copper, molybdenum, boron, phosphate, sulphate, chloride and carbonate ions.

Cultivation of the organism will generally be effected at a temperature of from 20 to 40$^{0}$C preferably from 20 to 35$^{0}$C, especially around 25 to 28$^{0}$C, and will desirably take place with aeration and agitation e.g. by shaking or stirring. The medium may initially be inoculated with a small quantity of mycelium and/or spores. The vegetative inoculum obtained may be transferred to the fermentation medium, or to one or more seed stages where further growth takes place before transfer to the principal fermentation medium. The fermentation will generally be carried out in the pH range 3.5 to 9.5, preferably 4.5 to 7.5. It may be necessary to add a base or an acid to the fermentation medium to keep the pH within the desired range. Suitable bases which may be added include alkali metal hydroxides such as aqueous sodium hydroxide or potassium hydroxide. Suitable acids include mineral acids such as hydrochloric, sulphuric or phosphoric acid.

The fermentation may be carried out for a period of 4-30 days, preferably about 7-18 days. An antifoam may be present to control excessive foaming and added at intervals as required. Carbon and/or nitrogen sources may also be fed into the fermentation medium as required.

Compounds of formula (IV) may be present in both the fermentation liquor and the mycelial fraction, which may conveniently be separated by filtration or centrifugation. The liquor may be optionally thereafter treated with an acid such as sulphuric acid in the presence of an organic solvent until the pH is below pH 6 (e.g. about pH 3).

Compounds of formula (IV) may be separated from the fermentation broth by conventional isolation and separation techniques. It will be appreciated that the choice of isolation techniques may be varied widely.

Compounds of formula (IV) may be isolated and purified by a variety of fractionation techniques, for example adsorption-elution, precipitation, fractional crystallisation, solvent extraction and liquid-liquid partition which may be combined in various ways.

Adsorption onto a solid support followed by elution has been found to be suitable for isolating and purifying compounds of the invention.

Compounds of formula (IV) may be extracted from the cells and the aqueous phase with an appropriate organic solvent such as a ketone (e.g. acetone, methyl ethyl ketone or methyl isobutyl ketone), a halogenated hydrocarbon, an alcohol, a diol (e.g. propane-1,2-diol or butane-1,3-diol) or an ester (e.g. methyl acetate or ethyl acetate). Generally, more than one extraction may be desirable to achieve optimum recovery. The water-immiscible solvent extracts may themselves be extracted with basic aqueous solutions, and after acidification of these basic solutions the desired compounds may be reextracted into water-immiscible organic phase. Removal of the solvent from the organic extracts (e.g. by evaporation) yields a material containing the desired compounds.

Chromatography (including high performance liquid chromatography) may be effected on a suitable support such as silica; a non-functional macroreticular adsorption resin for example cross-linked styrene divinyl benzene polymer resins such as Amberlite XAD-2, XAD-4, XAD-16 or XAD-1180 resins (Rohm & Haas Ltd) or Kastell S112 (Montedison); a substituted styrene-divinyl benzene polymer, for example a halogenated (e.g. brominated) styrene-divinyl benzene polymer such as Diaion SP207 (Mitsubishi); an anion exchanger (e.g. IRA-35 or IRA-68) an organic solvent-compatible cross-linked dextran such as Sephadex LH20 (Pharmacia UK Ltd), or on reverse phase supports such as hydrocarbon linked silica e.g. $C_{18}$-linked silica. An alternative chromatographic means for the purification/separation of compounds of formula (IV) is countercurrent chromatography using a coil extracter such as a multi-layer coil extracter.

Compounds of formula (IV) may also be isolated and purified by the use of a liquid anion exchanger such as LA 2.

When IRA-68 or, particularly, IRA-35 is used as the solid adsorbant the cell extracts may be loaded directly without removal of solvent. The extract may either be loaded directly at about pH3 or at about pH8 following filtration of solid impurities.

Suitable solvents/eluants for the chromatographic purification/ separation of compounds of formula (IV) will, of course, depend on the nature of the column type and support. When using countercurrent chromatography we have found a solvent system comprising ethyl acetate, hexane, methanol and an aqueous acid (e.g. aqueous sulphuric acid) to be particularly suitable. When using an anion exchanger such as IRA-35 the resin may conveniently be washed with aqueous acetone followed by elution with sulphuric acid in aqueous acetone.

The presence of compounds of formula (IV) during the extraction/isolation procedures may be monitored by conventional techniques such as h.p.l.c. or UV spectroscopy or by utilising the properties of the compounds.

Where a compound of formula (IV) is obtained in the form solution in an organic solvent, for example after purification by chromatography, the solvent may be removed by conventional procedures, e.g. by evaporation, to yield the required compound. If desired, the compound may be further purified by the aforementioned chromatographic techniques.

Acylation to provide a compound of formula (XV) may conveniently be effected by treating a corresponding compound of formula (XVI) or a protected derivative thereof with an acid of formula (XVII)

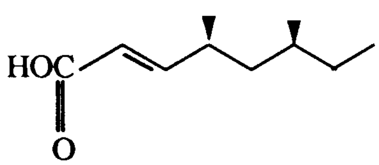

(XVII)

or an activated derivative thereof such as the corresponding acid chloride under conventional esterification conditions followed by removal of any protecting groups present. The acylation reaction may conveniently be carried out in the presence of 4-dimethylaminopyridine with or without a suitable base such as a tertiary amine (e.g. triethylamine) or using an alkali metal carbonate or alkaline earth metal carbon (e.g. calcium carbonate) in a solvent such as a halogenated hydrocarbon (e.g. dichloromethane).

The compound of formula (XVII) may conveniently be prepared by hydrolysis of a compound of formula (XV), for example by base catalysed hydrolysis using a base such as aqueous sodium hydroxide in a solvent such as an alcohol (e.g. methanol).

Deacylation of compounds of formulae (IX), (X) and (XV) may be effected by acid or base catalysed hydrolysis. Suitable bases include hydroxides such as sodium hydroxide and potassium hydroxide and alkoxides (e.g. methoxides). The base catalysed hydrolysis may take place in water optionally in the presence of an organic co-solvent such as an ether (e.g. tetrahydrofuran) or an alcohol (e.g. methanol) at a temperature in the range of $0^0$ to $100^0C$, preferably at elevated temperature. When an alkoxide is used as the base the reaction may conveniently be effected in an alcohol solvent (e.g. methanol) at a temperature in the range of $0^0$ to $100^0C$. Suitable acids include mineral acids (e.g. hydrochloric acid) and organic acids (e.g. p-toluenesulphonic acid). The acid catalysed hydrolysis may be carried out in water optionally in the presence of an organic co-solvent such as an ether (e.g. dioxan or tetrahydrofuran) or a ketone (e.g. acetone) at a temperature in the range of $0^0$ to $100^0C$, preferably at room temperature.

Compounds of formulae (IX) and (X) where $R_1$ represents a hydroxyl group may conveniently be prepared from the corresponding compounds where $R_1$ represents an acyloxy group as previously defined in formula (I) using the following procedure. Thus, for example, when $R_1$ represents a substituted 2(E)-octenoyl group this may be converted to a hydroxyl group by treatment with a hydroxylamine (e.g. N-methylhydroxylamine) in the presence of a base (e.g. an organic base such as triethylamine) in a suitable organic solvent such as an amide (e.g. dimethylformamide) at a temperature ranging from, for example, $0^0$ to $50^0C$, preferably $20^0$ to $30^0C$.

It is to be understood that the acylation or deacylation and esterification processes may be combined as sequential or simultaneous reaction steps as appropriate.

Salts of compounds of formula (I) may be conveniently formed by treating a compound of formula (I) with an appropriate salt or base. Thus, for example, salts may conveniently be prepared by treating a compound of formula (I) with a salt or a base selected from sodium or potassium hydroxide, hydrogen carbonate, carbonate or acetate (e.g. potassium hydroxide, potassium hydrogen carbonate, sodium hydrogen carbonate or potassium acetate), ammonium acetate, calcium acetate and L-lysine as appropriate. The salt may, for example, be prepared by adding the appropriate salt or base (if necessary as an aqueous solution) to a solution or suspension of the compound of formula (I) in a suitable solvent such as water and/or a cosolvent such as an alcohol (e.g. methanol), a nitrile (e.g. acetonitrile) or a ketone (e.g. acetone) at temperatures of for example $0^0C$ to $80^0C$ and conveniently at about room temperature.

Compounds of formulae (II), (V) and (VII) to (XVI) are novel intermediates and form a further aspect of the present invention.

The following examples are provided by way of illustrating the invention and are not intended to limit the invention in any way.

Intermediate 1

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane 3,4,5-tricarboxyic acid, 6-(4,6-dimethyl-2-octenoate)

(a) IMI 332962 was grown on agar plates of the following composition:

| | |
|---|---|
| Malt extract (Oxoid L39)<br>Mycological peptone (Oxoid L40)<br>Yeast extract (Oxoid L21)<br>Agar (Oxoid No 3)<br>Distilled water to 1 litre | 30g<br>5g<br>0.5g<br>20g |

The pH of the medium before autoclaving was in the range of 5.3-5.5. The inoculated plates were incubated at $28^0C$ for 14 days. Several 6mm diameter plugs of agar covered with fungal mycelium were cut from the growing edge of the culture and two plugs were transferred into each of several cryotubes containing 1.6ml of sterile distilled water. The tubes were capped and stored at room temperature until required.

Two agar plugs were used to inoculate each of eight 50ml aliquots of seed medium (A) contained in 250ml Erlenmeyer flasks :

| | | |
|---|---|---|
| Seed medium (A): | Peptone (Oxoid L34)<br>Malt extract (Oxoid L39)<br>Glycerol<br>Junlon 110 (Honeywill & Stein Ltd., Wallington, Surrey)<br>Distilled water to 1 litre | 10g<br>21g<br>40g<br>1g |

The pH of the medium was adjusted to 6.3-6.5 with aqueous sodium hydroxide before autoclaving

The flasks of inoculated seed medium were incubated at $25^0C$ on a shaker platform, which rotated at 250rpm with a 50mm diameter orbital motion, for 5 days.

The contents of the flasks were pooled and homogenised. The homogenised seed culture was used at 3% (v/v) to inoculate 120, 50ml aliquots of fermentation medium (B) in 250ml Erlenmeyer flasks :

| | | |
|---|---|---|
| Fermentation medium (B) : | Glycerol<br>Soyabean oil<br>Cottonseed flour (Sigma)<br>Distilled water to 1 litre | 50g<br>30g<br>10g |

The pH of the medium before autoclaving was in the range 6.1-6.3. The flasks were incubated as above with shaking for 8 days.

The fermentation broth (approximately 6L) from flasks incubated for 8 days was filtered to remove the mycelium and the filtrate adjusted to pH 2.8 with sulphuric acid (20% v/v) and extracted with 3 x 2 volumes of ethyl acetate. The ethyl acetate extracts were bulked and back extracted with 2 x 400ml of aqueous sodium hydrogen carbonate solution (1% w/v). The aqueous back extracts were bulked, adjusted to pH 2.8 as above and re-extracted into 2 x 800ml of ethyl acetate. These extracts were combined and evaporated to dryness to yield a brown oil. This oil was further processed by countercurrent chromatography using an Ito Multi-layer Coil Extractor (P. C. Inc., Potomac, Maryland, USA). The coil used was the standard preparative coil consisting of approximately 70 metres of 2.6mm internal diameter PTFE tubing giving a total volume of about 380ml. The solvent system used was a mixture of ethyl acetate, hexane, methanol and N/100 sulphuric acid (6:5:5:6 by volume). The lower phase was kept stationary. The coil was filled with the lower phase using a Gilson Model 303 pump and a Model 804C Manometric Module (Gilson, Villiers Le Bel, France). The oil (497mg in 4ml of the upper phase +4ml of the lower phase) was then injected at the "tail" end of the column. The centrifuge was then operated at 800 rev./min. and the mobile (upper) phase pumped at 4ml/min. from the "tail" end of the column. 20ml fractions were collected and monitored by measuring inhibition of squalene synthase.

Consecutive fractions showing activity against squalene synthase were bulked. The earlier fractions were evaporated to dryness to yield the title compound (90mg) as a pale yellow oil.

(b) The mycelium separated from 6L broth, from flasks incubated for 8 days according to the procedure in part (a) above, was extracted with methanol (2 x 3L) and filtered. The filtrate was concentrated by evaporation to ca. 500ml, adjusted to pH 3.0 with formic acid and extracted with 3 x 500ml of ethyl acetate. The ethyl acetate extracts were bulked and back extracted with 2 x 200ml of sodium hydrogen carbonate solution (1% w/v). The aqueous back extracts were bulked, adjusted to pH 3.0 and re-extracted into 2 x 500ml of ethyl acetate. All the organic fractions were combined and reduced to dryness using a rotary evaporator to yield a brown oil. The oil (578mg) was further processed by high performance liquid chromatography (HPLC) using a Gilson autopreparative system composed of 3 Gilson solvent delivery pumps (model 303), an 811 Dynamic mixer and an 802C manometric module. The chromatography was carried out on a Dynamax Microsorb C18 (5$\mu$m) semi-preparative column (250 x 10mm). The mobile phase was a gradient composed of acetonitrile and 0.1% v/v formic acid to pH 3.15 with ammonium acetate (1:3 → 4:1 → 1:3) pumped at 2.8-5.6ml/min with a run time of 65 minutes. This method was repeated 16 times. 13 x 4.95 minute fractions were collected and monitored by measuring inhibition of squalene synthase. Fraction number 5 from each run was bulked, acidified to pH 3.0 with formic acid and extracted with 2 x 100ml ethyl acetate. The organic phase was removed and evaporated to dryness to yield the title compound (172mg) as a pale yellow oil.

(c) (i) Eight 0.5ml aliquots from a 5 day old fermentation carried out as in part (a) above were used to inoculate eight 50ml aliquots of seed medium (A) contained in 250ml Erlenmeyer flasks. The flasks were incubated at 25$^0$C on a shaker platform, which rotated at 250rpm with a 50mm diameter orbital motion, for 4 days. The contents of the flasks were pooled and homogenised.

The homogenised seed culture was used at 3% (v/v) to inoculate 120, 50ml aliquots of fermentation medium (B) in 250ml Erlenmeyer flasks. The flasks were incubated with shaking as above for 10 days.

(c) (ii) Homogenised seed culture prepared as in part (c)(i) above were used at 3% (v/v) to inoculate two fermentation vessels, each of 5 litres capacity, containing 3 litres of fermentation medium (B). The inoculated medium was maintained at 25$^0$C and agitated with two six bladed turbine impellers (70mm diameter) rotating at 500 rpm. The culture was aerated by sparging with sterile air at 3 Lpm. Provision was made for control of excessive foaming of the culture by the addition of silicone antifoam (Dow Corning 1520). The contents of the two culture vessels were combined after 11 days growth and further processed by countercurrent chromatography according to the procedure in part (a) above to give the title compound (137mg); 500MHz proton nmr in deutero-methanol includes signals at about $\delta$ 0.84-0.90 (m,9H), 1.03 (d,7,3H), 1.09-1.19 (m,2H), 2.10 (s,3H), 2.24 (m,1H), 2.34 (m,1H), 2.68 (dd,13,6,1H), 4.04 (d,2,1H), 4.97 (s,1H), 5.02 (s,1H), 5.08 (d, 5,1H), 5.27 (s,1H), 5.80 (d,16,1H), 6.31 (d,2,1H), 6.85 (dd,16,8,1H), 7.14 (t,7,1H), 7.19 (d,7,2H), 7.26 (t,7,2H); composite pulse decoupled 125.75 MHz carbon-13 nmr in deutero-methanol includes peaks at about δ 172.5 (0), 172.1(0), 170.1(0), 168.5(0), 166.5(0), 157.6(1), 147.7(0), 141.6(0), 130.2(1), 129.3 (1), 126.9 (1), 119.8 (1), 111.5 (2), 106.8 (0), 91.1 (0), 82.5 (1), 81.0 (1), 80.1 (1), 76.6 (1), 75.6 (0), 44.4 (2), 40.9 (2), 37.7 (1), 35.6 (1), 34.9 (2), 33.1 (1), 30.8 (2), 26.5 (2), 20.9 (3), 20.5 (3), 19.2 (3), 14.1 (3), 11.4 (3).

(d) (i) Frozen stocks of inoculum were prepared from a 5 day old fermentation carried out as in part (a) above. Samples of culture were centrifuged for 10 min and the mycelium resuspended to the original volume in 15% glycerol and 0.01% Tween 80. The mycelium was spun down and resuspended again before being distributed in 1.8ml amounts in plastic tubes and stored at $^{-}20^{0}$C. Eight 0.5ml aliquots of frozen inoculum were used to inoculate eight 50ml aliquots of seed medium (A) contained in 250ml Erlenmeyer flasks. The flasks were incubated at $25^{0}$C on a shaker platform, which rotated at 250rpm with a 50mm diameter orbital motion, for 4 days. The contents of the seed flasks were pooled and used at 3% (v/v) to inoculate 120 50ml aliquots of fermentation medium (B) in 250 ml Erlenmeyer flasks. The flasks were incubated with shaking as above for 9 days.

(d) (ii) The contents of 4 final stage flasks grown as in part (d)(i) above were pooled after 7 days incubation and homogenised to provide the seed for 120 50ml aliquots of fermentation medium (B) which were incubated for 8 days as in parts (c)(i) and (d)(i) above. The fermentation broth (approximately 6L) from flasks incubated for 8 days was filtered to remove the mycelium. The filtrate was adjusted to pH 2.8 with sulphuric acid (20% v/v) and extracted into ethyl acetate, back extracted into sodium hydrogen carbonate and re-extracted into ethyl acetate at pH 2.8 as described in part (a) above. The ethyl acetate extract was concentrated under reduced pressure to a yellow oil which was dissolved in methanol (10ml). This solution was evaporated to 3ml and applied to a column (32 x 2.5cm) of ODS-3 (Whatman Partisil Bioprep 40,75 Angstrom, slurry packed in acetonitrile-water, 20:80). The column was eluted with a stepwise gradient of a mixture of acetonitrile and water, increasing the proportion of acetonitrile as follows : 1:4, 3:7, 2:3, 1:1, 3:2. Fractions were monitored by HPLC and those containing the title compound were evaporated to remove acetonitrile. The resulting aqueous suspensions were pooled and freeze dried overnight to yield the title compound (59mg) as an off-white solid.

(e) The procedure in part (d)(i) was followed except that the pooled seed flasks were used at 3% (v/v) to inoculate 4 litres of seed medium (A) in a 7L fermenter. The culture was incubated with agitation as above at 500rpm for 2 days with the culture aerated at 4L/min. 1.2L of the culture was removed and used to inoculate a 70L fermenter filled with 40L seed medium (A). The culture was incubated as above at 500rpm for 2 days with the culture aerated at 40L/min. 15L of the culture was removed and added to a 780L fermenter filled with 500L fermentation medium (C).

| | | |
|---|---|---|
| Fermentation medium (C) : | Fructose<br>Soyabean oil<br>Cottonseed flour (sigma) | 50g<br>30g<br>20g |
| Natural pH | | |

The culture was incubated with shaking as above at 200rpm for 450h with the culture aerated at 500L/min and fed at 120h with a 50% (w/v) solution of fructose at 5g/L/day increasing to 7.5g/L/day at 162h. Analysis of the broth at 450h indicated a yield of the title compound of 1056 mg/L.

The above procedure was repeated on a reduced scale but replacing fructose with other sources of carbon selected from glucose, galactose, sucrose, maltose, lactose, myo-inositol, D-mannitol and soyabean oil. Analysis of the broth from each experiment at 450h indicated a substantial titre of the title compound.

The title compound prepared according to the above procedures was consistent with a product having the following characterising features :

Approximate molecular weight 690; -FAB mass spectrometry [M-H]-689.2789; +FAB mass spectrometry [M+Na}+ 713.2753; Molecular formula $C_{35}H_{46}O_{14}$.

500 MHz proton nmr spectrum in deutero-chloroform [δ values with multiplicities, coupling constants ($H_z$) and integration values in parenthesis] : 0.79 to 0.85 (m,9H), 1.00 (d,7,3H), 1.04 to 1.15 (m,2H), 2.09 (s,3H), 2.40 (m,1H), 2.69 (dd,13,5,1H), 4.05 (s,1H), 4.94 (s,1H), 4.96 (s,1H), 5.06 (d,4,1H), 5.30 (s,1H), 5.78 (d,16,1H), 5.92 (s,1H), 6.88 (dd,16,8,1H), 7.11 (d,7,2H), 7.14 (t,7,1H), 7.24 (t,7,2H).

Composite pulse decoupled 125.75MHz carbon-13 nmr spectrum in deutero-chloroform [δ values with the number of attached protons in parenthesis] : 171.5 (0), 171.0 (0), 169.1 (0), 167.0 (0), 166.7 (0), 157.9 (1), 145.4 (0), 140.1 (0), 128.9 (1), 128.1 (1), 125.8 (1), 117.8 (1), 111.4 (2), 105.8 (0), 88.5 (0), 81.6 (1), 80.7 (1), 79.3 (1), 75.1 (1), 74.2 (0), 42.9 (2), 39.7 (2), 36.7 (1), 34.2 (1), 33.6 (2), 31.6 (1), 29.4 (2), 25.4 (2), 20.9 (3), 19.8 (3), 18.8 (3), 13.5 (3), 10.9 (3).

Intermediate 2

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxyic acid, 6-(4,6-dimethyl-2-octenoate),3,4,5-trimethyl ester

A solution of the freeze dried product of Intermediate 1 (940mg) in methanol (15ml) was treated with a solution of diazomethane in diethyl ether (0.4M; 16ml). The excess diazomethane was quenched with acetic acid (0.1ml) and the solution was concentrated under reduced pressure. The residue was chromatographed on silica gel (Merck 7734, 50g) eluting with dichloromethane increasing to 2% methanol/dichloromethane to give the title compound (906mg); δ ($CDCl_3$) includes 0.8-0.9 (m,$CH_3$), 1.04 (d,J7Hz,CH=CHCHC$H_3$), 2.09 (s,$CH_3CO_2$), 3.76, 3.81 and 3.93 (3s, $CO_2CH_3$), 4.05 (d,J2Hz,7-H), 4.98 and 5.00 (2s, C=$CO_2$), 5.10 (d,J6Hz,CHOAc), 5.26 (s,3-H), 5.75 (d,J16Hz,CH=CH$Co_2$), 5.81 (d,J2Hz,6-H), 6.84 (dd,$J_1$ 16Hz,$J_2$ 8.5Hz,CH=CH$CO_2$), 7.13-7.28 (m,aromatic).

Intermediate 3

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxyic acid, 6-(4,6-dimethyl-2-oxtenoate), 4,5-dimethyl ester

A solution of Intermediate 2 (173mg) in tetrahydrofuran (5 ml) at room temperature was treated with aqueous sodium hydroxide (0.1N, 2.36ml). After 10mins, most organic solvent was removed in vacuo. The resultant aqueous solution was diluted with water (20ml) and washed with ether (2x). The aqueous solution was made acidic with 0.5M aqueous hydrochloric acid, extracted with ethyl acetate (3x), dried over magnesium sulphate and filtered. Removal of solvent gave the title compound as a light brown gum (145.2mg); δ ($CDCl_3$) values include 7.1-7.3 (m,5H,Ph), 6.85 (dd,1H,CH=CH$CO_2$,J=15.5 and 8.75Hz), 5.81 (d,1H,CHOCOCH=CH,J=2Hz), 5.75 (d,1H,CH=CH$CO_2$,J=15.5Hz), 5.22 (s,1H,CH$CO_2$H), 5.08 (d,1H,AcOCH,J=5Hz), 5.01 (s,2H,C=$CH_2$), 5.0 (broad s,1H,OH), 4.06 (d,1H,CHOH,J=2Hz), 3.93 and 3.79 (2s,6H,2$CO_2$Me), 2.69 (dd,1H,one of PhCH,J=13.7 and 6.2Hz), 2.1 (s,3H,$CH_3CO_2$),1.05 (d,3H,CH=CHCHC$H_3$,J-7Hz),0.8-0.9 (m,9H,3C$H_3$).

Intermediate 4

[1S-[1α(4R*,5S*),3α,4β,5α,6α(2E,4R*,6R*),7β]] 1-(4-Acetyloxy-5-methyl-3-methylene-6-phenylhexyl)-4,5-bis-(methoxycarbonyl)-4,6,7-trihydroxy-2,8-dioxabicyclo[3.2.1]octane-3-peroxycarboxylic acid, 6-(4,6-dimethyl-2-octenoate), 1,1-dimethylethyl ester

A solution of Intermediate 3 (5.952g) in a mixture of dry dichloromethane (20ml) and acetonitrile (10ml) was added to the Vilsmier salt [prepared by dropwise addition of oxalyl chloride (1.3ml) to a dichloromethane (20ml) solution of dry dimethylformamide (0.77ml) at 0°C, stirred for 30 mins and evaporated to dryness] at 0°C under nitrogen. After 1.5h of stirring, tert-butyl hydroperoxide (3M in 2,2,4-trimethylpentane, 6.1ml) followed by 4-dimethylaminopyridine (0.1g) and dry triethylamine (3.5ml) were added. The mixture was stirred at 0°C to room temperature for 18h, quenched with aqueous saturated sodium hydrogen carbonate, extracted with ethyl acetate (3x), dried over magnesium sulphate and filtered. Removal of solvent gave a foam which was subjected to flash column chromatography ($SiO_2$/1:4 to 2:3 ethyl acetate-cyclohexane) to give the title compound (4.022g) as a white foam; δ ($CDCl_3$) values include 0.8-0.9-(m,9H,3Me), 1.1(d,3H,C=CCHMe,J=7Hz), 1.3(s,9H,tBu), 2.1(s,3H,$CH_3$CO), 2.7(dd,1H,one proton of Ph$CH_2$,J=13.2 and 5.7Hz), 3.27(d,1H,CHOH,J=2Hz), 3.8(s,3H,$CO_2$Me), 3.94(s,3H,$CO_2$Me), 3.96-(s,1H,HOC$CO_2$Me), 4.3(broad t,1H,CHOH,J=2Hz), 4.96 and 4.99(2s,2H,C=$CH_2$), 5.12(d,1H,CHOAcJ=5Hz), 5.38(s,1H,CH$CO_3$tBu), 5.75(d,1H,CH=CH$CO_2$,J=15.5Hz), 5.78(s,1H,CH$O_2$CCH=CH), 6.85-(dd,1H,CH=CH$CO_2$,J=15.5 and 8Hz), 7.1-7.3(m,5H,Ph).

Intermediate 5

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, tripotassium salt

A suspension of the product of Intermediate 1(d)(ii) (1g) in water (100ml) was treated with a solution of potassium bicarbonate (430mg) in water (10ml). The resulting solution was subjected to freeze drying to give the title compound (1.08g) as a beige coloured solid; $\nu_{max}$ (Nujol), 3491-3167 (broad OH), 1731 (ester C=O), 1614cm$^{-1}$ (carboxylate C=O and C=C);

| | |
|---|---|
| Analysis Found: | C,48.65; H,5.70; K,14.1; $H_2O$,6.2%. |
| $C_{35}H_{43}K_3O_{14}.3H_2O$ requires: | C,48.93;H,5.75;K,13.65;$H_2O$,6.29%. |

Intermediate 6

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), 4,5-bis(1,1-dimethylethyl)ester, 3-methyl ester

A stirred suspension of Intermediate 5 (15.6g) in methanol (1L) was treated dropwise with concentrated hydrochloric acid (13ml). The resulting clear solution was stirred at room temperature for 24h. It was then treated with solid sodium hydrogen carbonate (13.2g) and most of the solvent was evaporated under reduced pressure. The residue was treated with aqueous hydrochloric acid (2M; 500ml) and extracted with ethyl acetate (1Lx3). The organic extract was washed with water (1L), dried over magnesium sulphate, filtered and evaporated. The residue was dissolved in dry toluene (130ml), heated to 80°C under nitrogen and then treated dropwise with N,N-dimethylformamide di-t-butyl acetal (38ml) over 30mins. The reaction mixture was stirred at 80°C for 3¼h and then allowed to cool. It was diluted with ether (700ml) and washed with brine (600ml). The organic layer was dried over magnesium sulphate and the solvent was evaporated under reduced pressure. The residue subjected to flash column chromatography on silica gel (Merck 9385, 900g) eluting with 5:1 to 1:1 cyclohexane:ethyl acetate. The appropriate fractions were combined and the solvent was evaporated to give the title compound (5.93g) as a yellow foam; δ ($CDCl_3$) values include 0.8-0.9 (m,9H,$3CH_3$), 1.05 (d,3H,=$CHCHCH_3$,J=6.2Hz), 1.6 and 1.48 (2s,18H,$2CO_2C(CH_3)_3$), 2.1 (s,3H,$CH_3CO_2$), 2.71 (dd, 1H, one of PhCH,J=13.7 and 5Hz), 2.96 (d,1H,CHOH,J=2Hz), 3.73 (s,3H,$CO_2CH_3$), 4.1 (s,1H,OH), 4.05 (t,1H,CHOH,J=2Hz), 4.97 (s,2H,C=$CH_2$), 5.1 (d,1H,AcOCH,J=5Hz), 5.26 (s,1H,$CHCO_2CH_3$), 5.77 (d,1H,CH=$CHCO_2$,J=16.2Hz), 5.97 (d,1H,CHOCOCH=CH,J=2Hz), 6.91 (dd,1H,CH=$CHCO_2$,J=16.2 and 8.7Hz), 7.1-7.3 (m,5H,Ph);
t.l.c. ($SiO_2$) ethyl acetate/cyclohexane (1:1) Rf 0.53.

Intermediate 7

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), 4,5-bis(1,1-dimethylethyl) ester

A stirred solution of Intermediate 6 (1g) in tetrahydrofuran (25ml) at 0°C was treated with sodium hydroxide (1.025M; 1.3ml). The reaction mixture was stirred at 0°C for 4h and then acidified with 2M aqueous hydrochloric acid (1.2ml). The reaction mixture was concentrated under reduced pressure and the residue was partitioned between ethyl acetate (125ml) and aqueous hydrochloric acid (2M; 50ml). The organic layer was washed with water (50ml), then dried over magnesium sulphate and filtered. The removal of the solvent under reduced pressure gave the title compound as a colourless foam (952mg); δ($CD_3OD$) values includes 0.8-0.9 (m,9H,$3CH_3$), 1.02 (d,3H,=$CHCH(CH_3)$), 1.61 and 1.42 (2s,18H,$2CO_2C(CH_3)_3$), 2.1 (s,3H,$CH_3CO_2$), 2.7 (dd,1H,one of PhCH,J=13.7 and 6.2Hz), 4.09 (d,1H,CHOH,J=2Hz), 5.0 (s,2H,C=$CH_2$), 5.08 (d,1H,AcOCH,J=5Hz), 5.26 (s,1H,$CHCO_2H$), 5.82 (d,1H,CH=$CHCO_2$,J=15Hz), 6.43 (d,1H,CHOCOCH=CH,J=2Hz), 6.9 (dd,1H,CH=$CHCO_2$,J=15 and 8.7Hz), 7.1-7.3 (m,5H,Ph);

| | |
|---|---|
| Analysis found: | C,64.64%,H,7.80%; |
| $C_{43}H_{62}O_{14}$ requires: | C,64.32%,H,7.78%. |

Intermediate 8

[1S-[1α(4R*,5S*),3α,4β,5α,6α(4S*,6R*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate), 4,5-bis(1,1-dimethylethyl) ester

To a solution of Intermediate 7 (500mg) and N-hydroxysuccinimide (78mg) in dry tetrahydrofuran (5ml) at room temperature, 1-cyclohexyl-3-(2-morpholinoethyl)carbodiimide metho-p-toluenesulphonate (290mg) was added. After 2h further quantities of N-hydroxysuccinimide (40mg) and 1-cyclohexyl-3-(2-morpholinoethyl)carbodiimide metho-p-toluene-sulphonate (148mg) were added. The mixture was stirred for a further 3.5h when sodium borohydride (108mg) was added. After stirring at room temperature for 1h the reaction was kept unstirred for 17h at ~4°C followed by a further period of 7h, stirring at room temperature. The reaction mixture was treated with aqueous citric acid (10% w/v; 60ml) and shaken with ethyl acetate (3x50ml) and the combined organic phases washed with saturated brine (4x30ml), dried over magnesium sulphate, filtered and evaporated. The resultant brown gum was partially purified by column chromatography using silica gel, eluting with ethyl acetate-cyclohexane (1:2) and further purified by preparative reverse-phase HPLC using a 1 inch spherisorb ODS-2 column eluting with 90% acetonitrile/water. Appropriate fractions were evaporated to dryness under reduced pressure to give the title compound as a white solid (0.0637g); δ ($CDCl_3$) values include: 0.8-0.9(m,12H,$4CH_3$), 1.50 and 1.60(2s,18H,$2CO_2$t-Bu), 2.1-(s,3H,$CH_3CO_2$), 2.69(dd,1H one of PhCH,J=13.7 and 6.2Hz), 3.7(b,2H,$CH_2$OH), 3.88(s,1H,HOC$CO_2$), 3.97-(b,1H,CHOH), 4.52(t,1H,CH$CH_2$OH,J=5Hz), 4.97(2s,2H,C=$CH_2$), 5.10(d,1H,AcOCH,J=5Hz), 5.83-(d,1H,CHOCO$CH_2CH_2$,J=2Hz), 7.10-7.30(m,5H,Ph).

| | | |
|---|---|---|
| Analysis Found: | C,65.43%; | H,8.68%, |
| $C_{43}H_{66}O_{13}$ requires: | C,65.30%; | H,8.41%. |

Intermediate 9

[1S-[1α(4R*,5S*),3α,4β,5α,6α(2E,4R*,6R*),7β]] 1-(4-Acetyloxy-5-methyl-3-methylene-6-phenylhexyl)-4,6-dihydroxy-7-[(2-methoxyethoxy)methoxyl-3-[[(2-methoxyethoxy)methoxy]methyl]-2,8-dioxabicyclo[3.2.1]-octane-4,5-dicarboxylic acid, 6-(4,6-dimethyl-2-octenoate), 4,5-dimethyl ester

A solution of the product of Example 5 hereinafter (3.452g) in dichloroethane (55ml) was treated with N,N-diisopropylethylamine (4.3ml) followed by 2-methoxyethoxymethyl chloride (2.80ml). The mixture was heated at 85°C for 20h then allowed to cool. The solution was diluted with ethyl acetate (300ml) and was then washed successively twice with aqueous hydrochloric acid (2M, 250ml), twice with saturated sodium hydrogen carbonate solution (250ml), and once with water (300ml), then dried ($MgSO_4$), filtered and concentrated in vacuo to a light brown gum. This was purified by flash column chromatography on silica gel eluting with ethyl acetate:cyclohexane [(3:7), (9:11), (1:1)] and appropriate fractions were combined and concentrated to give the title compound (3.23g) as a colourless film; δ ($CDCl_3$) includes 1.02 (d,J7Hz,=CHCH($CH_3$)), 2.09 (s,$O_2CCH_3$), 2.71 (dd,J13 and 5Hz,CHPh), 3.34 and 3.39 (2s,2x$OCH_2CH_2OCH_3$), 3.69 and 3.87 (2s,2x$CO_2CH_3$), 4.62, 4.82 and 4.92 (s and 2d,7JHz,2x$OCH_2$O), 4.62-4.72 (m,3-H), 4.95 and 4.99 (2s,C=$CH_2$), 5.11 (d,J5Hz,CHOAc), 5.72 (d,J16Hz,$O_2$CCH=CH), 6.42 (d,J2Hz,6-H) 6.82 (dd, J16 and 8Hz,$O_2$CCH=CH), 7.1-7.3 (m,$C_6H_5$).

| | |
|---|---|
| Analysis found: | C,61.44;H,8.10 |
| $C_{45}N_{68}O_{17}$ requires: | C,61.35;H,7.78%. |

Intermediate 10

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-3-[[(2-methoxyethoxy)methoxy]methyl]-2,8-dioxabicyclo[3.2.1]octane-4,5-dicarboxylic acid, 4,5-dimethyl ester

A solution of Intermediate 9 (3.00g) in dry dimethylformamide (40ml) was treated with N-methylhydroxylamine hydrochloride (0.568g) and triethylamine (2.9ml), and the mixture heated at 55°C for 8h, then

allowed to cool. The mixture was diluted with water (100ml) and saturated sodium chloride solution (100ml), then extracted twice with ethyl acetate (400ml). The combined organic extracts were washed with aqueous hydrochloric acid (2M, 300ml), then dried ($MgSO_4$), filtered and concentrated in vacuo to a colourless gum. This was purified by flash column chromatography on silica gel eluting with ethyl acetate:cyclohexane [-(3:7), (1:1), (1:0)] and appropriate fractions were combined and evaporated to give the title compound - (2.12g) as a clear colourless gum; δ($CDCl_3$) includes 2.09 (s,$O_2CCH_3$), 2.70 (dd,J13 and 5Hz,CHPh), 3.38 and 3.40 (2s,2x$OCH_2CH_2OCH_3$), 3.80 and 3.84 (2s,2x$CO_2CH_3$), 4.54 (dd,J7 and 5Hz,3-H), 4.63, 4.73 and 4.88 (s and 2d,J7Hz,2x$OCH_2O$), 4.95 and 4.99 (2s, C = $CH_2$), 5.09 (d,J5Hz,CHOAc), 5.19 (dd,J5 and 2Hz,6-H), 7.1-7.3 (m,$C_6H_5$).

| | |
|---|---|
| Analysis found: | C,56.95;H,7.37 |
| $C_{35}H_{52}O_{16}$.0.4 $H_2O$ requires : | C,57.11;H,7.23%. |

Intermediate 11

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-3-[[(2-methoxyethoxy)methoxy]methyl]-2,8-dioxabicyclo[3.2.1]octane-4,5-dicarboxylic acid, 6-heptanoate, 4,5-dimethyl ester

A solution of Intermediate 10 (0.466g) in dry dichloromethane (15ml) was treated with 4-dimethylaminopyridine (1 small spatula full) and heptanoic anhydride (0.50ml). After 2h at room temperature, a further portion of heptanoic anhydride (0.17ml) was added, and after an additional 1.5h at room temperature the mixture was treated with saturated aqueous sodium hydrogen carbonate solution (10ml). The mixture was extracted twice with ethyl acetate (15ml) and the combined organic phases dried ($MgSO_4$), filtered and concentrated in vacuo to a clear colourless gum. This was purified by flash column chromatography on silica gel eluting with ethyl acetate:cyclohexane [(1:4), (1:1), (13:7), (17:3)] and appropriate fractions were combined and evaporated to give the title compound (0.574g) as a clear colourless liquid; δ-($CDCl_3$) includes 2.09 (s,$O_2CCH_3$), 2.71 (dd,J13 and 5Hz,CHPh), 3.34 and 3.38 (2s,2x$OCH_2CH_2OCH_3$), 3.73 and 3.86 (2s,2x$CO_2CH_3$), 4.02 (d,J2Hz,7-H), 4.61, 4.78 and 4.89 (s and 2d,J7Hz,2x$OCH_2O$), 4.66 (dd,J7 and 5Hz,3-H), 4.95 and 5.00 (2s,C = $CH_2$), 5.10 (d,J5Hz,CHOAc), 6.40 (d,J2Hz,6-H), 7.1-7.3 (m,$C_6H_5$).

Intermediate 12

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-3-[[(2-methoxyethoxy)methoxy]methyl]-2,8-dioxabicyclo[3.2.1]octane-4,5-dicarboxylic acid, 6-nonanoate, 4,5-dimethyl ester

A solution of Intermediate 10 (0.282g) in dry dichloromethane (11ml) was treated with 4-dimethylaminopyridine (½ small spatula full), triethylamine (0.12ml) and nonanoyl chloride (0.071ml), and the mixture stirred at room temperature. A further portion of 4-dimethylaminopyridine (1 small spatula) was added after 1h, and after a further 17h and then 1.5h respectively, additional portions of nonanoyl chloride (0.032ml and 0.019ml) were added. After a total of 22h at room temperature, the mixture was treated with saturated aqueous sodium hydrogen carbonate solution (20ml). The mixture was extracted twice with ethyl acetate (50ml) and the combined organic phases dried ($MgSO_4$), filtered and concentrated in vacuo to a clear colourless gum. This was purified by flash column chromatography on silica gel eluting with ethyl acetate:cyclohexane [(1:4), (3:7), (2:3), (1:1), (3:2)] and appropriate fractions were combined and evaporated to give the title compound (0.300g) as a clear colourless film; δ($CDCl_3$) includes 2.09 (s,$O_2CCH_3$), 2.71 (dd,J13 and 5Hz, CHPh), 3.35 and 3.39 (2s,2x$OCH_2CH_2OCH_3$), 3.74 and 3.97 (2s,2x$CO_2CH_3$), 4.02 (d,J2Hz,7-H), 4.61, 4.79 and 4.90 (s and 2d,J7Hz,2x$OCH_2O$), 4.67 (dd,J7 and 5Hz,3-H), 4.95 and 4.99 (2s,C = $CH_2$), 5.10 (d,J5Hz,CHOAc), 6.40(d,J2Hz,6-H), 7.1-7.3 (m,$C_6H_5$).

| | |
|---|---|
| Analysis found: | C,60.98;H,8.14 |
| $C_{44}H_{68}O_{17}$ requires: | C,60.81;H,7.89%. |

Intermediate 13

[1S-[1α(4R*,5S*),3α,4β,5α,6α(4R*S*),7β]] 1-(4-Acetyloxy-5-methyl-3-methylene-6-phenylhexyl)-4,6,dihydroxy-7-[(2-methoxyethoxy)methoxy]-3-[[(2-methoxyethoxy)methoxy]methyl]-2,8-dioxabicyclo[3.2.1]octane-4,5-dicarboxylic acid, 6-(4-methyl)pentanoate, 4,5-dimethyl ester

A solution of Intermediate 10 (0.298g) in dry dichloromethane (14ml) was treated with 4-dimethylaminopyridine (1 small spatula full), 1-cyclohexyl-3-(2-morpholinoethyl)carbodiimide metho-p-toluenesulfonate (0.382g) and 4-methylpentanoic acid (0.112ml). After 3.5h, the mixture was diluted with ethyl acetate (50ml) and washed successively three times with water (30ml), and once with saturated sodium chloride solution (30ml), then dried ($MgSO_4$), filtered and concentrated in vacuo to a clear gum. This was purified by flash column chromatography on silica gel eluting with ethyl acetate:cyclohexane [(1:4), (9:11), (3:1)] and appropriate fractions were combined and evaporated to give the title compound (0.206g) as a clear colourless gum; δ($CDCl_3$) includes 2.10 (s,$O_2CCH_3$), 2.72 (dd,J13 and 5Hz,CHPh), 3.35 and 3.39 (2s,2x$OCH_2CH_2OCH_3$), 3.75 and 3.87 (2s,2x$CO_2CH_3$), 4.03 (d,J2Hz,7-H), 4.62, 4.79 and 4.89 (s and 2d,J7Hz,2x$OCH_2O$), 4.66 (dd,J7½ and 5Hz,3-H), 4.95 and 5.00 (2s,C=$CH_2$), 5.10 (d,J5Hz,CHOAc), 6.40 (d,J2Hz,6-H), 7.1-7.3 (m,$C_6H_5$).

Example 1

[1S-[1α(4R*,5S*),4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-4,5-dicarboxylic acid, 6-(4,6-dimethyl-2-octenoate), 4,5-dimethyl ester

A mixture of Intermediate 4 (2.97g) and tert-butyl mercaptan (50ml) in toluene (420ml) was irradiated with a 125W medium pressure mercury lamp in a pyrex immersion-well reactor under nitrogen for 31h at room temperature. The reaction mixture was rotary-evaporated to give an oil which was subjected to multiple flash column chromatography ($SiO_2$/1:4 to 2:3 ethyl acetate-cyclohexane) to give the title compound as a white solid (742.4mg); δ ($CDCl_3$) 0.82-0.90(m,9H,3Me), 1.05(d,3H,$O_2CCH$=CHCHMe,J=7Hz), 1.09-1.20(2H) and 1.24-1.43(3H)[2m,MeCH$CH_2$CH$CH_2$Me], 1.97-2.15(m,3H,Ph$CH_2$CHMe and $CH_2CH_2C$=$CH_2$), 2.09(s,3H,$CH_3CO_2$), 2.20-2.49(m,3H,$CH_2C$=$CH_2$ and $O_2CCH$=CHCHMe), 2.36(dd,1H,one proton of Ph$CH_2$,J=13 and 9.5Hz), 2.69(dd,1H,other proton of Ph$CH_2$,J=13 and 7Hz), 3.28-(d,1H,CHOH,J=2.5Hz), 3.80 and 3.88(2s,6H,2$CO_2$Me), 3.84(s,1H,HOC$CO_2$Me), 3.86(d,1H,one proton of $CH_2O$,J=13Hz), 4.03(t,1H,CHOH,J=2.5Hz), 4.61(d,1H,other proton of $CH_2O$,J=13Hz), 4.95 and 4.99-(2s,2H,C=$CH_2$), 5.09(d,1H,CHOAc J=5Hz), 5.75(d,1H,$O_2C$CH=CH,J=15.5Hz), 5.78-(d,1H,$CHO_2C$CH=CH,J=2.5Hz), 6.84(dd,1H,$O_2CCH$=CH,J=15.5 and 9Hz), 7.1-7.21 and 7.23-7.29-(2m,5H,Ph); δ ($^{13}C$, $CDCl_3$) 11.10($CH_3CH_2$), 13.87($CH_3$CH$CH_2$Ph), 18.94($CH_3$CHEt), 20.23-($O_2CCH$=CHCHMe), 21.02(Me$CO_2$), 25.55($CH_2C$=$CH_2$), 29.75($CH_2$Me), 31.99(EtCHMe), 34.53-($O_2CCH$=CHCH), 36.93(Ph$CH_2$CH), 40.00(Ph$CH_2$), 43.26(MeCH$CH_2$CHMe), 52.70(OC$CO_2$Me), 53.44-(HOC$CO_2$Me), 69.00($CH_2O$), 74.12(HOC$CO_2$Me), 78.95(CHOAc), 82.02($CHO_2C$CH=CH), 82.13(CHOH), 88.32(OC$CO_2$Me), 106.18(OCO), 111.53(C=$CH_2$), 118.16(CH=$CHCO_2$), 125.96(para C of Ph), 128.30(2 meta C of Ph), 129.11(2 ortho C of Ph), 140.35(quaternary C of Ph), 145.81(C=$CH_2$), 157.64(CH=CHCO), 165.81(OC$CO_2$Me), 167.02(CH=$CHCO_2$), 169.89(HOC$CO_2$Me), 170.07(Me$CO_2$); $C_{36}H_{50}O_{12}$ requires 64.08% C,7.47% H; analysis found 63.74% C,7,44% H.

Example 2

[1S-[1α(4R*,5S*),4β,5α,6α,7β]] 1-(4-Hydroxy-5-methyl-3-methylene-6-phenylhexyl)-4,6,7-trihydroxy-2,8-dioxabicyclo[3.2.1]octane-4,5-dicarboxylic acid

A mixture of Example 1 (153.7mg) and aqueous sodium hydroxide (3M,3ml) in tetrahydrofuran (2ml) was heated under reflux for 26h. Organic solvent was removed in vacuo. The resultant solution was diluted with water, adjusted the pH6 with 2M aqueous hydrochloric acid, extracted with ether (3x), acidified to pH2. The acidic solution was saturated with sodium chloride, extracted with ethyl acetate (4x), dried over magnesium sulphate and filtered. Rotary-evaporation gave a residue which was subjected to reverse phase preparative HPLC (on a 1 inch Spherisorb ODS-2 column with 15ml/min flow rate and eluting with a gradient of 15% to 95% acetonitrile-water containing 0.15ml per litre concentrated sulphuric acid over 23mins) to give the appropriate fractions which were combined and rotary-evaporated to remove most of the

acetonitrile. The aqueous solution was saturated with sodium chloride, extracted with ethyl acetate (4x), dried over magnesium sulphate and filtered. Removal of solvent gave the title compound (17.4mg); δ ($d^4$-MeOH) 0.81(d,3H,MeCH,J=7Hz), 1.9-2.1 and 2.1-2.45(2m,6H,MeCH,$CH_2CH_2C=CH_2$ and one proton of $PhCH_2$), 2.77(dd,1H,other proton of $PhCH_2$,J=13.7 and 6Hz), 3.69(d,1H,one proton of $CH_2O$,J=12.7Hz), 3.91(d,1H,$CH_2=CCHOH$,J=5.2Hz), 4.06(d,1H,$CH_2CCHOH$,J=2Hz), 4.53(d,1H,other proton of $CH_2O$,J=12.7Hz), 4.97-5.1(m,3H,$C=CH_2$ and $HO_2CCCHOH$), 7.1-7.3(m,5H,Ph); δ($^{13}C$, $d^4$-MeOH) 16.6-($CHCH_3$), 28.7($CH_2C=CH_2$), 38.4($CH_2CH_2C=CH_2$), 41.9($CHCH_3$), 44($CH_2Ph$), 72.8($CH_2O$), 78($HOCCO_2H$), 81.5($CH_2=CCHOH$), 82.3($HO_2CCCHOH$), 87($CH_2CCHOH$),95($OCCO_2H$), 109.3(OCO), 113($C=CH_2$), 129.4-(para C of Ph), 131.8(2meta C of Ph), 133(2 ortho C of Ph), 145.5(quaternary C of Ph), 155.2($C=CH_2$), 172.6($OCCO_2H$), 176.4($HOCCO_2H$).

Example 3

[1S-[1α(4R*,5S*),4β,5α,6α(2E,4R*,6R*),7β]] 1-(4-Acetyloxy-5-methyl-3-methylene-6-phenylhexyl)-4,6,7-trihydroxy-2,8-dioxabicyclo [3.2.1]octane-4,5-dicarboxylic acid, 6-(4,6-dimethyl-2-octenoate) [Compound 1], [1S-[1α(4R*,5S*),4β,5α,6α(2E,4R*,6R*),7β]] 1-(4-acetyloxy-5-methyl-3-methylene-6-phenylhexyl)-4,6,7-trihydroxy-2,8-dioxabicyclo[3.2.1]octane-4,5-dicarboxylic acid, 6-(4,6-dimethyl-2-octenoate), 4-methyl ester [Compound 2] and [1S-[1α(4R*,5S*), 4β,5α,6α(2E,4R*,6R*),7β]] 1-(4-acetyloxy-5-methyl-3-methylene-6-phenylhexyl)-4,6,7-trihydroxy-2,8-dioxabicyclo[3.2.1]octane-4,5-dicarboxylic acid, 6-(4,6-dimethyl-2-octenoate), 5-methyl ester [Compound 3]

A mixture of Example 1 (372.9mg), lithium iodide (236.1mg) and water (0.23ml) in dimethyl sulphoxide (4ml) was heated at 141-150C for 22h under nitrogen. The cooled mixture was diluted with saturated sodium chloride solution (50ml), extracted with ethyl acetate (5x), dried over magnesium sulphate and filtered. Removal of solvent gave a gum which was subjected to reverse phase preparative HPLC (on a 1 inch Spherisorb ODS-2 column with 15ml/min flow rate and eluting with a gradient of 15% to 95% acetonitrile-water containing 0.15ml per litre concentrated sulphuric acid over 23mins) to give the appropriate fractions which were combined and rotary-evaporated to remove most of the acetonitrile. The aqueous solution was saturated with sodium chloride, extracted with ethyl acetate (4x), dried over magnesium sulphate and filtered. Removal of the solvent gave title compound 1 (116.8mg); δ ($d^4$-MeOH) 0.83-0.92(m,9H,3Me), 1.04-(d,3H,$O_2CCH=CHCHMe$,J=7.5Hz), 1.08-1.2 and 1.2-1.45(2m,5H,$MeCHCH_2CHCH_2Me$), 1.9-1.98-(m,2H,$CH_2CH_2C=CH_2$), 2.10(s,3H,$MeCO_2$), 2.18-2.5(m,4H,$CH_2C=CH_2$,$PhCH_2CHMe$ and $O_2CCH=CHCHMe$), 2.44(dd,1H,one proton of $PhCH_2$, J=14.3 and 7.6Hz), 2.70(dd,1H,other proton of $PhCH_2$,J=14.3 and 6.7Hz), 3.78(d,1H,one proton of $CH_2O$,J=12.5Hz), 4.02(d,1H,CHOH,J=2.5Hz), 4.65-(d,1H,other proton of $CH_2O$,J=12.5Hz), 4.98 and 5.00(2s,2H,$C=CH_2$), 5.08(d,1H,CHOAc,J=5Hz), 5.81-(d,1H,$O_2CCH=CH$,J=16.5Hz), 6.27(d,1H,$CHO_2CCH=CH$,J=2.5Hz), 6.88(dd,1H,$O_2CCH=CH$,J=16.5 and 9Hz), 7.12-7.21(m,3H,2 meta and one para protons of Ph), 7.24-7.31(m,2H,2 ortho protons of Ph); δ ($^{13}C$,$d^4$-MeOH) 10.4($CH_3CH_2$), 12.8($CH_3CHCHOAc$), 18.2($CH_3CHEt$), 19.4($CH=CHCHCH_3$), 19.7($CH_3CO_2$), 25.4-($CH_2C=CH_2$), 29.7($CH_2CH_3$), 32.1(CHEt), 34.2($CH_2CHC=CH_2$), 34.4($O_2CCH=CHCHMe$), 36.6-($CH_3CHCH_2Ph$), 39.8($PhCH_2$), 43.2($MeCHCH_2CHMe$), 69.2($CH_2O$), 74($HOCCO_2H$), 78.9(CHOAc), 80.2-($CHO_2CCH=CH$), 81.6(CHOH), 89.2($OCCO_2H$), 105.9(OCO), 110.2($C=CH_2$), 118.6($CH=CHCO_2$), 125.8-(para C of Ph), 128(2 meta C of Ph), 129(2 ortho C of Ph), 140.6(quarternary C of Ph), 146.5($C=CH_2$), 156.3($CH=CHCO_2$), 165.7($CH=CHCO_2$), 167.8($OCCO_2H$), 171($HOCCO_2H$), 172($MeCO_2$); title Compound 2 (26.5mg) δ ($CDCl_3$) 0.8-0.9(m,9H,3Me), 1.05(d,3H,$O_2CCH=CHCHMe$,J=7Hz), 1.0-1.2 and 1.2-1.5-(2m,5H,$MeCHCH_2CHCH_2Me$), 1.96-2.16(m,3H,$CH_2CH_2C=CH_2$ and $PhCH_2CHMe$), 2.17-2.5-(m,3H,$CH_2C=CH_2$ and $O_2CCH=CHCHMe$), 2.1(s,3H,$CH_3CO_2$), 2.39(dd,1H,one proton of $PhCH_2$,J=13.5 and 9Hz), 2.69(dd,1H,other proton of $PhCH_2$,J=13.5 and 5.7Hz), 4.06(broad s,3H,$CO_2H$ and 2OH), 3.89-(s,3H,$CO_2Me$), 3.88(d,1H,one proton of $CH_2O$,J=12.7Hz), 4.02(d,1H,CHOH,J=2Hz), 4.59(d,1H,other proton of $CH_2O$,J=12.7Hz), 4.96 and 4.98(2s,2H,$C=CH_2$), 5.13(d,1H,CHOAc,J=5Hz), 5.77-(d,1H,$CHO_2CCH=CH$,J=2Hz), 5.8(d,1H,$O_2CCH=CH$,J=16Hz), 6.92(dd,1H,$O_2CCH=CH$,J=16 and 8.5Hz), 7.1-7.3(m,5H,Ph); $\nu_{max}$ ($CHBr_3$) 3662,3551 and 3465 (OH) and 1729cm$^{-1}$ (C=O); and title Compound 3 - (49.2mg); δ ($CDCl_3$) 0.8-0.9(m,9H,3Me), 1.05(d,3H,$CH_3CHCH=CHCO_2$,J=7Hz), 1.0-1.2 and 1.2-1.4-(2m,5H,$MeCHCH_2CHCH_2Me$), 2.0-2.17(m,3H,$CH_2CH_2C=CH_2$ and $PhCH_2CHMe$), 2.1(s,3H,$MeCO_2$), 2.2-2.5-(m,4H,$CH_2C=CH_2$,$O_2CCH=CHCHMe$ and one proton of $PhCH_2$), 2.68(dd,1H,other proton of $PhCH_2$,J=13 and 5.5Hz), 3.82(s,3H,$OCH_3$), 3.87(d,1H,one proton of $CH_2O$,J=12.5Hz), 4.3-5.5(very broad s,3H,$CO_2H$ and 2OH), 4.65(d,1H,other proton of $CH_2O$,J=12.5Hz), 4.94 and 4.98(2s,2H,$C=CH_2$), 5.08(d,1H,CHOAc,J=5Hz), 5.75(d,1H,$O_2CCH=CH$,J=16Hz), 5.82(s,1H,$CHO_2CCH=CH$), 6.83(dd,1H,$O_2CCH=CH$,J=16 and 9Hz), 7.1-7.3(m,5H,Ph); $\nu_{max}$ ($CHBr_3$) 3669, 3550 and 3430 (OH), 1767 and 1726cm$^{-1}$ (C=O).

Example 4

[1S-[1α(4R*,5S*),4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-4,5-dicarboxylic acid, 6-(4,6-dimethvl-2-octenoate), dipotassium salt

To a solution of Compound 1 of Example 3 (113.4mg) in dioxan (14ml), an aqueous potassium hydrogen carbonate (35.1mg) solution in deionised water (10ml) was added. After stirring for 10mins, most organic solvent was removed in vacuo to give a cloudy solution. The aqueous solution was extracted with ether (3x) and freeze-dried overnight to give the title compound as a light fluffy white solid (122.5mg). δ - ($D_2O$) 0.79(m,overlapped with 0.8 signal,3H,$MeCH_2$), 0.8(d,3H,MeCH,J=7Hz), 0.88(d,3H,MeCH,J=7Hz), 1.01(d,3H,$O_2CCH=CHCHMe$,J=7Hz), 1.0-1.2 and 1.2-1.46(2m,5H,$MeCHCH_2CHCH_2Me$), 1.73-1.9-(m,2H,$CH_2CH_2C=CH_2$), 2.0-2.38(m,3H,$PhCH_2CH$ and $CH_2C=CH_2$), 2.16(s,3H,$MeCO_2$), 2.4-2.68-(m,3H,$PhCH_2$ and $O_2CCH=CHCH$), 3.66(d,1H,one proton of $CH_2O$,J=12.5Hz), 3.95(s,1H,CHOH), 4.53-(d,1H,other proton of $CH_2O$,J=12.5Hz), 4.90(d,1H,CHOAc,J=4.5Hz), 4.97 and 5.03(2s,2H,$C=CH_2$), 5.91-(d,1H,$O_2CCH=CH$,J=15.5Hz), 6.13(s,1H,$CHO_2CCH=CH$), 6.95(dd,1H,$O_2CCH=CH$,J=15.5 and 8.2Hz), 7.17-7.37(m,5H,Ph); $C_{34}H_{44}K_2O_{12}$;$2.9H_2O$ requires 52.68%C, 6.48%H, 6.74%$H_2O$; analysis found 52.32%C,6.11%H, 6.4%$H_2O$.

Example 5

[1S-[1α(4R*,5S*),3α,4β,5α,6α(2E,4R*,6R*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate), 4,5-dimethyl ester

Intermediate 3 (500mg) and N-hydroxysuccinimide (88mg) in dry tetrahydrofuran (7ml) at room temperature was treated with 1-cyclohexyl-3-(2-morpholinoethyl)carbodiimide metho-p-toluenesulphonate (324mg). After 2h further quantities of N-hydroxysuccinimide (40mg) and 1-cyclohexyl-3-(2-morpholinoethyl) carbodiimide metho-p-toluenesulphonate (147mg) were added. The mixture was stirred for a further 1.5h when sodium borohydride (132mg) was added, followed after a further hour by aqueous citric acid [(10%w/v), 50ml]. The resultant mixture was shaken with ethyl acetate (3x50ml) and the combined organic phase washed with saturated brine (4x30ml), dried over magnesium sulphate and filtered. Evaporation of the filtrate gave a gum which was purified by column chromatography using silica gel, eluting with ethyl acetate-cyclohexane [(3:7), (1:1), (11:9)] to give the title compound as a white solid (270mg); δ ($CDCl_3$) values include: 0.8-0.9 (m,9H,$3CH_3$), 1.05 (d,3H,$CH=CHCHCH_3$,J=7Hz), 2.1 (s,3H,$CH_3CO_2$), 2.69 (dd,1H,one of PhCH,J=13.7 and 6.2Hz), 3.74 (b,2H,$CH_2OH$), 3.80 and 3.88 (2s,6H,$2CO_2Me$), 4.03 (b,1H,CHOH), 4.64 (t,1H,$CHCH_2OH$,J=5Hz), 4.94 and 4.96 (2s,2H,$C=CH_2$), 5.10 (d,1H,AcOCH,J=5Hz), 5.74 (d,1H,$CH=CHCO_2$,J=15.5Hz), 5.83 (d,1H,$CHOCOCH=CH$,J=2Hz), 6.85 (dd,1H,$CH=CHCO_2$,J=15.5 and 8.7Hz), 7.1-7.4 (m,5H,Ph); analytical HPLC, retention time 21.51 min [Spherisorb ODS-2, solvent acetonitrile-water (14.25-90.25% over 25 min) containing 0.15ml concentrated sulphuric acid/L].

Example 6

[1S-[1α(4R*,5S*),3α,4β,5α,6α,7β]] 1-(4-Hydroxy-5-methyl-3-methylene-6-phenylhexyl)-3-hydroxymethyl-4,6,7-trihydroxy-2,8-dioxabicyclo[3.2.1]octane-4,5-dicarboxylic acid

Example 5(118mg) in tetrahydrofuran (1ml) was heated with aqueous sodium hydroxide solution [(3M), 2.25ml]. The mixture was heated for 24h at 80°C under nitrogen, then the solvent was removed by evaporation, water (10ml) added, followed by concentrated sulphuric acid, until a pH of 5-6 was achieved. The aqueous solution was then shaken with diethyl ether(4x20ml) and then acidified by the addition of concentrated sulphuric acid. The white emulsion that formed was shaken with ethyl acetate (3x50ml), the combined organic phases were dried over magnesium sulphate and filtered. Evaporation of the solvent yielded the title compound as a white solid (47.5mg); δ ($^1H$,$CD_3OD$) values include: 0.8 (d,3H,$CH_3$), 1.98-2.13 (m,3H,$CH_2CH_2C=CH_2$,$CH(CH_3)CH_2Ph$), 2.2-2.45 (m,3H,$CH_2CH_2C=CH_2$,one of PhCH), 2.78 (dd,1H,one of PhCH J=13.7 and 6.2Hz), 3.62 (m,2H,$CH_2OH$), 3.94 (d,1H,CHOH,J=5Hz), 4.05 (d,1H,$HO_2CCCH(OH)CH(OH)$,J=2Hz), 4.55 (t,1H,$CHCH_2OH$,J=5Hz), 4.98 and 5.09 (2s,2H,$C=CH_2$), 5.12 (d,1H,$HO_2CCCH(OH)$,J=2Hz), 7.10-7.30 (m,5H,Ph); composite pulse decoupled 125.75MHz carbon-13 nmr spectrum in deuterochloroform [δ values with the number of attached protons in parenthesis]: 14.0 (3), 26.0 (2), 35.6 (2), 39.1 (1), 41.3 (2), 62.2 (2), 75.8 (0), 76.7 (1), 78.9 (1), 79.4(1), 84.4 (1), 93.0 (0), 106.7 (0), 110.3

(2), 126.6 (1), 129.1 (1), 130.1 (1), 142.5 (0), 152.3 (0), 169.8 (0), 173.7 (0).

Example 7

[1S-[1α(4R*,5S*),3α,4β,5α,6α(2E,4R*,6R*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate) [Compound 1] and [1S-[1α(4R*,5S*),3α,4β,5α,6α(2E,4R*,6R*),7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate),4-methyl ester [Compound 2]

A solution of Example 5 (200mg) in 2,4,6-trimethylpyridine (10ml) was heated to 45°C under a slow steam of nitrogen. To this was added anhydrous lithium iodide (759mg). After 7 days the solvent was evaporated off under reduced pressure, saturated brine (50ml) added and the mixture shaken with diethyl ether (3x50ml). The combined organic phase was dried over magnesium sulphate, filtered and the solvent evaporated off under reduced pressure to yield a product which was subjected to preparative reverse-phase HPLC [using a 1 inch Spherisorb ODS-2 column eluting with a gradient of 40 to 95% acetonitrile/water containing concentrated sulphuric acid (0.15ml/L) over 23 mins] to give appropriate fractions. Evaporation of the acetonitrile under reduced pressure followed by extraction with ethyl acetate, drying with magnesium sulphate, filtering and evaporation to dryness gave the title Compound 1 as a white solid (90.2mg); δ - ($^1H$,$CD_3OD$) values include 0.8-0.9 (m,9H,3$CH_3$), 1.05 (d,3H,CH = CHCH$CH_3$,J = 7Hz), 2.1 (s,3H,$CH_3CO_2$), 2.65 (dd,1-H one of PhCH,J = 13.7 and 6.2Hz), 3.65 (b,2H,$CH_2$OH), 4.0 (b,1H,CHOH), 4.64 (b 1H CH$CH_2$OH), 4.94-4.96 (2s,2H,C = $CH_2$), 5.07 (d,1H,AcOCH,J = 5Hz), 5.80 (d,1H,CH = CH$CO_2$,J = 15.5Hz), 6.34 (b,1H,CHOCOCH = CH), 6.85 (dd,1H,CH = CH$CO_2$,J = 15.5 and 8.70Hz), 7.1-7.3 (m,5H,Ph); composite pulse decoupled 125.75MHz carbon-13 nmr spectrum in deuterochloroform [δ values with the number of attached protons in parenthesis] : 11.39 (3), 14.15 (3), 19.25 (3), 20.48 (3), 20.87 (3), 26.71 (2), 30.74 (2), 33.14 (1), 35.43 (2), 35.53 (1), 37.76 (1), 40.95 (2), 44.4 (2), 62.44 (2), 76.0 (0), 77.03 (1), 80.17 (1), 81.63 (1), 83.14 (1), 91.1 (0), 106.41 (0), 111.39 (2), 120.11 (1), 126.90 (1), 129.27 (1), 130.13 (1), 141.6 (0), 147.96 (0), 157.2 (1), 167.12 (0), 170.0 (0), 172.0 (0), 174.5 (0). -FAB mass spectrometry values include: 675.8 (M-H) and 631.6 (M-$CO_2$H); and the title Compound 2 as a white solid (11.5mg); δ ($CDCl_3$) values include: 0.8-0.9 (m,9H,3$CH_3$), 1.05 (d,3H,CH = CHCH$CH_3$,J = 7Hz), 2.1 (s,3H,$CH_3CO_2$), 2.69 (dd,1H,one of PhCH,J = 13.7 and 6.2Hz), 3.75 (d,2H,$CH_2$OH), 3.85 (s,3H,C(OH)$CO_2CH_3$),4.0(s,1H,CHOH), 4.64(t,1H,CH$CH_2$OH,J = 5Hz),4.94 and 4.96 (2s,2H,C = $CH_2$), 5.10 (d,1H,AcOCH,J = 5Hz), 5.74 (d,1H,CH = CH$CO_2$,J = 15.5Hz), 5.74 (s,1H,CHOCOCH = CH), 6.85 (dd,1H,CH = CH$CO_2$,J = 15.5 and 8.70Hz), 7.1-7.4 (m,5H,Ph); analytical HPLC, retention time 18.70min [Spherisorb ODS-2, solvent acetonitrile-water (14.25-90.25% over 25 min) containing 0.15ml concentrated sulphuric acid/L].

Example 8

[1S-[1α(4R*,5S*),3α,4β,5α,6α(2E,4R*,6R*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate), dipotassium salt

To a solution of Compound 1 of Example 7 (83.2mg) in a mixture of dioxan (10ml) and deionized water (4ml) was added a solution of potassium hydrogen carbonate (24.6mg) in deionized water(2ml). Freeze-drying of the mixture gave the title compound as a white solid (79.9mg); δ ($D_2O$) values include: 0.7-0.85 (m,9H,3$CH_3$), 0.95 (d,3H,CH = CHCH$CH_3$,J = 7Hz), 2.1 (s,3H,$CH_3CO_2$), 3.45 (m,1H,one of $CH_2$OH), 3.58 (m,1H,one of $CH_2$OH), 3.88 (s,1H,CHOH), 4.45 (m,1H,CH$CH_2$OH), 4.83 (d,ACOCH,J = 5Hz), 4.90 and 4.95 (2s,2H,C = $CH_2$), 5.83 (d,1H,CH = CH$CO_2$,J = 15.5Hz), 6.03 (s,1H,CHOCOCH = CH), 6.85 (dd,1H,CH = CH$CO_2$,J = 15.5 and 8.70Hz), 7.1-7.3 (m,5H,Ph); analytical HPLC, retention time 17.75min [Spherisorb ODS-2, solvent acetonitrile-water (14.25-90.25% over 25 min) containing 0.15ml concentrated sulphuric acid/L].

Example 9

[1S-[1α(4R*,5S*),3α,4β,5α,6α,7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid

A solution of Example 5 (832mg) in 2,4,6-trimethylpyridine (42ml) was heated to 45°C under a slow

stream of nitrogen. To this was added anhydrous lithium iodide (1.58g). After ten days the solvent was evaporated off under reduced pressure, the residue taken up in ethyl acetate (200ml) and washed with a mixture of dilute hydrochloric acid (2M) and saturated brine (1:1) (2x100ml), then saturated sodium thiosulphate solution (1x100ml). The organic phases were combined and dried over magnesium sulphate, filtered and evaporated to dryness. The mixture was purified by reverse-phase column chromatography using a partisil prep 40 ODS-3 75Å packing eluting with acetonitrile/water (30:70). Evaporation of the solvent from the appropriate fraction yielded a product that was further purified by preparative reverse-phase HPLC using a one inch Spherisorb ODS-2 column eluting with a gradient of 30 to 95% acetonitrile/water containing concentrated sulphuric acid (0.15ml/L) over 20mins. The appropriate fractions were combined and the acetonitrile removed by evaporation under reduced pressure. Extraction of the resultant into ethyl acetate followed by drying with magnesium sulphate, filtering and evaporation to dryness gave the title compound as a white solid (11.6mg); δ ($CD_3OD$) values include: 0.85 (d,3H,$CH_3$,J = 7.5Hz), 2.0 (m,2H,$CH_2CH_2C=CH_2$), 2.11 (s,3H,$CH_3CO_2$), 2.2-2.5 (m,4H,$CH_2CH_2C=CH_2$,one of PhCH,CH($CH_3$)$CH_2$Ph), 2.78 (dd,1H,one of PhCH,J = 13.7 and 6.2Hz), 3.62 (b,2H,$CH_2OH$), 4.05 (d,1H,$HO_2CCCH(OH)CH(OH)$-,J = 2Hz), 4.55 (b,1H,$CHCH_2OH$),4.94 and 4.96 (2s,2H,$C=CH_2$), 5.07 (d,1H,AcOCH,J = 5Hz), 5.12 (b,1H,$HO_2CCCH(OH)$), 7.10-7.30 (m,5H,Ph); analytical HPLC, retention time 9.47min [Spherisorb ODS-2, solvent acetonitrile-water (14.25-90.25% over 25 min) containing 0.15ml sulphuric acid/L].

Example 10

[1S-[1α(4R*,5S*),3α,4β,5α,6α(4S*,6R*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate), dipotassium salt

A solution of Intermediate 8 (0.0637g) in dioxan (saturated with hydrogen chloride gas) was stirred at room temperature for 1 day. The solvent was evaporated under reduced pressure and the crude product purified by preparative reverse-phase HPLC using a 1 inch spherisorb ODS-2 column eluting with a gradient of 40 to 95% acetonitrile/water containing concentrated sulphuric acid (0.15ml/L) over 24 mins. Appropriate fractions were combined, the acetonitrile removed under reduced pressure, and the remainder extracted with ethyl acetate (5x25ml). The extracts were dried (magnesium sulphate), filtered and evaporated to dryness to give an off-white solid. This material was dissolved in a mixture of dioxan (4ml) and deionized water (1.5ml) and treated with a solution of potassium hydrogen carbonate (6.28mg) in deionized water (2ml). Freeze-drying of the mixture gave the title compound as a white solid (26.6mg); δ ($D_2O$) values include: 0.7-0.95(m,12H,4$CH_3$), 2.14(s,3H,$CH_3CO_2$), 3.51(m,1H,one of $CH_2OH$), 3.65(m,1H,one of $CH_2OH$), 3.97(s,1H,CHOH), 4.52(m,1H,$CHCH_2OH$), 4.92(b,AcOCH), 4.95 and 5.05(2s,2H,$C=CH_2$), 6.10-(s,1H,$CHOCOCH_2$-$CH_2$), 7.1-7.4(m,5H,Ph).

Example 11

[1S-[1α(4R*,5S*),3α,4β,5α,6α(2E,4R*,6R*),7β]] 1-(4-Hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate)

To a solution of Example 7, Compound 1 (0.231g) in methanol (40ml), concentrated hydrochloric acid (0.5ml) was added. After stirring at room temperature for three days the solvent was removed by evaporation at reduced pressure. The residue was taken up in ethyl acetate (50ml) and the solution washed with saturated brine (50ml). The organic phase was dried over magnesium sulphate, filtered and evaporated to dryness. The crude product was purified by reverse-phase column chromatography using a partisil prep 40 ODS-3 75Å packing eluting with acetonitrile/water (40:60). Evaporation of the solvent from the appropriate fractions yielded the title compound as a white solid (81.5mg); δ ($CD_3OD$) values include 0.7-0.9-(m,9H,3$CH_3$), 1.05(d,3H,$CH=CHCHCH_3$,J = 7Hz), 2.78(dd,1H one of PhCH,J = 13.7 and 6.2Hz), 3.68-(b,2H,$CH_2OH$), 3.85(d,1H,$CH_2=C$-CHOH,J = 5Hz), 4.05(b,1H,CHOH), 4.65(b,1H,$CHCH_2OH$), 4.95 and 5.10-(2s,2H,$C=CH_2$), 5.80(d,1H,$CH=CHCO_2$,J = 15.5Hz), 6.3(b,1H,$CHOCOCH=CH$), 6.85-(dd,1H,$CH=CHCO_2$,J = 15.5 and 8.70Hz), 7.1-7.3(m,5H,Ph).

| | |
|---|---|
| Analysis found : | C,60.88%; H,7.47%, |
| $C_{33}H_{46}O_{12}.0.72H_2O$ requires : | C,61.20%; H,7.38%. |

Example 12

[1S-[1α(4R*,5S*),3α,4β,5α,6α(2E,4R*,6R*),7β]] 1-(4-Hydroxy-5-methyl-3-methylene-6-phenylhexyl)-3-hvdroxymethyl-4,6,7-trihydroxy-2,8-dioxabicyclo[3.2.1]octane-4,5-dicarboxylic acid, 6-(4,6-dimethyl-2-octenoate), dipotassium salt

To a solution of Example 11 (73.1mg) in a mixture of dioxan (10ml) and deionized water (4ml) was added a solution of potassium hydrogen carbonate (23.06mg) in deionized water (2ml). Freeze-drying of the mixture gave the title compound as a white solid (90.6mg); δ ($D_2O$) values include: 0.7-0.9(m,9H,3$CH_3$), 1.02(d,3H,CH = CHCHCH$_3$,J = 7Hz), 2.71(dd,1H,one of PhCH,J = 13.7 and 6.2Hz), 3.51(m,1H,one of $CH_2OH$), 3.69(m,1H,one of $CH_2OH$), 3.94(d,1H,$CH_2$ = C-CHOH,J = 5Hz), 3.99(s,1H,CHOH), 4.52(m,1H,CHCH$_2$OH), 5.06 and 5.12(2s,2H,C = $CH_2$), 5.90(d,1H,CH = CHCO$_2$,J = 15.5Hz), 6.13(s,1H,CHOCOCH = CH), 6.95-(dd,1H,CH = CHCO$_2$,J = 15.5 and 8.70Hz), 7.2-7.45(m,5H,Ph.

Example 13

[1S-[1α(4R*,5S*),4α,4β,5α,6α(2E,4R*,6R*),7β]] 1-(4-Hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate), 4,5-dimethyl ester

To a solution of Example 5 (8.94g) in methanol (1000ml), concentrated hydrochloric acid (15ml) was added. After stirring at room temperature for six days the solvent was removed by evaporation at reduced pressure. The residue was taken up in ethyl acetate (1000ml) and the solution washed with saturated sodium bicarbonate solution (3x200ml). The organic phase was dried over magnesium sulphate, filtered and the solvent evaporated. The crude product was purified by reverse-phase column chromatography using Partisil prep 40 ODS-3 75Å packing eluting with acetonitrile/water 1:1. The appropriate fractions were combined, the acetonitrile removed by evaporation under reduced pressure, saturated brine (250ml) added, and the mixture shaken with ethyl acetate (3x300ml). The organic phases were combined, dried over magnesium sulphate filtered and the solvent removed by evaporation under reduced pressure to yield a white foam. This product was further purified by preparative reverse-phase HPLC using a two inch spherisorb ODS-2 column eluting with acetonitrile/water (63:37). The appropriate fractions were combined, the acetonitrile removed by evaporation under reduced pressure, saturated brine (500ml) added and the mixture shaken with ethyl acetate (5x300ml). The organic phases were combined, dried over magnesium sulphate, filtered and the solvent removed by evaporation to yield the title compound as a white solid (3.75g); δ ($CDCl_3$) values include 0.75-0.95(m,9H,3$CH_3$), 1.05(d,3H,CH = CHCHCH$_3$,J = 7Hz), 2.73(dd,1H,one of PhCH,1 = 13.7 and 6.2Hz), 3.72(b,2H,$CH_2OH$), 3.80 and 3.84(2s,6H,2$CO_2$Me), 4.0(d,1H,$CH_2$ = C-CHOH,J = 5Hz), 4.05(s,1H,CHOH), 4.59(t,1H,CHCH$_2$OH,J = 5Hz), 4.96 and 5.09(2s,2H,C = $CH_2$), 5.73-(d,1H,CH = CHCO$_2$,J = 15.5Hz), 5.83(s,1H,CHOCOCH), 6.83(dd,1H,CH = CHCO$_2$,J = 15.5Hz and 8.7Hz), 7.1-7.3(m,5H,Ph).

| | | |
|---|---|---|
| Analysis found : | C,63.10%; | H,7.75%, |
| $C_{35}H_{50}O_{12}$ requires : | C,63.43%; | H,7.60%. |

Example 14

[1S-[1α(4R*,5S*),3α,4β,5α,6α(4S*,6R*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate), 4,5-dimethyl ester

To a solution of Example 5 (100mg) in ethanol (10ml) was added an aqueous solution of copper (II) sulphate (1M; 0.14ml). After cooling the solution to 0°C, sodium borohydride (26.8mg) was added

portionwise over 1 minute. The resultant black suspension was stirred for 2 hours and allowed to warm to room temperature, whereupon, the organic solvent was evaporated off under reduced pressure. The black solid was solubilised by consecutive shaking with ethyl acetate (10ml) and dilute hydrochloric acid (0.1M; 10ml x5). The solutions were combined and the organic phase separated. The aqueous phase was then shaken with ethyl acetate (4x20ml), all the organic phases combined, dried over magnesium sulphate and filtered. Evaporation of the filtrate gave a brown gum which was purified by column chromatography using silica gel, eluting with ethyl acetate-cyclohexane (1:1) to give the title compound as a white solid (53mg); δ-($CDCl_3$) values include: 0.8-0.9(m,12H,4$CH_3$), 2.1(s,3H,$CH_3CO_2$), 2.69(dd,1H one of PhCH,J=13.7 and 6.2Hz), 3.72(b,2H,$CH_2OH$), 3.78 and 3.85(2s,6H,2$CO_2$Me), 3.97(b,1H,CHOH), 4.58(t,1H,CHC$H_2$OH,J=5Hz), 4.94 and 4.96(2s,2H,C=$CH_2$), 5.10(d,1H,AcOCH,J=5Hz), 5.78(d,1H,CHOCOC$H_2$C$H_2$,J=2Hz), 7.10-7.30-(m,5H,Ph).

| | | |
|---|---|---|
| Analysis found: | C,62.96%; | H,7.36%. |
| $C_{37}H_{54}O_{13}$ Requires : | C,62.88%; | H,7.70%. |

Example 15

[1S-[1α(4R*,5S*),3α,4β,5α,6α,7β]] 1-(4-Acetyloxy-5-methyl-3-methylene-6-phenylhexyl)3-hydroxymethyl-4,6,7-trihydroxy-2,8-dioxabicyclo[3.2.1]octane-4,5-dicarboxylic acid, 6-heptanoate, 4,5-dimethyl ester

A solution of Intermediate 11 (0.500g) in formic acid (21.3ml) was treated with water (7.1ml) and the mixture heated at 70° C for 4h, then allowed to cool. The solution was diluted with ethyl acetate (100ml) and progressively neutralised with saturated aqueous sodium hydrogen carbonate solution and solid sodium hydrogen carbonate. The organic phase was separated and the aqueous phase extracted with ethyl acetate (100ml). The combined organic phases were washed with saturated aqueous sodium hydrogen carbonate solution (100ml), dried ($MgSO_4$), filtered and concentrated in vacuo to a colourless gum. This was dissolved in methanol (2.5ml) and treated with an aqueous slurry of potassium hydrogen carbonate (0.596g in 1.25ml). After 2h at room temperature the mixture was diluted with ethyl acetate (10ml) and saturated aqueous sodium chloride solution (10ml). The aqueous phase was separated, extracted with ethyl acetate (15ml) and the combined organic phases were washed with saturated aqueous sodium chloride solution (20ml), dried ($MgSO_4$), filtered and concentrated in vacuo to a clear colourless gum. This was purified by flash column chromatography on silica gel eluting with methanol:ethyl acetate:cyclohexane [(0:1:4), (0:2:3), (0:3:2), (0:4:1), (1:19:0)] and appropriate fractions were combined and evaporated to give the title compound (0.252g) as a colourless foam; δ($CDCl_3$) includes 2.10 (s,$O_2CCH_3$), 2.69 (dd,J13 and 5Hz,CHPh), 3.7-3.8 (m,$CH_2$OH), 3.80 and 3.88 (2s,2x$CO_2CH_3$), 3.98 (t,3Hz,7-H), 4.59 (t,J6Hz,3-H), 4.96 and 4.99 (2s,C=$CH_2$), 5.10 (d,J5Hz CHOAc), 5.77 (d,J3Hz,6-H), 7.1-7.3 (m,$C_6H_5$); retention time 18.41 minutes on reverse-phase HPLC (15x0.4cm, Spherisorb,ODS-2) eluting with acetonitrile-water (14.25% to 90.25% over 25minutes), acidified with 0.15ml $l^{-1}$ conc $H_2SO_4$.

Example 16

[1S-[1α(4R*,5S*),3α,4β,5α,6α,7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-heptanoate

A solution of Example 15 (0.180g) in 2,4,6-collidine (8ml) at 45° C with a stream of nitrogen over the top of the flask was treated with lithium iodide (0.362g). After 23h, the mixture was allowed to cool, then diluted with ethyl acetate (130ml) and washed successively twice with aqueous hydrochloric acid (2M, 100ml), once with saturated sodium thiosulphate solution (100ml), once with saturated sodium chloride solution (100ml), then dried ($MgSO_4$), filtered and concentrated in vacuo to give a beige powdery film. This was purified by reverse-phase HPLC (1 inch Spherisorb, ODS-2) eluting with acetonitrile-water (38.00% to 90.25% over 20 minutes) acidified with 0.15ml $l^{-1}$ concentrated sulphuric acid. Appropriate fractions were combined and concentrated in vacuo to remove acetonitrile. The aqueous solution was extracted with ethyl acetate, and the combined organic phases were washed once with saturated aqueous sodium chloride solution, dried ($MgSO_4$)), filtered and concentrated to give the title compound (0.095g) as a colourless gum; δ($CD_3OD$) includes 2.10 (s,$O_2CCH_3$), 2.68 (dd,J13 and 6Hz,CHPh), 3.60-3.66(m,$CH_2$OH), 3.98 (d,J2Hz,7-H), 4.64-(t,J5Hz,3-H),4.95 and 4.99 (2s,C=$CH_2$), 5.06 (d,J5Hz,CHOAc), 6.27 (d,J2Hz,6-H), 7.1-7.3 (m,$C_6H_5$); retention

time 15.34 minutes on reverse-phase HPLC (15x0.4cm,Spherisorb,ODS-2) eluting with acetonitrile-water (14.25% to 90.25% over 25 minutes) acidified with 0.15ml $l^{-1}$ conc $H_2SO_4$.

Example 17

[1S-[1α(4R*,5S*),3α,4β,5α,6α,7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-heptanoate, 4,5-dipotassium salt

A solution of Example 16 (0.051g) in 1,4-dioxan (7ml) was treated with aqueous potassium hydrogen carbonate solution (0.081M, 2.0ml). After 0.5h at room temperature, the solution was freeze-dried to give the title compound (0.067g) as a white solid; δ($D_2O$) includes 2.20 (s,$O_2CCH_3$), 3.45-3.80(m,$CH_2OH$), 3.96-4.04 (bs,7-H), 6.10-6.17 (bs,6-H), 7.2-7.45 (m,$C_6H_5$); retention time 15.20minutes on reverse-phase HPLC (15x0.4cm,Spherisorb,ODS-2) eluting with acetonitrile-water (14.25% to 90.25% over 25minutes) acidified with 0.15ml $l^{-1}$ conc $H_2SO_4$.

Example 18

[1S-[1α(4R*,5S*),3α,4β,5α,6α,7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-nonanoate, 4,5-dimethyl ester

A solution of Intermediate 12 (0.296g) in formic acid (12.3ml) was treated with water (4.1ml) and the mixture heated at 70°C for 4h, then allowed to cool. The solution was diluted with ethyl acetate (150ml) and progressively neutralised with saturated aqueous sodium hydrogen carbonate solution and solid sodium hydrogen carbonate. The organic phase was separated and the aqueous phase extracted twice with ethyl acetate (70ml). The combined organic phases were washed with saturated aqueous sodium hydrogen carbonate solution (100ml), dried ($MgSO_4$), filtered and concentrated in vacuo to a colourless gum. This was dissolved in methanol (1.4ml) and treated with an aqueous slurry of potassium hydrogen carbonate (0.350g in 0.70ml). After 1h at room temperature the mixture was diluted with ethyl acetate (15ml) and saturated aqueous sodium chloride solution (10ml). The aqueous phase was separated, extracted twice with ethyl acetate (10ml) and the combined organic phases were washed with saturated aqueous sodium chloride solution (20ml), dried ($MgSO_4$), filtered and concentrated in vacuo to a clear colourless gum. This was purified by flash column chromatography on silica gel eluting with ethyl acetate:cyclohexane [(1:4), (3:7), (2:3), (1:1), (7:3)] and appropriate fractions were combined and evaporated to give the title compound - (0.102g) as a colourless film; δ($CDCl_3$) includes 2.10 (s,$O_2CCH_3$), 2.69 (dd,J13 and 5Hz,CHPh), 3.7-3.8 (m,$CH_2OH$), 3.80 and 3.88 (2s,2x$CO_2CH_3$), 3.95-3.98 (t,J2Hz,7-H), 4.58 (t,J6Hz,3-H), 4.95 and 4.99 (2s,$C=CH_2$), 5.10 (d,J5Hz,CHOAc), 5.77 (d,J2.5Hz,6-H), 7.1-7.3 (m,$C_6H_5$); retention time 22.16 minutes on reverse-phase HPLC (15x0.4cm, Spherisorb,ODS-2) eluting with acetonitrile-water (14.25% to 90.25% over 25minutes), acidified with 0.15ml $l^{-1}$ conc $H_2SO_4$.

| | |
|---|---|
| Analysis found : | C,62.22;H,7.21 |
| $C_{36}H_{52}O_{13}$ requires : | C,62.41;H,7.56%. |

Example 19

[1S-[1α(4R*,5S*),3α,4β,5α,6α,7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-nonanoate

A solution of Example 18 (0.102g) in 2,4,6-collidine (4ml) at 45°C with a stream of nitrogen over the top of the flask, was treated with lithium iodide (0.197g). After 27.5h, the mixture was allowed to cool, then diluted with ethyl acetate (15ml) and washed successively twice with aqueous hydrochloric acid (2M, 20ml), once with saturated sodium thiosulphate solution (20ml), once with saturated sodium chloride solution (20ml), then dried ($MgSO_4$), filtered and concentrated in vacuo to give a brown film. This was purified by reverse-phase HPLC (1 inch Spherisorb, ODS-2) eluting with acetonitrile-water (57.00% to 90.25% over 13minutes) acidified with 0.15ml $l^{-1}$ concentrated sulphuric acid. Appropriate fractions were combined and concentrated in vacuo to remove acetonitrile. The aqueous solution was saturated with sodium chloride and extracted three times with ethyl acetate and the combined organic phases were washed once with saturated

aqueous sodium chloride solution, dried ($MgSO_4$), filtered and concentrated to give the title compound - (0.044g) as an off-white colourless film; δ($CD_3OD$) includes 2.10 (s,$O_2CCH_3$), 2.68 (dd,J13 and 6Hz,CHPh), 3.60-3.66 (m,$CH_2OH$), 3.99 (d,J2Hz,7-H), 4.63 (t,J5Hz,3-H), 4.95 and 4.99 (2s,C=$CH_2$), 5.06 (d,J5Hz,CHOAc), 6.28 (d,J2Hz,6-H), 7.1-7.3 (m,$C_6H_5$); retention time 17.58minutes on reverse-phase HPLC (15x0.4cm,Spherisorb,ODS-2) eluting with acetonitrile-water (14.25% to 90.25% over 25minutes) acidified with 0.15ml $l^{-1}$ conc $H_2SO_4$.

| | |
|---|---|
| Analysis found: | C,61.66;H,7.49 |
| $C_{34}H_{48}O_{13}$ requires : | C,61.43;H,7.13%. |

Example 20

[1S-[1α(4R*,5S*),3α,4β,5α,6α,7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-nonanoate, 4,5-dipotassium salt

A solution of Example 19 (0.030g) in 1,4-dioxan (4ml) was treated with aqueous potassium hydrogen carbonate solution (0.090M, 1.0ml). After 1.5h at room temperature, the solution was freeze-dried to give the title compound (0.044g) as a white solid; δ($D_2O$) includes 2.15-2.20 (bs,$O_2CCH_3$), 3.45-3.75 (m,$CH_2OH$), 3.95-4.00 (bs,7-H), 5.00 and 5.08 (2bs,C=$CH_2$), 6.08-6.15 (bs,6-H), 7.2-7.45 (m,$C_6H_5$); retention time 17.18minutes on reverse-phase HPLC (15x0.4cm,Spherisorb,ODS-2) eluting with acetonitrile-water (14.25% to 90.25% over 25minutes) acidified with 0.15ml $l^{-1}$ conc $H_2SO_4$.

Example 21

[1S-[1α(4R*,5S*),3α,4β,5α,6α(4R*S*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4-methyl)pentanoate, 4,5-dimethyl ester

A solution of Intermediate 13 (0.190g) in formic acid (8.4ml) was treated with water (2.8ml) and the mixture heated at 70°C for 4h, then allowed to cool. The solution was diluted with ethyl acetate (200ml) and progressively neutralised with saturated aqueous sodium hydrogen carbonate solution and solid sodium hydrogen carbonate. The organic phase was separated and the aqueous phase extracted with ethyl acetate (150ml). The combined organic phases were washed successively with saturated aqueous sodium hydrogen carbonate solution (200ml) and saturated aqueous sodium chloride solution (200ml), then dried ($MgSO_4$), filtered and concentrated in vacuo to a colourless foam. This was dissolved in methanol (1.2ml) and treated with an aqueous slurry of potassium hydrogen carbonate (0.276g in 0.6ml). After 1.25h at room temperature the mixture was diluted with ethyl acetate (30ml) and saturated aqueous sodium chloride solution (20ml). The aqueous phase was separated, extracted with ethyl acetate (30ml) and the combined organic phases were washed with saturated aqueous sodium chloride solution (30ml), dried ($MgSO_4$), filtered and concentrated in vacuo to a clear colourless film. This was purified by flash column chromatography on silica gel eluting with ethyl acetate:cyclohexane [(3:7), (1:1), (7:3)], and appropriate fractions were combined and evaporated to give the title compound (0.112g) as a beige solid; δ($CDCl_3$) includes 0.90 (d,J6Hz,CH($CH_3$)$_2$), 2.10 (s,$O_2CCH_3$), 2.69 (dd,J13 and 5Hz,CHPh), 3.7-3.8 (m,$CH_2OH$), 3.81 and 3.88 (2s,2x$CO_2CH_3$), 3.95-3.98 (m,7-H), 4.59 (t,J5Hz,3-H), 4.96 and 4.99 (2s,C=$CH_2$), 5.10 (d,J5Hz,CHOAc), 5.74-5.78 (m,6-H), 7.1-7.3 (m,$C_6H_5$); retention time 17.79 minutes on reverse-phase HPLC (15x0.4cm, Spherisorb,ODS-2) eluting with acetonitrile-water (14.25% to 90.25% over 25minutes), acidified with 0.15ml $l^{-1}$ concentrated $H_2SO_4$.

| | |
|---|---|
| Analysis found : | C,60.91;H,7.28 |
| $C_{33}H_{46}O_{13}$ requires : | C,60.91;H,7.12%. |

Example 22

[1S-[1α(4R*,5S*),3α,4β,5α,6α(4R*S*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4-methyl)pentanoate

A solution of Example 21 (0.10g) in 2,4,6-collidine (5ml) at 45°C with a stream of nitrogen over the top of the flask was treated with lithium iodide (0.206g). After 2.5days, the mixture was allowed to cool, and after 3days at room temperature was diluted with ethyl acetate (25ml) and washed successively twice with aqueous hydrochloric acid (2M, 20ml), twice with saturated sodium thiosulphate/sodium chloride solution (20ml), once with saturated sodium chloride solution (20ml), then dried ($MgSO_4$), filtered and concentrated in vacuo to give a beige solid film. This was purified by reverse-phase HPLC (1 inch Spherisorb, ODS-2) eluting with acetonitrile-water (47.50% to 78.85% over 12minutes, then to 90.25% over 1 minute) acidified with 0.15ml $l^{-1}$ concentrated sulphuric acid. Appropriate fractions were combined and concentrated in vacuo to remove acetonitrile. The aqueous solution was saturated with sodium chloride and extracted three times with ethyl acetate, and the combined organic phases were washed once with saturated aqueous sodium chloride solution, dried ($MgSO_4$), filtered and concentrated to give the title compound (0.043g) as a colourless film; $\delta$($CD_3OD$) includes 0.89 (d,J5Hz,$CH(CH_3)_2$), 2.10(s,$O_2CCH_3$), 2.68 (dd,J13 and 6Hz,CHPh), 3.60-3.66 (m,$CH_2OH$), 3.99 (d,J2Hz,7-H), 4.63(t,J6Hz,3-H), 4.96 and 4.99 (2s,C=$CH_2$), 5.06 (d,J5Hz,CHOAc), 6.26 (d,J2Hz,6-H), 7.1-7.3 (m,$C_6H_5$); retention time 14.34minutes on reverse-phase HPLC (15x0.4cm,Spherisorb,ODS-2) eluting with acetonitrile-water (14.25% to 90.25% over 25minutes) acidified with 0.15ml $l^{-1}$ conc $H_2SO_4$.

Example 23

[1S-[1α(4R*,5S*),3α,4β,5α,6α(4R*S*),7β]] 1-(4-Acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4-methyl)pentanoate, 4,5-dipotassium salt

A solution of Example 22 (0.030g) in 1,4-dioxan (4ml) was treated with aqueous potassium hydrogen carbonate solution (0.071M, 1.30ml). After 1h at room temperature, the solution was freeze-dried to give the title compound (0.046g) as a white solid; $\delta$($D_2O$) includes 2.18 (s,$O_2CCH_3$), 3.45-3.80 (m,$CH_2OH$), 3.95-4.00 (bs,7-H), 4.99 and 5.04 (2bs,C=$CH_2$), 6.08-6.12 (bs,6-H), 7.2-7.4 (m,$C_6H_5$); retention time 13.87 minutes on reverse-phase HPLC (15x0.4cm,Spherisorb,ODS-2) eluting with acetonitrile-water (14.25% to 90.25% over 25minutes) acidified with 0.15ml $l^{-1}$ conc $H_2SO_4$.

Example 24

[1S-[1α(4R*,5S*),3α,4β,5α,6α(4S*,6R*),7β]] 1-(4-Hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate), 4,5-dimethyl ester

To a solution of Example 13 (1g) in ethanol (100ml) was added an aqueous solution of copper (II) sulphate (1M; 1.5ml). After cooling the solution to 0°C, sodium borohydride (285mg) was added portionwise over 1 minute. The resultant black suspension was stirred for 4 hours allowing it to warm to room temperature, whereupon, the organic solvent was evaporated off under reduced pressure. The black solid was solubilised by shaking with 2M aqueous ammonia solution (200ml) and the resultant deep-blue solution was extracted with ethyl acetate (3x300ml). The combined organic phases were washed with 2M aqueous hydrochloric acid (50ml), saturated aqueous sodium bicarbonate solution (50ml), dried and evaporated to dryness under reduced pressure. The resultant gum was subjected to a repeat of the above reaction. The product was purified by column chromatography using silica gel eluting with ethyl acetate-cyclohexane(1:1) to give the title compound as a white solid (0.40g); $\delta$($CDCl_3$) includes 0.8-0.9 (m,12H,4$CH_3$), 2.75 (dd,J=14 and 6Hz,CHPh), 3.80 and 3.87 (2s,$CO_2CH_3$), 4.58 (t,J=5Hz,3-H), 4.99 and 5.12 (2s,C=$CH_2$), 5.89 (d,J=2Hz,6-H), 7.1-7.3 (m,$C_6H_5$).

| | |
|---|---|
| Analysis Found : | C,63.49;H,8.18; |
| $C_{35}H_{52}O_{12}$ requires : | C,63.24;H,7.88%. |

Example 25

[1S-[1α(4R*,5S*),3α,4β,5α,6α(4S*,6R*),7β]] 1-(4-Hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate)

A solution of Example 24 (400mg) in 2,4,6-trimethylpyridine (20ml) was heated to 45°C under a slow stream of nitrogen. To this was added anhydrous lithium iodide (0.8g). After 14hours the solvent was evaporated under reduced pressure, ethyl acetate (100ml) added and the mixture washed with aqueous hydrochloric acid solution (1M; 2x50ml) and saturated aqueous sodium thiosulphate (50ml). The organic phase was dried over magnesium sulphate, filtered and the solvent evaporated off under reduced pressure to yield a crude product which was purified by preparative reverse-phase HPLC using a 2inch Spherisorb ODS-2 column eluting with 57% acetonitrile/water containing concentrated sulphuric acid (0.15ml/L). Appropriate fractions were combined and the acetonitrile was removed under reduced pressure. The aqueous phase was extracted with ethyl acetate, dried and evaporated to dryness to give the title compound as an off-white solid (100mg); δ($CD_3OD$) includes 0.8-0.9 (m,12H,4$CH_3$), 2.77 (dd,J=14 and 6Hz CHPh), 3.63 (m,$CH_2$OH), 3.92 (d,J=5Hz,=CCHOH), 4.03 (br s,7-H), 4.64 (br t,3-H), 4.97 and 5.10- (2s,C=$CH_2$), 6.28 (br s,6-H), 7.1-7.3 (m,$C_6H_5$).

| | |
|---|---|
| Analysis Found: | C,60.94;H,7.81; |
| $C_{33}H_{48}O_{12}$.0.75$H_2O$ requires : | C,60.95;H,7.67%. |

Example 26

[1S-[1α(4R*,5S*),3α,4β,5α,6α(4S*,6R*),7β]] 1-(4-Hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate), dipotassium salt

A solution of Example 25 (9.8mg) in dioxan (2ml) and water (1ml) was treated with potassium bicarbonate (3.082mg) and the mixture was freeze-dried to give the title compound as a white powder (13mg); δ($D_2O$) includes 0.75-0.9 (m,12H,4$CH_3$), 2.75 (dd,J=14 and 6Hz CHPh), 3.98 (d,J=5Hz,=CCHOH), 4.00 (br s,7-H), 4.57 (br,3-H), 5.08 and 5.13 (2s C=$CH_2$), 6.12 (br s,6-H),7.2-7.4(m,$C_6H_5$).

Example 27

[1S-[1α(4R*,5S*),3α,4β,5α,6α(4S*,6R*),7β]] 1-(4-Hydroxy-5-methyl-3-oxo-6-phenylhexyl)-3-hydroxymethyl-4,6,7-trihydroxy-2,8-dioxabicyclo[3.2.1]octane-4,5-dicarboxylic acid, 6-(4,6-dimethyloctanoate)

A solution of Example 25 (90mg) in methanol (10ml) was cooled to -78°C and ozone rich oxygen was passed through the solution until a blue colour developed. Nitrogen gas was passed through the solution and then triphenylphosphine (41mg) in methanol (2ml) and dichloromethane (0.5ml) was added allowing the solution to warm to 20°C. The solvent was removed under reduced pressure and the residue was partitioned between ether (20ml) and water (28ml) containing 0.880 ammonia solution (2ml). The aqueous layer was extracted with ether (2x20ml), acidified with dilute sulphuric acid and extracted with ethyl acetate (3x30ml). The organic solution was dried, filtered and evaporated to give the title compound (64.5mg) as a clear glass; δ($CD_3OD$) includes 0.77 (d,J=7Hz,PhCH$_2$CHCH$_3$), 0.8-0.9 (m,9H,3$CH_3$), 2.58 (dd,J=14 and 7Hz,PhCH), 3.58 (m,$CH_2$OH), 3.98 (d,J=2Hz,7-H), 4.10 (d,J=3Hz,COCHOH), 4.60 (t,J=5Hz,3-H), 6.23 (d,J=2Hz,6-H),7.1-7.3 (m,$C_6H_5$); analytical HPLC retention time 11.2min (Spherisorb ODS-2 25cmx0.4cm column, solvent 35-95% acetonitrile-water containing 0.15ml conc. $H_2SO_4$/L over 25min).

Example 28

[1S-[1α(4R*,5S*),3α,4β,5α,6α(4S*,6R*),7β]] 1-(4-Hydroxy-5-methyl-3-oxo-6-phenylhexyl)-3-hydroxymethyl-4,6,7-trihydroxy-2,8-dioxabicyclo[3.2.1]octane-4,5-dicarboxylic acid, 6-(4,6-dimethyloctanoate), dipotassium salt

A solution of Example 27 (59mg) in dioxan (8ml) and water (3ml) was treated with potassium bicarbonate (18.49mg) in water (2ml) and the solution was freeze-dried to give the title compound (62mg) as a white powder; δ($D_2O$) includes 0.75-0.88 (m,12H,4$CH_3$), 3.50 (m,$CH_2$OH), 3.95 (s,7-H), 4.27 (s,COCHOH), 4.48 (br,3-H), 6.10 (s,6-H), 7.2-7.5 (m,$C_6H_5$); analytical HPLC retention time 5.58min (Spherisorb ODS-2

15cmx0.46 column, solvent 45% acetonitrile-water containing 0.15ml conc. $H_2SO_4$/L run isocratically).

Example 29

[1S-[1α(3R*S*,4S*,5S*),3α,4β,5α,6α(4S*,6R*),7β]] 1-(4-Acetyloxy-3,5-dimethyl-6-phenylhexyl)-3-hydroxymethyl-4,6,7-trihydroxy-2,8-dioxabicyclo[3.2.1]octane-4,5-dicarboxylic acid, 6-(4,6-dimethyloctanoate) [Compound A] and [1S-[1α(3R*S*,5R*),3α,4β,5α,6α(4S*,6R*),7β]] 1-(3,5-dimethyl-6-phenylhexyl)-3-hydroxymethyl-4,6,7-trihydroxy-2,8-dioxabicyclo[3.2.1]octane-4,5-dicarboxylic acid, 6-(4,6-dimethyloctanoate) [Compound B]

A solution of Example 7 Compound 1 (100mg) in ethanol (10ml) was hydrogenated for 2½ days using 10% palladium-on-carbon (50mg) as catalyst. The catalyst was removed by filtration through Supercell and the filtrate was evaporated to dryness to give a yellow foam which was dissolved in acetonitrile/water (9:1;4.5ml) and loaded onto a preparative HPLC column of Spherisorb ODS-2 (20mmx250mm). The column was eluted with acetonitrile/water (60:40) containing concentrated sulphuric acid (0.15ml/L), flow rate 15ml/min. The acetonitrile was removed from the required fractions by evaporation under reduced pressure (bath temp ca. 35°C) and the aqueous solutions saturated with sodium chloride and extracted with ethyl acetate (x3), dried ($MgSO_4$) and evaporated to dryness. The first component eluted from the column was title compound A (8mg); δ($CD_3OD$) includes 0.78-0.95 (m,9H,$CH_3$), 2.10 and 2.12 (2s,3H,OAc), 2.66-2.77 (m,1H,one of Ph$CH_2$), 3.55-3.68 (m,2H,$CH_2$OH), 3.96 and 3.98 (2d,J=2Hz,1H,7-H), 4.60-4.65 (m,1H,3-H), 6.26 (d,J=2Hz,1H,6-H), 7.1-7.3 (m,5H,Ph); analytical HPLC retention time 17.62min (Spherisorb ODS-2 25cmx0.4cm column, solvent 35-95% acetonitrile/$H_2O$ containing 0.15ml conc. $H_2SO_4$/L over 23min). The second compound eluted from the column was title compound B (32mg); δ($CD_3OD$) includes 0.79-0.94 (m,9H,$CH_3$), 3.56-3.65 (m,2H,$CH_2$OH), 4.00 (t,J=2.5Hz,1H,7-H), 4.62 (t,J=5Hz,3-H), 6.24 (d,J=2.5Hz,1H,6-H), 7.1-7.3 (m,5H,Ph); analytical HPLC retention time 18.83min (Spherisorb ODS-2 25cmx0.4cm column, solvent 35-95% acetonitrile/$H_2O$ containing 0.15ml conc. $H_2SO_4$/L over 23min).

Example 30

[1S-[1α(3R*S*,5R*),3α,4β,5α,6α(4S*,6R*),7β]] 1-(3,5-Dimethyl-6-phenylhexyl)-3-hydroxymethyl-4,6,7-trihydroxy-2,8-dioxabicyclo[3.2.1]octane-4,5-dicarboxylic acid, 6-(4,6-dimethyloctanoate), dipotassium salt

A solution of Example 29 Compound B (30mg) in dioxan (15ml) was treated with a solution of potassium bicarbonate (0.1N;0.96ml) and freeze-dried to give the title compound (31.7mg); δ($D_2O$) includes 0.64-0.90 (m,$CH_3$), 2.50-2.74 (m,Ph$CH_2$), 3.45-3.75 (m,2H,$CH_2$OH), 4.00 (s,1H,7-H), 4.58 (br,1H,3-H), 6.10 (s,1H,6-H), 7.2-7.4(m,5H,Ph).

| | |
|---|---|
| Analysis found :<br>$C_{33}H_{48}K_2O_{11}$.2.4$H_2O$ requires : | C,53.25;H,6.87;<br>C,53.40;H,7.17%. |

Example 31

[1S-[1α(3R*S*,4S*,5R*),3α,4β,5α,6α(4S*,6R*),7β]] 1-(4-Acetyloxy-3,5-dimethyl-6-phenylhexyl)-3-hydroxymethyl-4,6,7-trihydroxy-2,8-dioxabicyclo[3.2.1]octane-4,5-dicarboxylic acid, 6-(4,6-dimethyloctanoate), dipotassium salt

A solution of Example 29 Compound A (6.2mg) in dioxan (3ml) was treated with solution of potassium bicarbonate (0.1N; 0.182ml) and freeze-dried to give the title compound (8.8mg); δ($D_2O$) includes 0.76-0.94 (m,$CH_3$), 2.16 (br,3H,OAc), 2.59-2.72 (m,Ph$CH_2$), 3.43-3.70 (m,$CH_2$OH), 3.96 (s,7-H), 4.51 (br,3-H), 6.07 (br,6-H), 7.2-7.4 (m,5H,Ph); analytical HPLC retention time 17.62min (Spherisorb ODS-2 25cmx0.4cm column, solvent 35-95% acetonitrile/water containing 0.15ml conc. $H_2SO_4$/L over 23min).

Example 32

Characteristics of IMI 332962

After 2-3 weeks growth at 25°C on oatmeal agar the colonies were smoke grey to mouse grey in colour(Rayner's Mycological Colour Chart, 1970; published by the Commonwealth Agricultural Bureaux) on both the surface and reverse of the colony.

Morphological observations of the strain grown at 25°C on oatmeal agar were made under an optical microscope. The fungus had no sexual reproductive stage but formed pycnidia, thereby placing it in the class Coelomycetes. The fungus produced rostrate pycnidia with loose hyphae and both aseptate and one-septate conidia. The conidia were approximately 5-9 x 1.5-3μM in dimensions (usually 7-9 x 1.502.5μM). Numerous multiseptate/multicellular, globose structures resembling chlamydospores or pycnidial initials were also observed. Distinct dictyochlamydospores were absent.

The isolate has been identified as a species of the genus Phoma, and the identity confirmed by the CAB International Mycological Institute.

Example 33

IN VITRO RESULTS

(1) The ability of compounds of the invention to inhibit the enzyme squalene synthase was demonstrated using [2-$^{14}$C] farnesylpyrophosphate as substrate under assay conditions similar to those described by S. A. Biller et al in J Medicinal Chemistry 31(10), 1869-1871 (1988). [$^{14}$C] Squalene was separated from unreacted substrate on thin layer chromatography plates and determined by liquid scintillation counting. Inhibition of squalene synthase was quantified by incubating rat liver homogenate with various concentrations of the test compound in the presence of[2-$^{14}$C] farnesylpyrophosphate. The concentration of compound giving 50% inhibition of [$^{14}$C] squalene production in a 30 minute assay was taken as the $IC_{50}$ value.

In this test the title compounds in Examples 10, 11, 25, Example 3 Compound 1, Example 3 Compound 2 and Example 7 Compounds 1 and 2 had $IC_{50}$ values of less than 100nM.

(2) The in vitro evaluation of the antifungal activity of compounds of the invention was performed by determining the minimum concentration (MIC) of the test compound at which growth of the particular microorganism in a suitable medium failed to occur. In practice, a series of agar plates, each having the test compound incorporated at a particular concentration, was inoculated with a standard culture of a clinically relevant pathogen, for example Candida albicans, and each plate was then incubated at 37°C for 24 to 48 hours depending on the pathogen. The plates were then examined for the presence or absence of growth of the fungus and the appropriate MIC was noted.

In this test the title compounds in Examples 19, Example 3 Compound 1 and Example 7 Compound 1 had MICs in the range of 1 to 31 μg/ml against a variety of clinically relevant pathogens.

Pharmaceutical Examples

In the following examples the term 'Active Ingredient' refers to a compound of the present invention, for example a compound described in the Examples hereinabove.

Example 1 - Tablets

| | |
|---|---|
| a) Active Ingredient | 5.0mg |
| Lactose | 95.0mg |
| Microcrystalline Cellulose | 90.0mg |
| Cross-linked Polyvinylpyrrolidone 8.0mg | |
| Magnesium Stearate | 2.0mg |
| Compression Weight | 200.0mg |

The active ingredient, microcrystalline cellulose, lactose and cross-linked polyvinylpyrrolidone are sieved through a 500 micron sieve and blended in a suitable mixer. The magnesium stearate is sieved though a 250 micron sieve and blended with the active blend. The blend is compressed into tablets using suitable punches.

| | |
|---|---|
| b) Active Ingredient | 5.0mg |
| Lactose | 165.0mg |
| Pregelatinised Starch | 20.0mg |
| Cross-linked Polyvinylpyrrolidone 8.0mg | |
| Magnesium Stearate | 2.0mg |
| Compression weight | 200.0mg |

The active ingredient, lactose and pregelatinised starch are blended together and granulated with water. The wet mass is dried and milled. The magnesium stearate and cross-linked polyvinylpyrrolidone are screened through a 250 micron sieve and blended with the granule. The resultant blend is compressed using suitable tablet punches.

Example 2 - Capsules

| | |
|---|---|
| a) Active Ingredient | 5.0mg |
| Pregelatinised Starch | 193.0mg |
| Magnesium Stearate | 2.0mg |
| Fill weight | 200.0mg |

The active ingredient and pregelatinised starch are screened through a 500 micron mesh sieve, blended together and lubricated with magnesium stearate (meshed through a 250 micron sieve). The blend is filled into hard gelatin capsules of a suitable size.

| | |
|---|---|
| b) Active Ingredient | 5.0mg |
| Lactose | 177.0mg |
| Polyvinypyrrolidone | 8.0mg |
| Cross-linked Polyvinylpyrrolidone 8.0mg | |
| Magnesium Stearate | 2.0mg |
| Fill weight | 200.0mg |

The active ingredient and lactose are blended together and granulated with a solution of polyvinylpyrrolidone. The wet mass is dried and milled. The magnesium stearate and cross-linked polyvinylpyrrolidone are screened through a 250 micron sieve and blended with the granule. The resultant blend is filled into hard gelatin capsules of a suitable size.

Example 3 - Syrup

| | |
|---|---|
| a) Active Ingredient | 5.0mg |
| Hydroxypropyl Methylcellulose 45.0mg | |
| Propyl Hydroxybenzoate | 1.5mg |
| Butyl Hydroxybenzoate | 0.75mg |
| Saccharin Sodium | 5.0mg |
| Sorbitol Solution | 1.0ml |
| Suitable Buffers | qs |
| Suitable Flavours | qs |
| Purified Water to | 10.0ml |

The hydroxypropyl methylcellulose is dispersed in a portion of hot purified water together with the hydroxybenzoates and the solution is allowed to cool to room temperature. The saccharin sodium, flavours and sorbitol solution are added to the bulk solution. The active ingredient is dissolved in a portion of the remaining water and added to the bulk solution. Suitable buffers may be added to control the pH in the region of maximum stability. The solution is made up to volume, filtered and filled into suitable containers.

Example 4 - Intranasal Solution

| a) Preserved solution | |
|---|---|
| | % w/w |
| Active Ingredient | 0.1 |
| Dextrose (Anhydrous) | 5.0 |
| Benzalkonium Chloride | 0.02 |
| Suitable buffers | qs |
| Purified Water to | 100 |

The active ingredient and dextrose are dissolved in a portion of the bulk solution. Suitable buffers may be added to control the pH in the region of maximum stability. The solution is made up to volume, filtered and filled into suitable containers.

Alternatively, the solution may be provided as a sterile unit dose presentation such that the preservatives are omitted from the formulation.

| b) Unpreserved sterile solution | |
|---|---|
| | % w/w |
| Active Ingredient | 0.1 |
| Dextrose (Anhydrous) | 5.0 |
| Suitable Buffers | qs |
| Purified Water to | 100 |

**Claims**

**1.** Compounds having the formula(I)

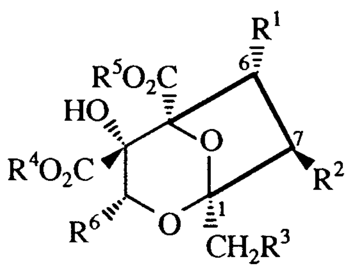

(I)

wherein $R^1$ represents a hydrogen atom or a hydroxyl, acyloxy, carbonate, carbamate or ether group;

$R^2$ represents a hydrogen atom, a hydroxyl group, a group $-OCOR^7$ or a group $-OCO_2R^7$ (where $R^7$ is a group selected from $C_{1-8}$alkyl, aryl, aryl$C_{1-4}$alkyl and $C_{3-8}$cycloalkyl);

$R^3$ represents a group selected from

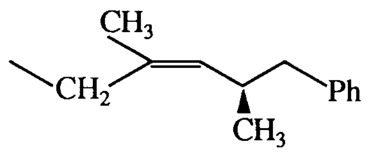 ,

$-CH{\cdots}CR^8CR^9R^{10}CHR^{11}(CH_2)_mPh,$

$-CH_2CR^{12}{\cdots}CR^{11}CR^9R^{10}CHR^{11}(CH_2)_nPh,$

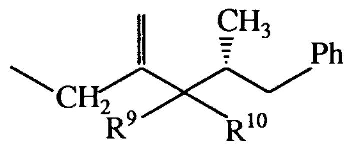 ,

$-CH_2C(CH_3)\overset{E}{=}CHCH(CH_2R^{13})CH_2Ph$, $-CH_2C(CH_2OH)\overset{Z}{=}CHCH(CH_3)CH_2Ph$,

$-CH_2C(=CH_2)CH(OH)CH(CH_2OH)CH_2Ph$,

$-CH_2C(=CH_2)CH(NHCOCH_3)CH(CH_3)CH_2Ph$,

$-CH_2C(CH_2NHCOCH_3)\overset{E}{=}CHCH(CH_3)CH_2Ph$ and

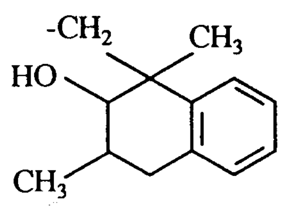

(where the dotted line represents the absence or presence of a single bond, $R^8$ represents a hydrogen atom or a hydroxyl, acyloxy, $C_{1-6}$alkoxy or $C_{1-4}$alkyl group, $R^9$ represents a hydrogen atom and $R^{10}$ represents a hydrogen atom or a hydroxyl, $C_{1-6}$alkoxy or acyloxy group or $CR^9R^{10}$ forms a group C=0, $R^{11}$ represents a hydrogen atom or a $C_{1-4}$alkyl group, $R^{12}$ represents a hydrogen atom or a methyl group, $R^{13}$ represents a hydrogen atom or a hydroxyl group, m represents 1 or 2 and n represents zero or 1);

$R^4$ and $R^5$ may each independently represent a hydrogen atom or a methyl group;

$R^6$ represents a hydrogen atom or a hydroxymethyl group; and salts thereof; with the proviso $R^1$ and $R^2$ cannot both represent hydrogen atoms.

**2.** Compounds according to Claim 1 in which $R^4$ and $R^5$ represent hydrogen atoms.

**3.** Compounds according to Claim 1 or Claim 2 in which $R^1$ represents an acyloxy group.

**4.** Compounds according to Claim 3 in which $R^1$ represents a group selected from

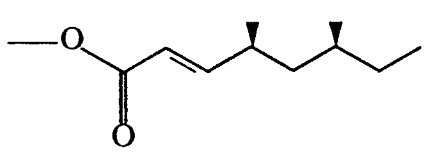 , 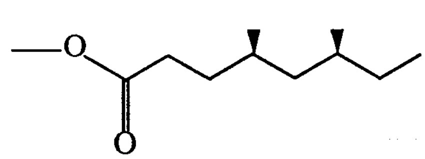

and alkanoyloxy.

**5.** Compounds according to any preceding claim in which $R^2$ represents a hydroxy, acetoxy or $-OCO_2CH_3$ group.

**6.** Compounds according to any preceding claim in which $R^3$ represents

$$-CH\underline{---}CR^{8}CR^{9}R^{10}CHR^{11}CH_{2}Ph \text{ or}$$

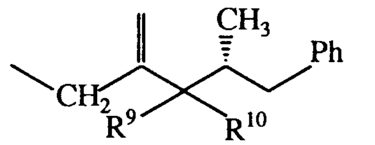

.

**7.** Compounds according to any preceding claim in which within $R^3$ the group

$$-CH\underline{---}CR^{8}-$$

represents $-CH_2CH(CH_3)-$ or $-CH_2C(=O)-$.

**8.** Compounds according to any preceding claim in which within $R^3$ the group $-CR^9R^{10}-$ represents -CH-(OH)-, $-CH_2-$ or $-CH(OCOCH_3)-$.

**9.** Compounds according to any preceding claim in which $R^6$ represents a hydrogen atom.

**10.** Compounds according to any of Claims 1 to 8 in which $R^6$ represents a hydroxymethyl group.

**11.** [1S-[1α(4R*,5S*),3α,4β,5α,6α(4S*,6R*),7β]] 1-(4-Hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate);
[1S-[1α(4R*,5S*),4β,5α,6α(2E,4R*,6R*),7β]] 1-(4-acetyloxy-5-methyl-3-methylene-6-phenylhexyl)-4,6,7-trihydroxy-2,8-dioxabicyclo [3.2.1]octane-4,5-dicarboxylic acid, 6-(4,6-dimethyl-2-octenoate);
[1S-[1α(4R*,5S*),3α,4β,5α,6α(2E,4R*,6R*),7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate);
[1S-[1α(4R*,5S*),3α,4β,5α,6α(4S*,6R*),7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyloctanoate);
[1S-[1α(4R*,5S*),3α,4β,5α,6α(2E,4R*,6R*),7β]] 1-(4-hydroxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4,6-dimethyl-2-octenoate);
[1S-[1α(4R*,5S*),3α,4β,5α,6α,7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-heptanoate;
[1S-[1α(4R*,5S*),3α,4β,5α,6α,7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-nonanoate;
[1S-[1α(4R*,5S*),3α,4β,5α,6α(4R*S*),7β]] 1-(4-acetyloxy-5-methyl-3-methylene-6-phenylhexyl)-3-hydroxymethyl-4,6,7-trihydroxy-2,8-dioxabicyclo[3.2.1]octane-4,5-dicarboxylic acid, 6-(4-methyl)-pentanoate;
[1S-[1α(4R*,5S*),3α,4β,5α,6α(4S*,6R*),7β]] 1-(4-hydroxy-5-methyl-3-oxo-6-phenylhexyl)-3-hydroxymethyl-4,6,7-trihydroxy-2,8-dioxabicyclo[3.2.1]octane-4,5-dicarboxylic acid, 6-(4,6-dimethyloctanoate);
and physiologically acceptable salts thereof.

**12.** A compound according to any preceding claim for use in therapy.

**13.** A compound according to any preceding claim for use in the treatment of conditions where a lowering

of the level of blood plasma cholesterol in animals, including humans, would be beneficial.

**14.** A compound according to any of Claims 1 to 12 for use in the treatment of fungal infections in a human or non-human animal patient.

**15.** A method of treatment of the human or non-human animal body to combat diseases associated with hypercholesterolemia and/or hyperlipoproteinemia or to combat fungal diseases, which method comprises administering to said body an effective amount of a compound as claimed in any of Claims 1 to 12 which inhibits squalene synthase.

**16.** A pharmaceutical composition comprising a compound according to any of Claims 1 to 12 together with one or more carriers and/or excipients.

**17.** A pharmaceutical composition comprising an active amount of a compound as claimed in any of Claims 1 to 12 for use in the treatment of conditions where a lowering of the level of blood plasma cholesterol in animals, including humans, would be beneficial.

**18.** A pharmaceutical composition comprising an active amount of a compound as claimed in any of Claims 1 to 12 for use in the treatment of fungal infections in a human or non-human animal patient.

**19.** A pharmaceutical composition according to any one of Claims 16 to 18 in a form suitable for oral, buccal, topical, parenteral, implant, rectal, ophthalmic or genito-urinary administration or in a form suitable for administration by inhalation or insufflation.

**20.** A pharmaceutical composition according to any one of Claims 16 to 19 in unit dosage form.

**21.** Use of a compound according to any of Claims 1 to 12 in the manufacture of a medicament for the treatment of hpercholesterolemia and/or hyperlipoproteinemia in a human or non-human animal patient.

**22.** Use of a compound according to any of Claims 1 to 12 in the manufacture of a medicament for the treatment of fungal infections in a human or non-human animal patient.

**23.** A process for the preparation of a compound as claimed in Claim 1 which comprises :
(A) (in the preparation of compounds of formula (I) in which $R^6$ represents a hydrogen atom) reacting a compound of formula (II)

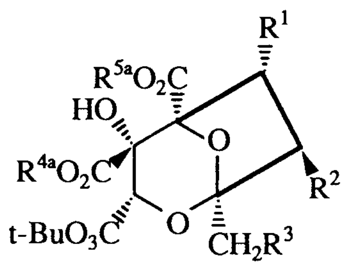

(II)

(wherein $R^1$, $R^2$ and $R^3$ are as defined in Claim 1 and $R^{4a}$ and $R^{5a}$ are protecting groups) to remove the group $t\text{-}BuO_3C$ by reduction under free radical conditions, followed by removal of the protecting groups present;
(B) (in the preparation of compounds of formula (I) in which $R^6$ represents a hydroxymethyl group) reacting a compound of formula (III)

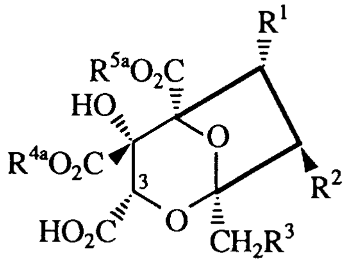

(III)

(wherein $R^1$, $R^2$, $R^3$, $R^{4a}$ and $R^{5a}$ are as defined herein) to reduce the 3-carboxyl group to a hydroxymethyl group, followed by removal of the protecting groups present;

(C) (in the preparation of compounds of formula (I) in which $R^3$ represents a group $-CH=CR^8CR^9R^{10}CHR^{11}(CH_2)_mPh$ or a group $-CH_2CR^{12}=CR^{11}CR^9R^{10}CHR^{11}(CH_2)_nPh$ where $R^8$ is hydrogen or $C_{1-4}$alkyl) reacting a compound of formula (V)

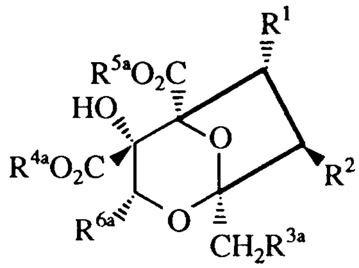

(V)

(wherein $R^{3a}$ represents CHO or $CH_2COR^{12}$ as appropriate, $R^1$ and $R^2$ are as defined in Claim 1, $R^{4a}$ and $R^{5a}$ are as defined for $R^4$ and $R^5$ in Claim 1 or are protecting groups and $R^{6a}$ is as defined for $R^6$ in Claim 1 or is a protected hydroxymethyl group) with a compound of formula (VIa) or (VIb)

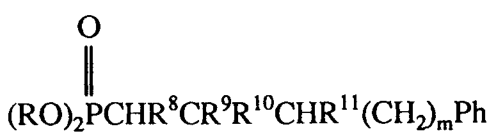

(VIa)

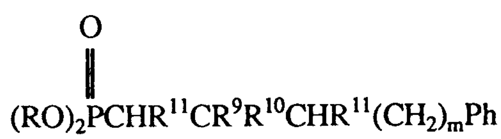

(VIb)

as appropriate (wherein $R^{11}$ is as defined in Claim 1, $R^8$ is hydrogen or $C_{1-4}$alkyl, $CR^9R^{10}$ forms a group C=0 and R represents a $C_{1-6}$alkyl group or an aryl group), and thereafter removing any protecting groups present;

(D) converting a compound of formula (I) or a protected derivative thereof to a different compound of formula (I) or a protected derivative thereof, followed, if necessary, by the removal of any protecting groups present;

(E) (in the preparation of compounds of formula (I) in which $R^2$ represents a group $-OCOR^7$ or $-OCO_2R^7$) reacting a compound of formula (XII)

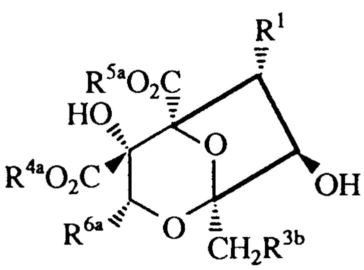

(XII)

(wherein $R^1$ is as defined in Claim 1, $R^{3b}$ is as defined for $R^3$ in Claim 1 or is a protected derivative

thereof and $R^{4a}$, $R^{5a}$ and $R^{6a}$ are as defined herein) under conditions for ester or carbonate formation, followed by removal of the protecting groups present; or

(F) (in the preparation of compounds of formula (I) in which $R^1$ represents an acyloxy, carbonate, carbamate or ether group) reacting a compound of formula (XIII)

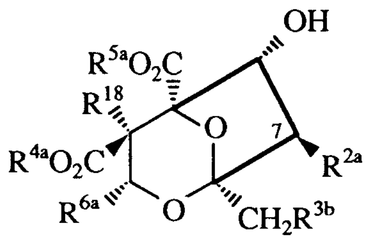

(XIII)

(wherein $R^{2a}$ is as defined for $R^2$ in Claim 1 or is a protected hydroxyl group, $R^{3b}$ and $R^{4a}$-$R^{6a}$ are as defined herein and $R^{18}$ is a hydroxyl group or a protected hydroxyl group) to convert the 6-position hydroxyl group to an acyloxy, carbonate, carbamate or ether group, followed by removal of any protecting groups present.

**24.** Compounds of formulae (II), (V) and (VII) to (XVI).

**25.** Compounds according to any of Claims 1 to 12 substantially as herein described.

**26.** Compositions according to any one of Claims 16 to 20 substantially as herein described.

European Patent Office

# PARTIAL EUROPEAN SEARCH REPORT

which under Rule 45 of the European Patent Convention shall be considered, for the purposes of subsequent proceedings, as the European search report

Application Number

EP 92 10 0122

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CH-A- 606 071 (CIBA-GEIGY)(13-10-1978)<br>* Column 1 - column 2, line 23 *<br>--- | 1,13,14 | C 07 D 493/08<br>A 61 K 31/34<br>C 12 P 17/18<br>C 07 H 19/01<br>C 12 P 19/02<br>A 61 K 31/71 //<br>(C 07 D 493/08<br>C 07 D 319:00<br>C 07 D 307:00 ) |
| A | EP-A-0 259 087 (MERCK)(09-03-1988)<br>* Claims 1,9 *<br>--- | 1,13,14 | |
| P,A | US-A-5 026 554 (K.F. BARTIZAI et al.)(25-06-1991)<br>* Column 1, line 50 - column 4, line 19 *<br>----- | 1,13,14 | |
| | | | TECHNICAL FIELDS SEARCHED (Int. Cl.5) |
| | | | C 07 D 493/00<br>C 12 P 17/00<br>C 07 H 19/00<br>C 12 P 19/00 |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claim 15 is directed to a method of treatment of the human/animal body (Art. 52(4) EPC) the search has been carried out and based on the alleged effects of the compound/composition

| Place of search | Date of completion of the search | Examiner |
|---|---|---|
| THE HAGUE | 31-03-1992 | HEYWOOD C.J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)